# EUROPEAN PATENT SPECIFICATION

(11) **EP 1 370 553 B1**
(45) Date of publication and mention of the grant of the patent: **10.05.2006**
(21) Application number: 02719303.6
(22) Date of filing: 22.03.2002
(51) Int. Cl.: C07D 403/04, C07D 401/14, C07D 403/14, A61K 31/517, A61P 15/10, C07D 403/12, C07D 405/14, C07D 409/14

(54) **RHO-KINASE INHIBITORS**
RHO-KINASE INHIBITOREN
INHIBITEURS DE RHO-KINASE

(30) Priority: 23.03.2001 US 277974 P; 29.08.2001 US 315341 P
(43) Date of publication of application: 17.12.2003
(73) Proprietor: BAYER CORPORATION, Pittsburgh, PA 15205 (US)
(72) Inventor: NAGARATHNAM, Dhanapalan, Bethany, CT 06524 (US); ASGARI, Davoud, Newtown, PA 18940 (US); SHAO, Jianxing, Cheshire, CT 06410 (US); LIU, Xiao-Gao, New Haven, CT 06515 (US); KHIRE, Uday, Hamden, CT 06518 (US); WANG, Chunguang, Hamden, CT 06514 (US); HART, Barry, Woodbridge, CT 06525 (US); BOYER, Stephen, Fairfield, CT 06430 (US); WEBER, Olaf, Woodbridge, CT 06525 (US); LYNCH, Mark, Madison, CT 06443 (US); BANKSTON, Donald, Wallingford, CT 06492 (US)
(74) Representative: Portal, Gérard
(86) International application number: PCT/US2002/008659
(87) International publication number: WO 2002/076976

(56) References cited:
- EP-A- 0 602 851
- WO-A-95/15758
- WO-A-96/39145
- WO-A-97/03069
- WO-A-99/35132

## Description

### Field of the Invention

This application claims the benefit of the filing date of U.S. Provisional Application No. 60/277,974, filed March 23, 2001 and U.S. Provisional Application No. 60/315,341, filed August 29, 2001.

The present invention relates to compounds and derivatives thereof, their synthesis, and their use as Rho-kinase inhibitors. These compounds of the present invention are useful for inhibiting tumor growth, treating erectile dysfunction, and treating other indications mediated by Rho-kinase, e.g., coronary heart disease.

### Background

The pathology of a number of human and animal diseases including hypertension, erectile dysfunction, coronary cerebral circulatory impairments, neurodegenerative disorders and cancer can be linked directly to changes in the actin cytoskeleton. These diseases pose a serious unmet medical need. The actin cytoskeleton is composed of a meshwork of actin filaments and actin-binding proteins found in all eukaryotic cells. In smooth muscle cells the assembly and disassembly of the actin cytoskeleton is the primary motor force responsible for smooth muscle contraction and relaxation. In non-muscle cells, dynamic rearrangements of the actin cytoskeleton are responsible for regulating cell morphology, cell motility, actin stress fiber formation, cell adhesion and specialized cellular functions such as neurite retraction, phagocytosis or cytokinesis (Van Aelst, et al. *Genes Dev* **1997,** *11*, 2295).

The actin cytoskeleton is controlled by a family of proteins that are a subset of the Ras superfamily of GTPases. This subset currently consists of RhoA through E and RhoG (refereed to collectively as Rho), Rac 1 and 2, Cdc42Hs and G25K and TC10 isoforms (Mackay, et al. *J Biol Chem* **1998,** *273,* 20685). These proteins are GTP (guanine nucleotide triphosphate) binding proteins with intrinsic GTPase activity. They act as molecular switches and cycles between inactive GDP (guanine nucleotide diphosphate) bound and active GTP bound states. Using biochemical and genetic manipulations, it has been possible to assign functions to each family member. Upon activation the Rho proteins controls the formation of actin stress fibers, thick bundles of actin filaments, and the clustering of integrins at focal adhesion complexes. When activated the Rac proteins control the formation of lamellopodia or membrane ruffles on the cell surface and Cdc42 controls filopodia formation. Together this family of proteins plays a critical part in the control of key cellular functions including cell movement, axonal guidance, cytokinesis, and changes in cell morphology, shape and polarity.

Depending on the cell type and the activating receptor, the Rho proteins can control different biological responses. In smooth muscle cells, Rho proteins are responsible for the calcium sensitization during smooth muscle contraction. In non-smooth muscle cells the Rho GTPases are responsible for the cellular responses to agonist such as lysophosphatidic acid (LPA), thrombin and thromboxane A₂ (Fukata, et al. *Trends Pharcol Sci* **2001**, *22*, 32). Agonist response is coupled through heterotrimeric G proteins Gₐₗₚₕₐ₁₂ or Gₐₗₚₕₐ₁₃ (Goetzl, et al. *Cancer Res* **1999**, *59,* 4732; Buhl, et al. *J Biol Chem* **1995**, *270,* 24631) though other receptors may be involved. Upon activation Rho GTPases activate a number of downstream effectors including PIP5-kinase, Rhothekin, Rhophilin, PKN and Rho-Kinase isoforms ROCK-1/ROKbeta and ROCK-1/ROKalpha (Mackay and Hall *J Biol Chem* **1998,** 273, 20685; Aspenstrom *Curr Opin Cell Biol* **1999,** *11*, 95; Amano, et al. *Exp Cell Res* **2000**, *261,* 44).

Rho-kinase was identified as a RhoA interacting protein isolated from bovine brain (Matsui, et al. *Embo J* **1996,** *15*, 2208). It is a member of the myotonic dystrophy family of protein kinase and contains a serine/threonine kinase domain at the amino terminus, a coiled-coil domain in the central region and a Rho interaction domain at the carboxy terminus (Amano, et al. *Exp Cell Res* **2000**, *261*, 44). Its kinase activity is enhanced upon binding to GTP-bound RhoA and when introduced into cells, it can reproduce many of the activities of activated RhoA. In smooth muscle cells Rho-Kinase mediates calcium sensitization and smooth muscle contraction and inhibition of Rho-kinase blocks 5-HT and phenylephrine agonist induced muscle contraction. When introduced into non-smooth muscle cells, Rho-kinase induces stress fiber formation and is required for the cellular transformation mediated by RhoA (Sahai, et al. *Curr Biol* **1999,** *9,* 136). Rho-kinase regulates a number of downstream proteins through phosphorylation, including myosin light chain (Somlyo, et al. *J Physiol (Lond)* **2000**, *522 Pt 2*, 177), the myosin light chain phosphatase binding subunit (Fukata, et al. *J Cell Biol* **1998,** *141,* 409) and LIM-kinase 2 (Sumi, et al. *J Bio Chem* **2001,** *276,* 670).

Inhibition of Rho-kinase activity in animal models has demonstrated a number of benefits of Rho-kinase inhibitors for the treatment of human diseases. Several patents have appeared claiming (+)-trans-4-(1-aminoethyl)-1-(pyridin-4-ylaminocarbonyl)cyclohexane dihydrochloride monohydrate (WO-00078351, WO-00057913) and substituted isoquinolinesulfonyl (EP-00187371) compounds as Rho-kinase inhibitors with activity in animal models. These include models of cardiovascular diseases such as hypertension (Uehata, et al. *Nature* **1997**, *389*, 990), atherosclerosis (Retzer, et al. *FEBS Lett* **2000,** *466*, 70), restenosis (Eto, et al. *Am J Physiol Heart Circ Physiol* **2000**, *278*, H1744; Negoro, et al. *Biochem Biophys Res Commun* **1999**, *262*, 211), cerebral ischemia (Uehata, et al. *Nature* **1997**, *389*, 990: Seasholtz, et al. *Circ Res* **1999**, *84,* 1186; Hitomi, et al. *Life Sci* **2000,** *67*, 1929; Yamamoto, et al. *J Cardiovasc Pharmacol* **2000,** *35*, 203), cerebral vasospasm (Sato, et al. *Circ Res* **2000,** *87,* 195; Kim, et al. *Neurosurgery* **2000,** 46, 440), penile erectile dysfunction (Chitaley, et al. *Nat Med* **2001**, *7*, 119), central nervous system disorders such as neuronal degeneration and spinal cord injury (Hara, et al. *J Neurosurg* **2000,** *93,* 94; Toshima, et al. *Stroke* **2000,** *31,* 2245) and in neoplasias where inhibition of Rho-kinase has been shown to inhibit tumor cell growth and metastasis (Itoh, et al. *Nat Med* **1999,** *5*, 221; Somlyo, et al. *Biochem Bioplrys Res Commun* **2000,** *269*, 652), angiogenesis (Uchida, et al. *Biochem Biophys Res Commun* **2000,** *269*, 633; Gingras, et al. *Biochem J* **2000,** *348 Pt 2*, 273), arterial thrombotic disorders such as platelet aggregation (Klages, et al. *J Cell Biol* **1999,** *144*, 745; Retzer, et al. *Cell Signal* **2000**, *12*, 645) and leukocyte aggregation (Kawaguchi, et al. *Eur J Pharmacol* **2000**, *403*, 203; Sanchez-Madrid, et al. *Embo J* **1999**, *18*, 501), asthma (Setoguchi, et al. *Br J Pharmacol* **2001**, *132*, 111; Nakahara, et al. *Eur J Pharmacol* **2000,** *389*, 103), regulation of intraoccular pressure (Honjo, et al. *Invest Ophthalmol Vis Sci* **2001**, *42*, 137) and bone resorption (Chellaiah, et al. *J Biol Chem* **2000**, *275*, 11993; Zhang, et al. *J Cell Sci* **1995**, *108*, 2285).

The inhibition of Rho-kinase activity in patients has benefits for controlling cerebral vasospasms and ischemia following subarachnoid hemorrhage (*Pharma Japan* **1995,** *1470,* 16).

EP-A-0 602 851 A relates to quinazoline derivatives and to the use of their receptor tyrosine kinase inhibitory properties in the treatment of cancer.

WO 95 15758 A relates to aryl and heteroaryl quinazoline compounds which are protein tyrosine kinase (PTK) inhibitors. A method of treting cell proliferation and/or differentiation or mediator release using the compounds is described.

WO 96 39145 A relates to protein tyrosine kinase aryl and heteroaryl quinazoline compounds having selective inhibition of human epidermal growth factor receptor type 2 (HER-2) autophosphorylation properties.

WO 99 35132 A relates to substituted heteroaromatic compounds.

WO 02 34744 A relates to quinazoline derivatives and to their use in the manufacture of a medicament for use as an anti-invasive agent in the containment and/or treatment of solid tumour disease.

In EP-A-0 602 851 A, WO 95 15758 A, WO 96 39145 A, WO 99 35132 A, WO 02 34744 A the group corresponding to the -X-A group of the present patent application is H. No compound of EP-A-0 602 851 A, WO 95 157 58, WO 96 39145 A, WO 99 35132 A, WO 02 34744 A falls into the definition of the claims of the present application, since the definition of A in the present claims is not H for all values of x. Furthermore, there is no teaching or suggestion for any other group in the 2-position of the quinazolines of EP-A-0 602 851 A or WO 95 15758 A, WO 96 39145 A, WO 99 35132, A WO 02 34744 A.

WO 97 03069 A relates to heterocyclic compounds which are protein tyrosine kinase inhibitors, particularly substituted quinolines and quinazolines, and to their use in medicine. The definition of the value A of the present patent application is not H, halogen, or -OR₈. No compound of WO 97 03069 A falls into the definition of the claims of the present patent application.

WO 02 24667 A1 relates to 4-aminoquinazolines and to their use as glycoprotein IbIX antagonists. The overlap of definitions of compounds of WO 02 24667 A1 and of compounds as defined in the claims of the present patent application is generic. The definition of the quinazoline compounds of the present patent application is limited to completely unsaturated, bicyclic heterocyclic radical having 9 ring members, said heterocyde being a 5(6)-amino indole/indazole/benzimidazole/benzotriazole, optionally bearing substituents. Furthermore, WO 02 24667 A1 has no anticipating species falling into the definition of the compounds of the present patent application as defined in the claims.

Finally, WO 97 20822 A1 relates to quinazolin-2,4-diazirines as neuropeptide Y (NPY) receptor antagonists. No compound of WO 97 20822 A1 falls into the definition of the compounds of the present invention as defined in the claims of the present patent application since the definition of A of compounds of formula (I) of the present patent application is not a 3-20 atom, cyclic or polycyclic moiety, containing 1-4 rings, which optionally contain 1-3 N, O or S atoms per ring, and may optionally be aryl or heteroaryl, which cyclic or polycyclic moiety may optionally be substituted up to 3 times by -OR⁸, wherein R⁸ is a hydrogen atom or a C₁₋₆ alkyl group.

N.B. WO 02 24667 A and WO 02 34744 A are prior art under Art. 54(3) EPC and are thus only relevant for assessing novelty, since they have been published after the filing date of the present patent application.

### Summary of the Invention

The compounds and their derivatives presented in this invention are useful as Rho-Kinase inhibitors and thus have utilities in the treatment of hypertension, a therosclerosis, restenosis, cerebral ischemia, cerebral vasospasm, neuronal degeneration spinal cord injury, cancers of the breast, colon, prostate, ovaries, brain and lung and their metastases, thrombotic disorders, asthma, glaucoma and osteoporosis.

In addition, the compounds of the invention are useful to treat erectile dysfunction, i.e., erectile dysfunction mediated by Rho-kinase. Erectile dysfunction can be defined as an inability to obtain or sustain an erection adequate for intercourse, WO 94/28902, U.S.P. 6,103,765 and U.S.P. 6,124,461.

The invention provides compounds of Formula I as defined in claim 1.

Suitable alkyl groups and alkyl portions of groups, e.g., alkoxy, etc. throughout include methyl, ethyl, propyl, butyl, etc., including all straight-chain and branched isomers such as isopropyl, isobutyl, *sec*-butyl, *tert*-butyl, etc.

Suitable cycloalkyl groups include cyclopropyl, cyclopropyl, cyclobutyl, cyclopentyl, cyclohexyl, etc.

Suitable halogen groups include F, Cl, Br, and/or I, from one to per-substitution (i.e., all H atoms on a group replaced by a halogen atom) being possible, mixed substitution of halogen atom types also being possible on a given moiety.

In Formula I, suitable aryl or heteroaryl groups, e.g., for A, include, but are not limited to, 5-12 carbon-atom aromatic rings or ring systems containing 1-3 rings, at least one of which is aromatic, in which one or more, e.g., 1-4 carbon atoms in one or more of the rings can be replaced by oxygen, nitrogen or sulfur atoms. Each ring typically has 3-7 atoms. For example, aryl or heteroaryl can be 2- or 3-furyl, 2- or 3-thienyl, 2- or 4-triazinyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or 5-yl, 1,2,4-triazol-1-, -3- or 5-yl, 1- or 5-tetrazolyl, 1,2,3-oxadiazol-4- or 5-yl, 1,2,4-oxadiazol-3- or 5-yl, 1,3,4-thiadiazol-2- or 5-yl, 1,2,4-oxadiazol-3-or 5-yl, 1,3,4-thiadiazol-2- or 5-yl, 1,3,4-thiadiazol-3- or 5-yl, 1,2,3-thiadiazol-4- or 5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-, 6- or 7-benzothiazolyl, 2-, 4-, 5-, 6- or 7-benzisothiazolyl, 2-, 4-, 5-, 6- or 7-benz-1,3-oxadiazolyl, 2-, 3-, 4-, 5-, 6-, 7- or 8-quinolinyl, 1-, 3-, 4-, 5-, 6-, 7-, 8- isoquinolinyl, 1-, 2-, 3-, 4- or 9-carbazolyl, 1-, 2-, 3-, 4-, 5-, 6-, 7-, 8- or 9-acridinyl, or 2-, 4-, 5-, 6-, 7- or 8-quinazolinyl, or additionally optionally substituted phenyl, 2- or 3-thienyl, 1,3,4-thiadiazolyl, 3-pyrryl, 3-pyrazolyl, 2-thiazolyl or 5-thiazolyl, etc.

Preferred moieties A include cyclohexyl; or C₅₋₁₂-aryl or C₅₋₁₂-heteroaryl each independently optionally substituted up to three times by (i) C₁-C₁₀-alkyl or C₂₋₁₀-alkenyl each optionally substituted with halogen up to perhalo; (ii) C₃-C₁₀ cycloalkyl; (iii) C₅₋₁₂-aryl optionally substituted by 1-3 halogen atoms; (iv) C₅₋₁₂-heteroaryl; (v) halogen; (vi) -CO-OR₈; (vii) -COR₈; (viii) cyano; (ix) -NR₈R₁₃; (x) nitro; (xi) -CO-NR₈R₉; (xii) -C₁₋₁₀-alkyl-NR₈R₉; (xiii) -NR₈-CO-R₁₂; (xiv) -NR₈-CO-OR₉; (xv) -NR₈-SO₂-R₉; (xvi) -SR₈; (xvii) -SO₂-R₈; (xviii) -SO₂-NR₈R₉, or (xix) NR₈-CO-NHR₉.

Further preferred moieties A include phenyl, pyridyl, pyrimidinyl, oxazolyl, furyl, thienyl, pyrrolyl, imidazolyl, isoxazolyl and pyrazinyl, each independently substituted up to three times by halogen, C₁₋₁₀-alkyl, C₁₋₁₀-alkoxyphenyl, naphthyl, -OR₁₀, 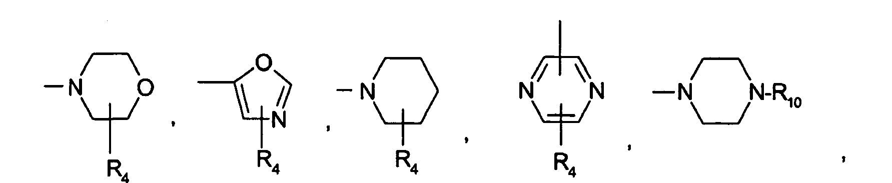 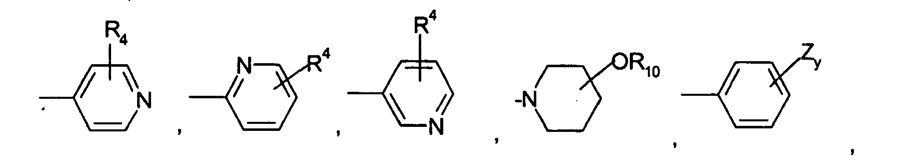 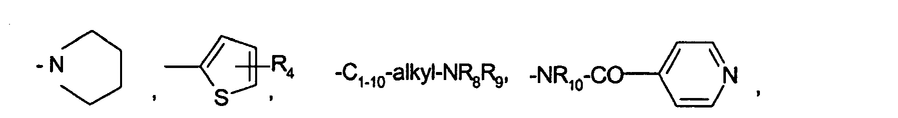 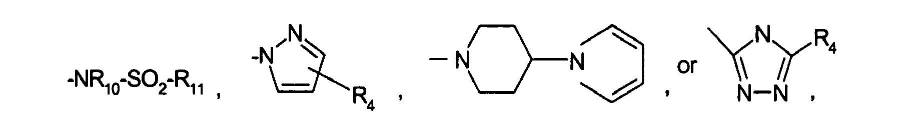 wherein each Z independently is halogen, hydroxy, hydroxy-C₁₋₁₀-alkyl, -CN, -NO₂, C₁₋₁₀₋alkoxycarboxyl, -NR₁₀-CO-R₁₁, or -NR₁₀-CO-OR₁₁,
y is 1-3,
and R₄ is as described above

Preferred moieties A additionally include 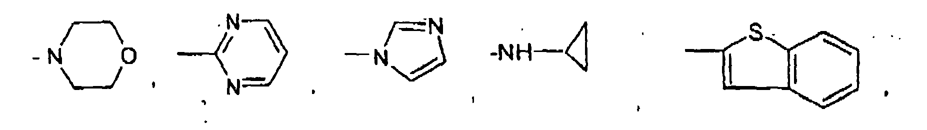 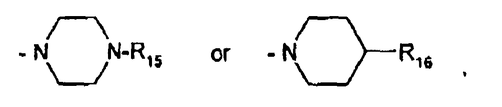 wherein R₁₅ is H; phenyl optionally substituted by C₁₋₁₀-alkyl, C₁₋₁₀-alkoxy, C₁₋₁₀₋alkylcarboxyl, or halogen; benzyl; pyramidal or pyridyl; and R₁₆ is H, phenyl, -COOR₁₀,

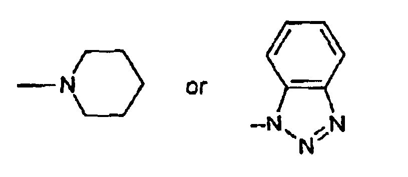

Further, preferred compounds of formula (I) are those wherein R⁵ in a, b or c is hydrogen or C₁₋₁₀₋alkyl or C₂₋₁₀-alkyl optionally substituted by amino, N-lower alkylamino, N,N-dllower alkylamino, N-lower alkanoylamino, hydroxy, cyano, -COOR₁₀, -COR₁₄, -OCOR₁₄, -OR₁₀, C₅₋₁₀-heteroaryl, C₅₋₁₀-heteroaryloxy, or C₅₋₁₀-heteroaryl-C₁₋₁₀-alkoxy, halogen up to perhalo; (iii) C₃-C₁₀ cycloalkyl, in which 1-3 carbon atoms are optionally independently replaced by O, N or S; (iv) C₃₋₁₀-cycloalkenyl; (v) partially unsaturated C₅₋₁₀-heterocyclyl; (vi) aryl; (vii) heteroaryl; (viii) halogen; (ix) -CO-OR₁₀; (x) -OCOR₁₀; (xi) -OCO₂R₁₀; (xii) -CHO; (xiii) cyano; (xiv) -OR₁₆; (xv) -NR₁₀R₁₅; (xvi) nitro; (xvii) -CO-NR₁₀R₁₁; (xviii) -NR₁₀-CO-R₁₂; (xix) -NR₁₀-CO-OR₁₁; (xx) -NR₁₀-SO₂₋R₁₂; (xxi) -SR₁₆; (xxii) -SOR₁₆; (xxiii) -SO₂-R₁₆; (xxiv) -SO₂-NR₁₀R₁₁; (xxv) NR₁₀-CO-NHR₁₁; (xxvi) amidino; (xxvii) guanidino; (xxviii) sulfo; (xxix) -B(OH)₂; (xxx) -OCON(R₁₀)₂; or (xxxi) -NR₁₀CON(R₁₀)₂.

Further, preferred compounds of formula (I) are those wherein Y is N and R₁ is H,
particularly wherein a is -NR₆-, R₆ is H, and c is -N=,
more particularly wherein p is 0 and R₁₋₄ are H,
more particularly wherein X is -(CH₂)ₓ- and x is 0,
more particularly wherein A is biphenyl optionally substituted by halogen.

The present invention is also directed to pharmaceutically acceptable salts of Formula 1. Suitable pharmaceutically acceptable salts are well known to those skilled in the art and include basic salts of inorganic and organic acids, such as hydrochloric acid, hydrobromic acid, sulphuric acid, phosphoric acid, methanesulphonic acid, sulphonic acid, acetic acid, trifluoroacetic acid, malic acid, tartaric acid, citric acid, lactic acid, oxalic acid, succinic acid, fumaric acid, maleic acid, benzoic acid, salicyclic acid, phenylacetic acid, and mandelic acid. In addition, pharmaceutically acceptable salts include acid salts of inorganic bases, such as salts containing alkaline cations (e.g., Li⁺, Na⁺ or K⁺), alkaline earth cations (e.g., Mg⁺, Ca⁺ or Ba⁺), the ammonium cation, as well as acid salts of organic bases, including aliphatic and aromatic substituted ammonium, and quaternary ammonium cations, such as those arising from protonation or peralkylation of triethylamine, *N*,*N*-diethylamine, *N,N-*dicyclohexylamine, pyridine, *N*,*N*-dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU).

A number of the compounds of Formula I possess asymmetric carbons and can therefore exist in racemic and optically active forms. Methods of separation of enantiomeric and diastereomeric mixtures are well known to one skilled in the art. The present invention encompasses any isolated racemic or optically active form of compounds described in Formula I which possess Rho-kinase inhibitory activity.

The invention also includes pharmaceutical compositions including a compound of Formula I, and a physiologically acceptable carrier.

Preferred compounds include:
2-(2,4-dichlorophenyl)-*N*-(1*H*-indazol-5-yl)-4-quinazolinamine, 2-(4-chlorophenyl)-*N*-(1*H*-indazol-5-yl)-4-quinazolinamine, 1-{4-[4-(1*H*-indazol-5-ylamino)-2-quinazolinyl]phenyl}ethanone, *N*-(1*H*-indazol-5-yl)-2-[4-(trifluoromethyl)phenyl]-4-quinazolinamine, 2-(3-chloro-4-fluorophenyl)-*N*-(1*H*-indazol-5-yl)-4-quinazolinamine, 2-(1,3-benzodioxol-5-yl)-*N*-(1*H*-indazol-5-yl)-4-quinazolinamine, *N*-(1*H*-indazol-5-yl)-2-(4-methylphenyl)-4-quinazolinamine, 2-(3,4-dichlorophenyl)-*N*-(1*H*-indazol-5-yl)-4-quinazolinamine, *N*-(1*H*-indazol-5-yl)-2-(1-naphthyl)-4-quinazolinamine, *N*-(1*H*-indazol-5-yl)-2-(3,4,5-trimethoxyphenyl)-4-quinazolinamine, 2-(1-benzofuran-2-yl)-*N*-(1*H*-indazol-5-yl)-4-quinazolinamine, *N*-(*1H*-indazol-5-yl)-2-(2-thienyl)-4-quinazolinamine , *N*-(1*H*-indazol-5-yl)-2-(3-thienyl)-4-quinazolinamine, 2-(3,5-dimethyl-4-isoxazolyl)-*N*-(1*H*-indazol-5-yl)-4-quinazolinamine, 2-(1,1'-biphenyl-4-yl)-*N-*(1*H*-indazol-5-yl)-4-quinazolinamine, 2-[4-(dimethylamino)phenyl]-*N*-(1*H*-indazol-5-yl)-4-quinazolinamine, 2-(1-benzothie*N*-2-yl)-*N*-(1*H*-indazol-5-yl)-4-quinazolinamine, 4-[4-(1*H*-indazol-5-ylamino)-2-quinazolinyl]phenol, 2-dibenzo[b,d]furan-1-yl-*N*-(1*H*-indazol-5-yl)-4-quinazolinamine, 2-(2-fluoro-1,1'-biphenyl-4-yl)-*N*-(1*H*-indazol-5-yl)-4-quinazolinamine, 7-chloro-*N*-(1*H*-indazol-5-yl)-2-phenyl-4-quinazolinamine, *N*-(1*H*-indazol-5-yl)-6-nitro-2-phenyl-4-quinazolinamine, 2-(4-fluorophenyl)-*N*-(1*H*-indazol-5-yl)-6-nitro-4-quinazolinamine, 6-chloro-*N*-(1*H*-indazol-5-yl)-2-(4-methylphenyl)-4-quinazolinamine, 6-chloro-2-(4-fluorophenyl)-*N*-(1*H*-indazol-5-yl)-4-quinazolinamine, 2-(4-bromophenyl)-6-chloro-*N*-(1*H*-indazol-5-yl)-4-quinazolinamine, *N*-(1*H*-indazol-5-yl)-2-(2-quinoxalinyl)-4-quinazolinamine, 5-fluoro-*N*-(1*H*-indazol-5-yl)-2-(2-methylphenyl)-4-quinazolinamine, 5-fluoro-2-(4-fluorophenyl)-*N*-(1*H*-indazol-5-yl)-4-quinazolinamine, 2-(3-chlorophenyl)-5-fluoro-*N*-(1*H*-indazol-5-yl)-4-quinazolinamine, 2-(4-bromophenyl)-5-fluoro-*N*-(1*H*-indazol-5-yl)-4-quinazolinamine, 5-fluoro-*N*-(1*H*-indazol-5-yl)-2-(3-methylphenyl)-4-quinazolinamine hydrochloride, 2-(3-bromophenyl)-5-fluoro-*N*-(1*H-*indazol-5-yl)-4-quinazolinamine hydrochloride, 2-(2-chlorophenyl)-5-fluoro-*N*-(1*H*-indazol-5-yl)-4-quinazolinamine, 5-fluoro-*N*-(1*H*-indazol-5-yl)-2-(2-quinoxalinyl)-4-quinazolinamine tris(trifluoroacetate), 5-fluoro-*N*-(1*H*-indazol-5-yl)-2-(1-naphthyl)-4-quinazolinamine bis(trifluoroacetate), 5-fluoro-*N*-(1*H*-indazol-5-yl)-2-(2-naphthyl)-4-quinazolinamine bis(trifluoroacetate), 5-fluoro-*N*-(1*H*-indazol-5-yl)-2-(4-pyridinyl)-4-quinazolinamine tris(trifluoroacetate), *N*-(1*H*-indazol-5-yl)-7-methyl-2-(2-quinoxalinyl)-4-quinazolinamine, 2-(3-chlorophenyl)-*N*-(1*H*-indazol-5-yl)-7-methyl-4-quinazolinamine, 2-(4-fluorophenyl)-*N*-(1*H*-indazol-5-yl)-7-methyl-4-quinazolinamine, *N*-(1*H*-indazol-5-yl)-7-methyl-2-(4-methylphenyl)-4-quinazolinamine", 2-(4-bromophenyl)-*N*-(1*H*-indazol-5-yl)-7-methyl-4-quinazolinamine, *N*-(1*H*-indazol-5-yl)-7-methyl-2-(2-methylphenyl)-4-quinazolinamine bis(trifluoroacetate), *N*-(1*H*-indazol-5-yl)-7-methyl-2-(3-methylphenyl)-4-quinazolinamine bis(trifluoroacetate), *N*-[2-(3-fluorophenyl)-7-methyl-4-quinazolinyl]-*N*-(1*H*-indazol-5-yl)amine bis(trifluoroacetate), 2-(3-bromophenyl)-*N*-(1*H*-indazol-5-yl)-7-methyl-4-quinazolinamine bis(trifluoroacetate), *N*-[2-(2-chlorophenyl)-7-methyl-4-quinazolinyl]-*N-*(1*H*-indazol-5-yl)amine bis(trifluoroacetate), 2-(3-furyl)-*N*-(1*H*-indazol-5-yl)-7-methyl-4-quinazolinaminebis(trifluotoacetate), *N*-(1*H*-indazol-5-yl)-7-methyl-2-(1-naphthyl)-4-quinazolinamine bis(trifluoroacetate), *N*-(1*H*-indazol-5-yl)-7-methyl-2-(2-naphthyl)-4-quinazolinamine bis(trifluoroacetate), *N*-(1*H*-indazol-5-yl)-7-methyl-2-(3-pyridinyl)4-quinazolinamine tris(trifluoroacetate), *N*-(1*H*-indazol-5-yl)-7-methyl-2-(4-pyridinyl)-4-quinazolinamine tris(trifluoroacetate), 7-chloro-2-(3-chloraphenyl)-*N*-(1*H*-indazol-5-yl)-4-quinazolinamine, 7-chloro-*N*-(1*H*-indazol-5-yl)-2-(4-methylphenyl)-4-quinazolinamine , 2-(4-bromophenyl)-7-chloro-*N*-(1*H*-indazol-5-yl)-4-quinazolinamine, 7-chloro-*N*-(1*H-*indazol-5-yl)-2-(3-methylphenyl)-4-quinazolinamine hydrochloride, 7-chloro-2-(3-fluorophenyl)-*N*-(1*H*-indazol-5-yl)-4-quinazolinamine bis(trifluoroacetate), 2-(3-bromophenyl)-7-chloro-*N*-(1*H*-indazol-5-yl)-4-quinazolinamine bis(trifluoroacetate), *N-*[7-chloro-2-(2-furyl)-4-quinazolinyl]-*N*-(1*H*-indazol-5-yl)amine bis(trifluoroacetate), 7-chloro-*N*-(1*H*-indazol-5-yl)-2-(2-quinoxalinyl)-4-quinazolinamine tris(trifluoroacetate), 7-chloro-*N*-(1*H*-indazol-5-yl)-2-(1-naphthyl)-4-quinazolinamine bis(trifluoroacetate), 7-chloro-*N*-(1*H*-indazol-5-yl)-2-(2-naphthyl)-4-quinazolinamine bis(trifluoroacetate), 7-chloro-*N*-(1*H-*indazol-5-yl)-2-(3-pyridinyl)-4-quinazolinamine tris(trifluoroacetate), 2-(4-fluorophenyl)-N-(1*H*-indazol-5-yl)-6,7-dimethoxy-4-quinazolinamine, 2-(1,1'-biphenyl-4-yl)-*N*-(1*H*-indazol-5-yl)-6,7-dimethoxy-4-quinazolinamine, *N*-(1*H*-indazol-5-yl)-6,7-dimethoxy-2-(4-vinylphenyl)-4-quinazolinamine, *N*-cyclopentyl-4-(1*H*-indazol-5-ylamino)-2-quinazolinecarboxamide, *N*-(3-fluorophenyl)-*N*-[4-(1*H*-indozol-5-ylamino)-6,7-dimethoxy-2-quinazolinyl]amine, *N*-(2,4-difluorobenzyl)-*N*-[4-(1*H*-indazol-5-ylamino)-6,7-dimethoxy-2-quinazolinyl]amine, *N*-(2-fluorobenzyl)-*N*-[4-(1*H*-indazol-5-ylamino)-6,7-dimethoxy-2-quinazolinyl]amine, *N*-(4-bromophenyl)-*N*-[4-(1*H*-indazol-5-ylamino)-6,7-dimethoxy-2-quinazolinyl]amine, *N*-(6,7-dimethoxy-2-{[4-(trifluoromethyl)phenyl]amino}-4-quinazolinyl)-*N*-(1*H*-indazol-5-yl)amine, *N*-(6,7-dimethoxy-2-{[4-(trifluoromethyl)benzyl]amino}-4-quinazolinyl)-*N*-(1*H*-indazol-5-yl)amine, *N*-[3-fluoro-5-(trifluoromethyl)benzyl]-*N*-[4-(1*H*-indazol-5-ylamino)-6,7-dimethoxy-2-quinazolinyl]amine, *N*-(3-fluorobenzyl)-*N*-[4-(1*H*-indazol-5-ylamino)-6,7-dimethoxy-2-quinazolinyl]amine, *N*-(2,4-difluorobenzyl)-*N*-[4-(1*H*-indazol-5-ylamino)-6,7-dimethoxy-2-quinazolinyl]amine, *N*-(4-fluorobenzyl)-*N*-[4-(1*H*-indazol-5-ylamino)-6,7-dimethoxy-2-quinazolinyl]amine, *N*-(2,6-difluorobenzyl)-*N*-[4-(1*H*-indazol-5-ylamino)-6,7-dimethoxy-2-quinazolinyl]amine, *N*-(3,5-difluorobenzyl)-*N*-[4-(1*H*-indazol-5-ylamino)-6,7-dimethoxy-2-quinazolinyl]amine, *N*-(3-bromophenyl)-*N*-[4-(1*H*-indazol-5-ylamino)-6,7-dimethoxy-2-quinazolinyl]amine, *N*-(2,6-difluorophenyl)-*N*-[4-(1*H*-indazol-5-ylamino)-6,7-dimethoxy-2-quinazolinyl]amine, *N*-(2,5-difluorophenyl)-*N*-[4-(1*H*-indazol-5-ylamino)-6,7-dimethoxy-2-quinazolinyl]amine, *N*-(2,4-difluorophenyl)-*N*-[4-(1*H*-indazol-5-ylamino)-6,7-dimethoxy-2-quinazolinyl]amine, *N*-(2,3-difluorophenyl)-*N*-[4-(1*H*-indazol-5-ylamino)-6,7-dimethoxy-2-quinazolinyl]amine, *N*-(3,4-difluorophenyl)-*N*-[4-(1*H*-indazol-5-ylamino)-6,7-dimethoxy-2-quinazolinyl]amine, *N*-(3,5-difluorophenyl)-*N*-[4-(1*H*-indazol-5-ylamino)-6,7-dimethoxy-2-quinazolinyl] amine, *N*-{6,7-dimethoxy-2-[(2,3,4-trifluorophenyl)amino]-4-quinazolinyl}-*N*-(1*H*-indazol-5-yl)amine, *N*-{6,7-dimethoxy-2-[(2,4,5-trifluorophenyl)amino]-4-quinazolinyl}-*N*-(1*H*-indazol-5-yl)amine, *N*-{6,7-dimethoxy-2-[(2,4,6-trifluorophenyl)amino]-4-quinazolinyl}-*N*-(1*H*-indazol-5-yl)amine, *N*-{6,7-dimethoxy-2-[(2,3,6-trifluorophenyl)amino]-4-quinazolinyl}-*N*-(1*H*-indazol-5-yl)amine, *N-*(4-bromophenyl)-*N*-[4-(1*H*-indazol-5-ylamino)-6,7-dimethoxy-2-quinazolinyl]amine, 2-(3-aminophenyl)-*N*-(1*H*-indazol-5-yl)-4-quinazolinamine, *N*-{3-[4-(1*H*-indazol-5-ylamino)-2-quinazolinyl]phenyl}isonicotinamide, *N*-{3-[4-(1*H*-indazol-5-ylamino)-2-quinazolinyl]phenyl}acetamide, *N*-(4-chlorophenyl)-*N*-[4-(1*H*-indazol-5-ylamino)-2-quinazolinyl]amine, *N*-(3-bromophenyl)-*N*-[4-(1*H*-indazol-5-ylamino)-2-quinazolinyl]amine, *N*-(2-chlorophenyl)-*N*-[4-(1*H*-indazol-5-ylamino)-2-quinazolinyl]amine, *N*-(3-fluorophenyl)-*N*-[4-(1*H*-indazol-5-ylamino)-2-quinazolinyl]amine, *N*-(2-fluorophenyl)-*N*-[4-(1*H*-indazol-5-ylamino)-2-quinazolinyl]amine, *N*-(3-chlorophenyl)-*N*-[4-(1*H*-indazol-5-ylamino)-2-quinazolinyl]amine, *N*-(4-bromophenyl)-*N*-(4-(1*H*-indazol-5-ylamino)-2-quinazolinyl)amine, *N*-(1*H*-indazol-5-yl)-*N*-(2-{[3-(trifluoromethyl)phenyl]amino}-4-quinazolinyl)amine, *N*-(1*H*-indazol-5-yl)-*N-*{2-[(4-Phenoxyphenyl)amino]-4-quinazolinyl}amine, *N*-(1*H*-indazol-5-yl)-*N*-(2-{[4-(trifluoromethoxy)phenyl]amino}-4-quinazolinyl)amine, *N*-(1*H*-indazol-5-yl)-*N*-(2-{[3-(trifluoromethoxy)phenyl]amino}-4-quinazolinyl)amine, *N*-(4-fluorophenyl)-*N*-[4-(1*H-*indazol-5-ylamino)-2-quinazolinyl]amine, *N*-(2-anilino-4-quinazolinyl)-*N*-(1*H*-indazol-5-yl)amine, 2-[4-(2-chlorophenyl)-1-piperazinyl]-*N*-(1*H*-indazol-5-yl)-4-quinazolinamine, *N-*(1*H*-indazol-5-yl)-2-[4-(2-pyrimidinyl)-1-piperazinyl]-4-quinazolinamine, *N*-(1*H*-indazol-5-yl)-2-[4-(2-methoxyphenyl)-1-piperazinyl]-4-quinazolinamine, 1-(4-{4-[4-(1*H*-indazol-5-ylamino)-2-quinazolinyl]-1-piperazinyl}phenyl)ethanone, 4-(1*H*-indazol-5-ylamino)-2-quinazolinecarboxamide", 4-(1*H*-indazol-5-ylamino)-*N*-(4-pyridinyl)-2-quinazolinecarboxamide, 4-(1*H*-indazol-5-ylamino)-*N*-(4-methoxyphenyl)-2-quinazolinecarboxamide, *N*-cyclohexyl-4-(1*H*-indazol-5-ylamino)-2-quinazolinecarboxamide, *N*-cyclopentyl-4-(1*H*-indazol-5-ylamino)-2-quinazolinecarboxamide, 4-(1*H*-indazol-5-ylamino)-*N*-(2-pyridinyl)-2-quinazolinecarboxamide, 4-(1*H*-indazol-5-ylamino)-*N*-(3-quinolinyl)-2-quinazolinecarboxamide, 4-(1*H*-indazol-5-ylamino)-*N*-methyl-2-quinizolinecarboxamide, *N-*(1*H*-indazol-5-yl)-2-(4-morpholinylcarbonyl)-4-quinazolinamine, 2-(2,3-dihydro-1-benzofuran-5-yl)-*N*-(1*H*-indazol-5-yl)-4-quinazolinamine, 2-cyclopropyl-*N*-(1*H*-indazol-5-yl)-4-quinazolinamine, *N*-(1*H*-indazol-5-yl)-2-(trifluoromethyl)-4-quinazolinamine, 2-chloro-*N*-(3-ethyl-1*H-*indazol-5-yl)-4-quinazolinamine, 2-(2-fluoro-1,1'-biphenyl-4-yl)-*N*-(1*H*-indazol-5-yl)-4-quinazolinamine dihydrochloride, 2-(2-fluoro-1,1'-biphenyl-4-yl)-*N*-(1*H*-indazol-5-yl)-4-quinazolinamine dimethanesulfonate, 2-(2-fluoro-1,1'-biphenyl-4-yl)-*N*-(1*H*-indazol-5-yl)-4-quinazolinamine benzenesulfonate, 2-(2-fluoro-1,1'-biphenyl-4-yl)-*N*-(1*H*-indazol-5-yl)-4-quinazolinamine 4-methylbenzenesulfonate, 2-dibenzo[*b*,*d*]furan-1-yl-*N*-(1*H*-indazol-5-yl)-4-quinazolinamine trifluoroacetate, 2-chloro-*N*-(1*H*-indazol-5-yl)-4-quinaxolinamine,
2-(2-furyl)-*N*-(1*H*-indazol-5-yl)-7-methyl-4-quinazolinamine; 2-(1-benzofuranyl)-*N*-(1*H*-indazol-5-yl)-6,7-dimethoxy-4-quinazolinamine; *N*-(2,5-difluorobenzyl)-*N*-[4-(1*H*-indazol-5-ylamino)-6,7-dimethoxy-2-quinazolinyl]amine; *N-*{6,7-dimethoxy-2-[(2,3,5-trifluorophenyl)amino]-4-quinazolinyl}-*N*-(1*H*-indazol-5-yl)amine; *N*-(1*H*-indazol-5-yl)-2-[5-(1*H*-indolyl)amino]-4-quinazolinamine; *N*-(1*H-*indazol-5-yl)-2-[4-phenoxyanilino]-4-quinazolinamine; *N*-(1*H*-indazol-5-yl)-2-[2-naphthylamino]-4-quinazolinamine, *N*-(1*H*-indazol-5-yl)-2-(trifluoromethyl)-4-quinazolinamine ; and 2-chloro-*N*-(1*H*-indazol-5-yl)-4-quinazolinamine.

The invention moreover encompasses treating indications mediated by Rho-kinase, by administering a compound of Formula I, or a pharmaceutical composition containing a compound of Formula I. Thus, the invention encompasses treating cardiovascular diseases such as hypertension, artherosclerosis, restenosis and cerebral ischemia, or vasospasrn central nervous system disorders such as neuronal degeneration and spinal cord injury, erectile dysfunction, e.g., in patients who do not have satisfactory response to PDE-5 inhibitors, and cancer (e.g., tumor growth) mediated by Rho-kinase, by administering, e.g., to a host in need thereof, of an effective amount of a compound of Formula I. Cancers and tumors mediated by Rho-kinase include cancers of the breast, colon, prostate, ovaries, brain and lung and their metastases.

The compounds may be administered orally, topically, parenterally, by inhalation or spray, vaginally, rectally or sublingually in dosage unit formulations. The term 'administration by injection' includes intravenous, intraarticular, intramuscular, subcutaneous and parenteral injections, as well as use of infusion techniques. Dermal administration may include topical application or transdermal administration. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing the active materials in admixture with excipients suitable for the manufacture of aqueous suspensions may also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or *n*-propyl *p*-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Compounds of the invention may also be administrated transdermally using methods known to those skilled in the art (see, for example: Chien; "Transdermal Controlled Systemic Medications"; Marcel Dekker, Inc.; 1987. Lipp et al. WO94/04157 3Mar94). For example, a solution or suspension of a compound of Formula I in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of a compound of Formula I may be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery system are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, n-butyl, sec-butyl, isobutyl, tertbutyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures of one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene copolymers, and natural and synthetic rubbers. Cellulose ethers, derivatized polyethylenes, and silicates may also be used as matrix components. Additional additives, such as viscous resins or oils may be added to increase the viscosity of the matrix.

Pharmaceutical compositions of the invention may also be in the form of oil-in-water emulsions. The oil phase may be a vegetable oil, for example olive oil or arachis oil, or a mineral oil, for example, liquid paraffin or mixtures of these. Suitable emulsifying agents may be naturally-occurring gums, for example, gum acacia or gum tragacanth, naturally-occurring phosphatides, for example, soy bean, lecithin, and esters or partial esters derived from fatty acids and hexitol anhydrides, for example, sorbitan monooleate, and condensation products of the said partial esters with ethylene oxide, for example, polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal or vaginal administration of the drug. These compositions can be prepared by mixing the drug with a suitable non-irritating excipient which is solid at ordinary temperatures but liquid at the rectal temperature or vaginal temperature and will therefore melt in the rectum or vagina to release the drug. Such materials include cocoa butter and polyethylene glycols.

Moreover, for treatment of erectile dysfunction, the present pharmaceutical compositions may take any form which is suitable for administration to the penis either via injection into the corpora cavernosa or transurethral administration, or topically applied to the urethral meatus. In the case of injection into the corpora cavernosa, the pharmaceutical composition is suitably in the form of a saline solution. Preferably, the pharmaceutical composition is in a form suitable for transurethral administration, and in this case the composition is typically in the form of a solution, an ointment, or a suppository. Typically, the pharmaceutical composition is administered 1 to 50 minutes, preferably 10 to 20 minutes, prior to the time of commencing sexual intercourse.

For all regimens of use disclosed herein for compounds of Formula I, the daily oral dosage regimen will preferably be from 0.01 to 200 mg/Kg of total body weight. The daily dosage for administration by injection, including intravenous, intramuscular, subcutaneous and parenteral injections, and use of infusion techniques will preferably be from 0.01 to 200 mg/Kg of total body weight. The daily vaginal dosage regime will preferably be from 0.01 to 200 mg/Kg of total body weight. The daily topical dosage regimen will preferably be from 0.01 to 200 mg administered between one to four times daily. The transdermal concentration will preferably be that required to maintain a daily dose is of from 0.1 to 200 mg/Kg. The daily inhalation dosage regimen will preferably be from 0.01 to 10 mg/Kg of total body weight.

It will be appreciated by those skilled in the art that the particular method of administration will depend on a variety of factors, all of which are considered routinely when administering therapeutics. It will also be understood, however, that the specific dose level for any given patient will depend upon a variety of factors, including, the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy. It will be further appreciated by one skilled in the art that the optimal course of treatment, i.e., the mode of treatment and the daily number of doses of a compound of Formula I or a pharmaceutically acceptable salt thereof given for a defined number of days, can be ascertained by those skilled in the art using conventional treatment tests.

The present compounds and compositions exhibit Rho-kinase inhibitory activity, and are thus useful to treat the indications listed above, e.g., indications mediated by Rho-kinase. By indications mediated by Rho-kinase is meant diseases or conditions whose progression proceeds, at least in part, via the Rho pathway.

Rho-kinase inhibitory activity, e.g., ROCK-1 inhibition, can be evaluated as follows:

The kinase domain of human ROCK-1, amino acids 27-530, is isolated as a glutathione S-transferase fusion protein from Sf9 insect cells. The protein is partially purified by glutathione Sepharose 4B (Pharmacia Biotech, Piscataway, NJ) affinity purification. Reactions is carried out in 96-well plates in a total volume of 100 uL containing 50 mM N-[2-Hydroxyethyl]piperazine-N'-[2-ethanesulfonic acid] pH 7.5, 5 mM MgCl₂, 1 mM dithiothreitol, 6 µM ATP, 0.2 µCi [³³P]ATP (NEN, Boston, MA), 1 µg myelin basic protein and 0.1 µg ROCK-1. Test compounds are dissolved in 100% dimethylsulfoxide, diluted to the appropriated concentration and added to the reaction. The final concentration of dimethylsulfoxide did not exceed 0.5%. The reaction is run for one hour at room temperature. The reaction is stopped with the addition of 7 mL of 1 N HCL, transferred to P30 membranes and the amount of [³³P]ATP, as counts per minute (c.p.m.) incorporated into the substrate, myelin basic protein, is read in a BetaPlate Reader (Packard Instrument Co., Meriden, CT.). (All reagents were purchased from Sigma Chemical Co., St. Louis, MO unless stated otherwise.) Percentage inhibition is measured by the amount of incorporation of radioactivity in the presence of the test compound when compared to the amount of incorporation in the absence of the test compound.

Inhibitory activity can also be evaluated by measurement of stress fiber formation, performed essentially as described by Ridley, A.J., and A. Hall, Cell 70:389-399 (1992). Human fibrosarcoma HT1080 (CCL-121, American Type Culture Collection, Manassas, VA) cells are plated on 22 X 22 mm #1 glass cover slips in six-well tissue culture plates (Costar) at 2.5 X 10⁴ cells/well in Delbeco's modified Eagle's Medium (DMEM, Gibco) supplemented with 10% fetal calf serum. Cells are maintained in a humidified, 5% CO₂ atmosphere at 37°C. After 24 hours the culture medium is removed and replaced with medium without 10% fetal calf serum and the cells cultured for an additional 48 hours. Test compounds are dissolved in 100% dimethylsulfoxide, diluted to the appropriated concentration and added to the culture medium 60 minutes prior to the induction of stress fiber formation. The final concentration of dimethylsulfoxide did not exceed 0.25%. Stress fiber formation is induced by the addition of lysophosphatidic acid (1-oleoyl-2-hydroxy-*sn-*glycerol-3-phosphate, Avanti Polar-Lipids, Alabaster, Al) to 10 µM final concentration in Delbeco's modified Eagle's Medium containing 0.1% fatty acid free bovine serum albumin for 15 minutes at 37°C. Cells are fixed with 4% paraformaldeyhde (Poly Scientific, Bay Shore, NJ) in phosphate buffered saline (PBS) for 15 minutes. Cells are then washed 3 times in PBS and them permeabilized using a solution containing 40mM piperazine-N-N'bis[2-ethanesulfonic acid], 50 mM N-[2-hydoryethyl]piperaxine-N'-[2-ethanesulfonic acid], 0.1% Triton X-100, 75 mM NaCl, mM MgCl₂, 0.5 mM EGTA, pH 7.2 for 2 minutes at room temperature. The cells are washed 3 times for 5 minutes each in PBS and then actin stress fibers are stained using 10 units/mL rhodamine phalloidin (Molecular Probes, Eugene, OR) in PBS for 60 minutes at room temperature. The cells are washed 3 times with PBS and the cover slips mounted on glass microscope slides. The percentage of stress fiber positive cells on each slide was determined visually using a Nikon Labphoto-2 microscope. At least 100 cells were counted per slide and experiments were done in duplicate. Percentage inhibition is measured by counting the number of stress fiber positive cells in the presence of the test compound when compared to the number of stress fiber positive cells in the absence of the test compound.

Using the above protocols, all of the compounds as disclosed herein are determined to have Rho-kinase inhibitory activity.

The compounds of the invention can be made according to routine, conventional chemical methods, and/or as disclosed below, from starting materials which are either commercially available or produceable according to routine, conventional chemical methods. General methods for the preparation of the compounds are given below, and the preparation of representative compounds is specifically illustrated in the Examples.

### ABBREVIATIONS AND ACRONYMS

When the following abbreviations are used herein, they have the following meaning:
- Ac₂O: acetic anhydride
- anhy: anhydrous
- *n*-BuOH: n-butanol
- *t*-BuOH: *t*-butanol
- CD₃OD: methanol-*d*_{*4*}
- Celite®: diatomaceous earth filter agent, ® Celite Corp.
- CH₂Cl₂: methylene chloride
- CI-MS: chemical ionization mass spectroscopy
- conc: concentrated
- dec: decomposition
- DME: dimethoxyethane
- DMF: *N,N*-dimethylformamide
- DMSO: dimethylsulfoxide
- ELSD: evaporative light scattering detector
- EtOAc: ethyl acetate
- EtOH: ethanol (100%)
- Et₂O: diethyl ether
- Et₃N: triethylamine
- HPLC ES-MS: high performance liquid chromatography-electrospray mass spectroscopy
- NMM: 4-methylmorpholine
- Ph₃P: triphenylphosphine
- Pd(dppf)Cl₂: [1,1'-bis(diphenylphosphino)ferrocene]dichloropalladium(II)
- Pd(PPh₃)₄: tetrakis(triphenylphosphine)palladium(0)
- Pd(OAc)₂: palladium acetate
- P(O)Cl₃: phosphorous oxychloride
- R_{*f*}: TLC retention factor
- RT: retention time (HPLC0
- rt: room temperature
- THF: tetrahydrofuran
- TFA: trifluoroacetic acid
- TLC: thin layer chromatography

### General Methods of Preparation

### General Method A

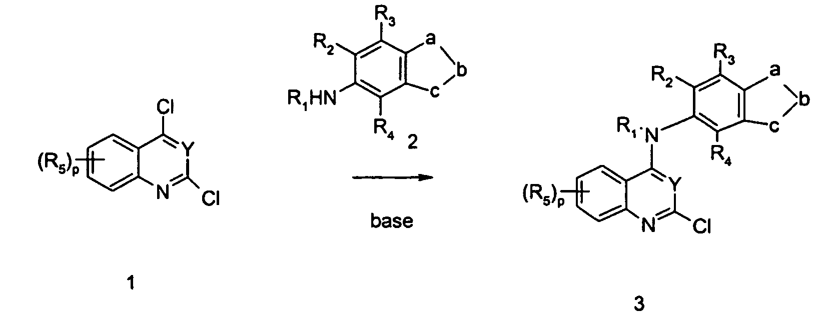

A mixture of compounds. 1 and 2, and potassium acetate in THF/water is stirred at room temperature overnight. Water is added to the mixture resulting in the formation of a precipitate. The precipitate is washed with water, filtered, and dried under high vacuum to afford 3.

### General Method B

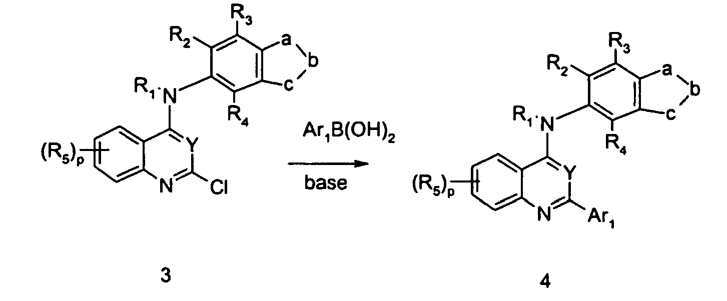

A mixture of compound 3, ethylene glycol dimethyl ether/water, Aryl boronic acid and sodium bicarbonate is degassed with argon for 15 minutes and Pd(dppf)Cl₂ is added. The mixture is heated to reflux overnight. After cooling to rt CH₂Cl₂ and H₂O are added to the mixture. The organic and aqueous layers are separated and the aqueous layer is extracted with CH₂Cl₂, and the combined organic layers are dried over anhydrous sodium sulfate. The organic solvent is removed under reduced pressure and the crude product is purified by silica gel chromatography of HPLC to afford compound 4.

### General Method C

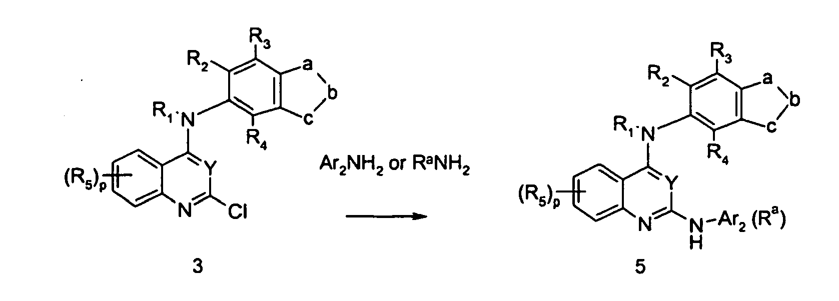

A mixture of compound. 3 and a substituted amine or aniline, is heated to 140°C for 2 hours. The mixture is cooled to room temperature and is treated with ether to form precipitate or is purified by silica gel column chromatography. Purification of precipitate: The precipitate is filtered, washed with ether several times, and is dried under high vacuum to provide product.

It is to be understood that the specific conditions selected from these General Methods A-C will depend on the particular structures of the starting materials chosen, in order to optimized the yield of the products desired.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius; and, unless otherwise indicated, all parts and percentages are by weight.

The entire disclosure of all applications, patents and publications, cited above or below, including U.S. Provisional Application No. 60/277,974, filed March 23, 2001 and U.S. Provisional Application No. 60/315,341, filed August 29, 2001, are hereby incorporated by reference.

### EXAMPLES

### Example 1

### Preparation of N-[2-(2,4-dichlorophenyl)-4-quinazolinyl]-N-(1H-indazol-5-yl)amine

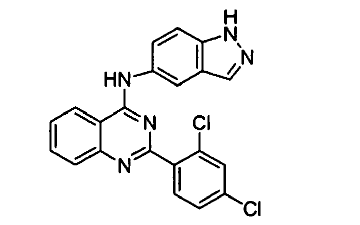

### Step1: Preparation of 2,4-Dichloroquinazoline

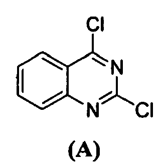

A solution of P(O)Cl₃ (800 mL) and DMF (4 mL) stirred at room temperature for 20 min and is added to a flask containing benzoyleneurea (200 g). The mixture is heated to reflux overnight. The brown solution is cooled to 50°C, poured into cold water (0°C, 8000 mL) while stirring vigorously. The aqueous mixture is maintained at a temperature below 30°C during the quench. The cold precipitate is filtered, washed with cold water (3 x 1200 mL) and dried under high vacuum at 40°C to afford 174 g of Intermediate A (71%).

### Step 2: Preparation of 2-N-5'-aminoindazole-4-chloroquinazoline

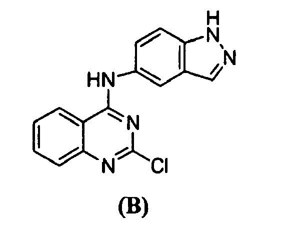

A mixture of 2,4-dichloroquinazoline (Intermediate **A**, 174 g, 0.874 mol), 5-aminoindazole (130 g, 0.98 mol), and potassium acetate (111.5 g, 1.14 mol) in THF/water (2 L/0.9 L) is stirred at room temperature overnight. Water (2 L) is added to the mixture resulting in the formation of a precipitate. The precipitate is washed with water, filtered, and dried under high vacuum to afford Intermediate **B** (241 g, 0.8 mol, 92%) as a gray powder.

### Step 3: Preparation of N-[2-(2,4-dichlorophenyl)-4-quinazolinyl]-N-(1H-indazol-5-yl)amine

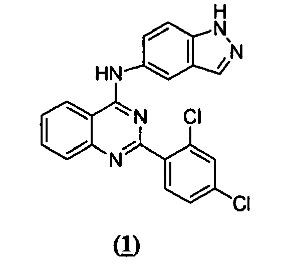

A mixture of 2-*N*-5'-aminoindazole-4-chloroquinazoline (0.21 g), ethylene glycol dimethyl ether/water (50 mL/6 mL), 2,4-dichlorophenyl boronic acid (0.11 g) and sodium bicarbonate (0.18 g) is degassed with argon for 15 minutes and Pd(dppf)Cl₂ (0.042 g) is added. The mixture is heated to reflux overnight. After cooling to rt CH₂Cl₂ (100 mL) and H₂O (50 mL) were added to the mixture. The organic and aqueous layers were separated and the aqueous layer is extracted with CH₂Cl₂ (2x75 mL), and the combined organic layers were dried over anhydrous sodium sulfate. The organic solvent is removed under reduced pressure and the crude product is purified by silica gel chromatography to afford Example **1** (0.08 g). Rf = 0.52 (CH₂Cl₂/MeOH = 95/5). ¹H NMR (CD₃OD) δ 8.44 (1H, dd, J = 2.7 Hz), 8.23 (1H, s), 8.01 (1H, s), 7.89-7.85 (2H, m), 7.84-7.78 (1H, m), 7.73-7.65 (2H, m), 7.58-7.53 (2H, m), 7.43 (1H, dd, J = 1.2, 2.7 Hz).

### Preparation of Examples 2 - 24

Using an analogous procedure to that described for Example **1**, Intermediate **B** (prepared in as described in Step 2) is reacted with the appropriate substituted boronic acid Ar₁B(OH)₂ to give the compounds of Examples **2-24** described in Table 1 below:

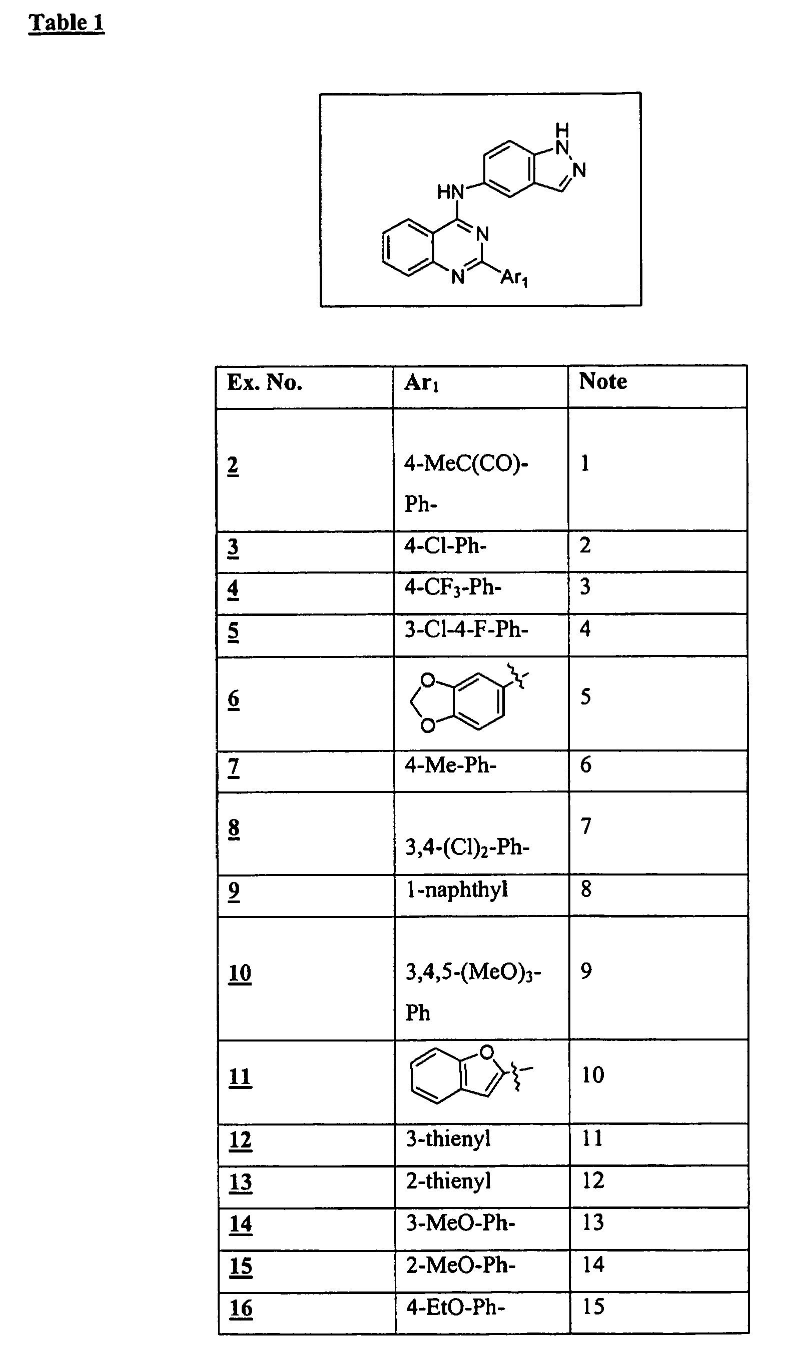

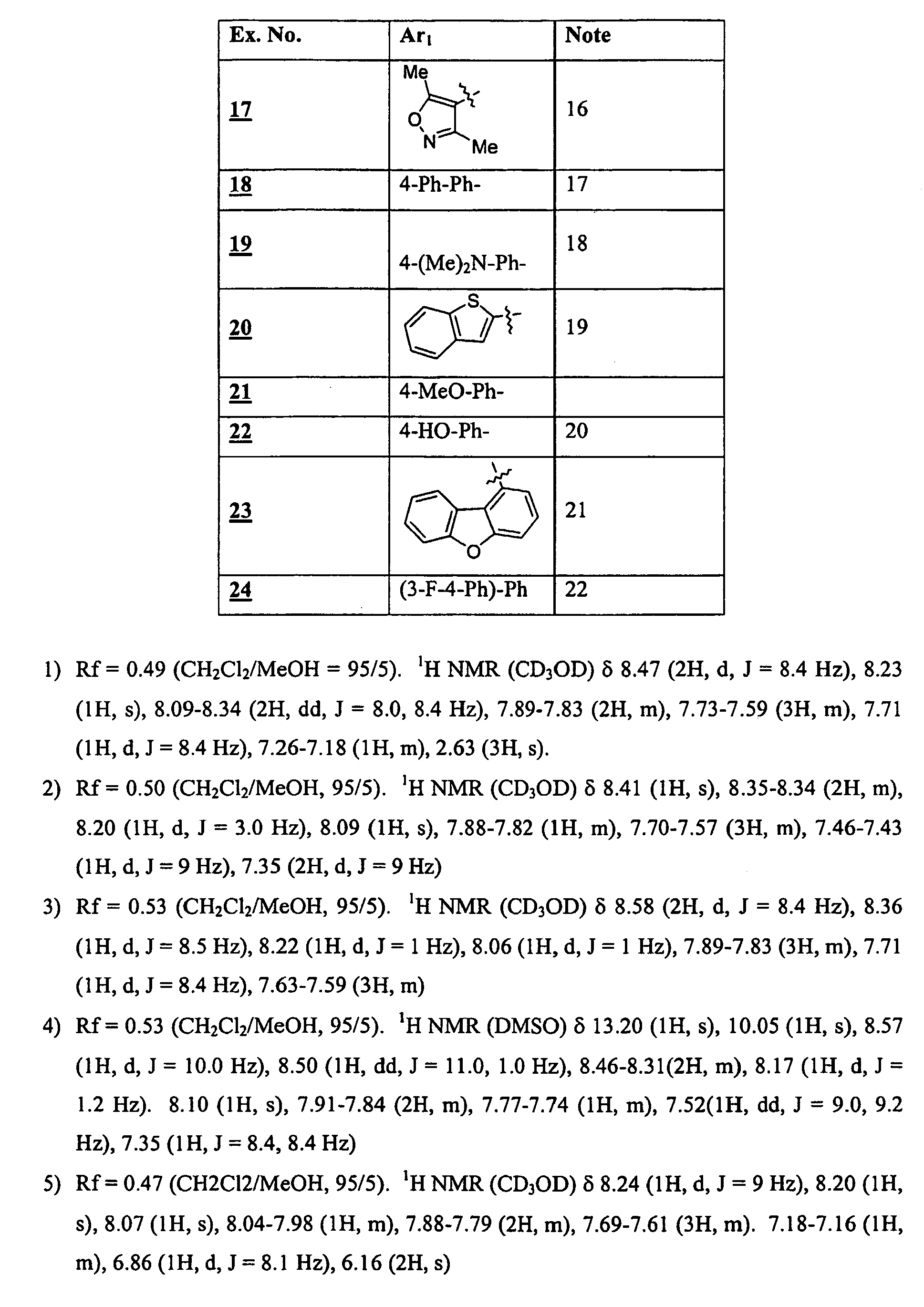

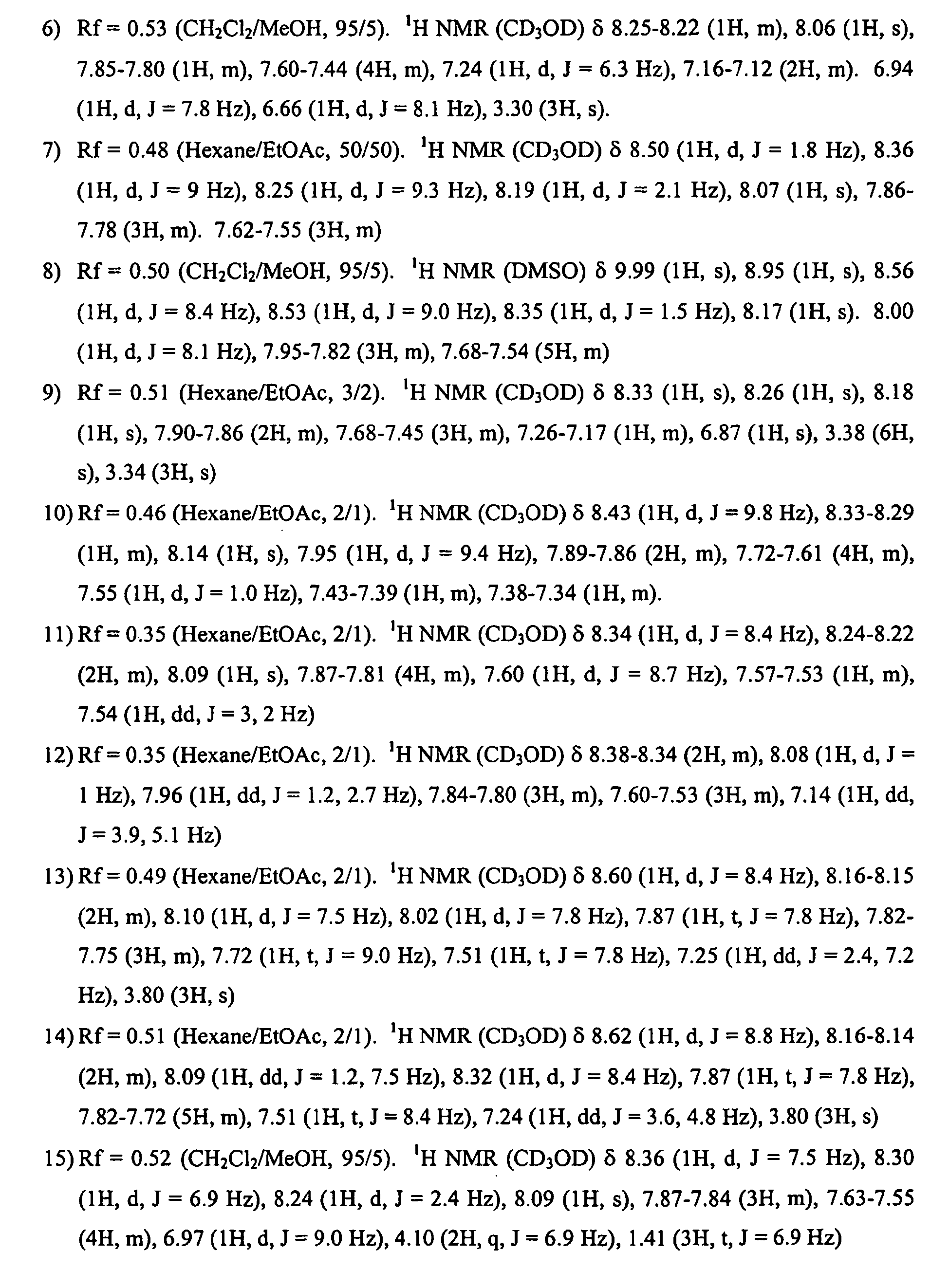

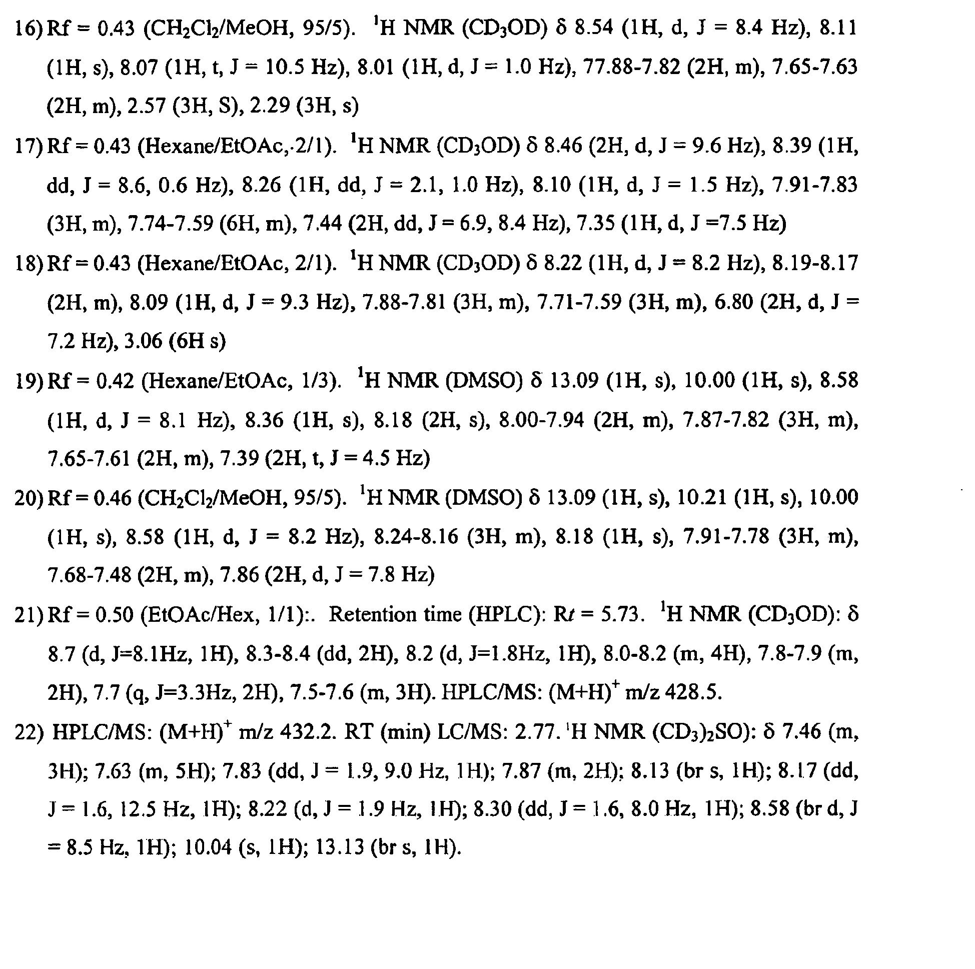

### Intermediate C1

### Preparation of 4,6-dichloro-2-phenylquinazoline

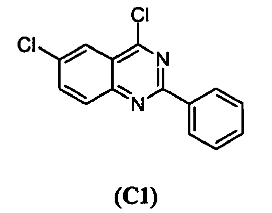

### Step 1: Preparation of N,N, dimethylbenzamides

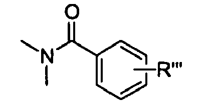

To a solution of dimethylamine (excess) in THF is added a substituted benzoyl chloride dropwise at 0 °C. The reaction mixture is stirred at room temperature for 2 hours. After removal of the solvent under reduced pressure the residue is dissolved EtOAc, and washed with water (3x). The organic layer is concentrated in vacuo and the crude product is either used directly or purified by silica gel chromatography (gradient from 10% to 50% ethyl acetate/ hexane).

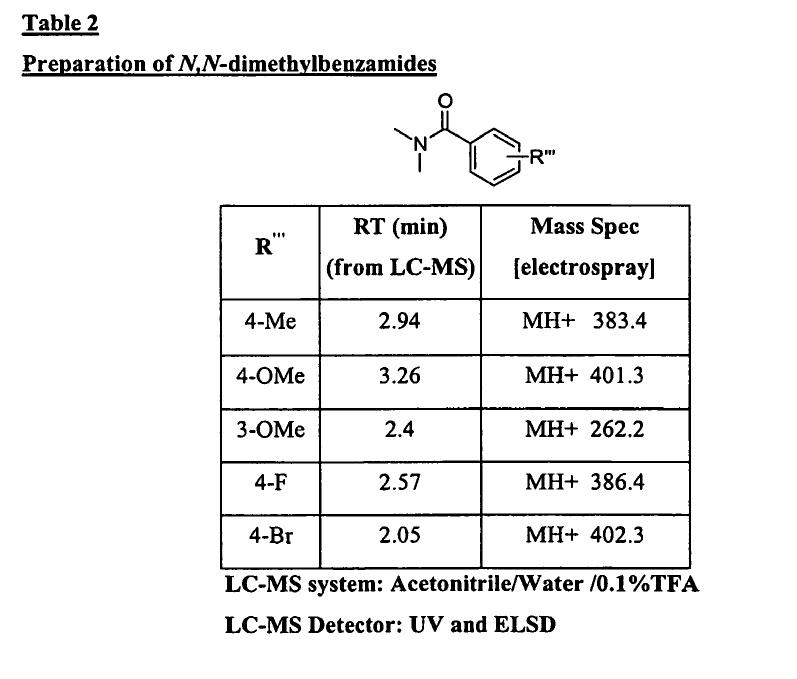

### Step 2: Preparation of 4,6-Dichloro-2-arylquinazolines

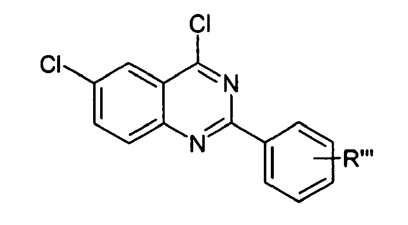

A solution of a substituted *N,N*-dimethylbenzamide (1.17g, 7.9mmol) and POCl₃ (3.0g, 19.7mmol) is stirred at 0 °C for 30 minutes. To this mixture is added 5-chloro-2-amino-benzonitrile (1.0g, 6.6mmol) and CH₂Cl₂ (5.0ml). The reaction mixture is stirred at 40 °C for 18 hours. The mixture is poured into ice water, basified to pH 9 with NaHCO₃, and extracted with CH₂Cl₂. The organic layer is dried over MgSO₄ and concentrated in vacuo. The crude product is purified by silica gel column (ethyl acetate/hexane, 10/90). Thus is obtained the Intermediate **C1,** (R = H) (0.45g, 25%) as pale yellow powder. HPLC/MS: (M+H)⁺ 275.2 *m*/*z*. Retention time (HPLC/MS) = 3.97 min.

Using the same procedure described above for Intermediate **C1** and substituting the appropriate benzamide intermediate starting material, Intermediates **C2** to **C6** were similarly prepared and are summarized in Table 3:

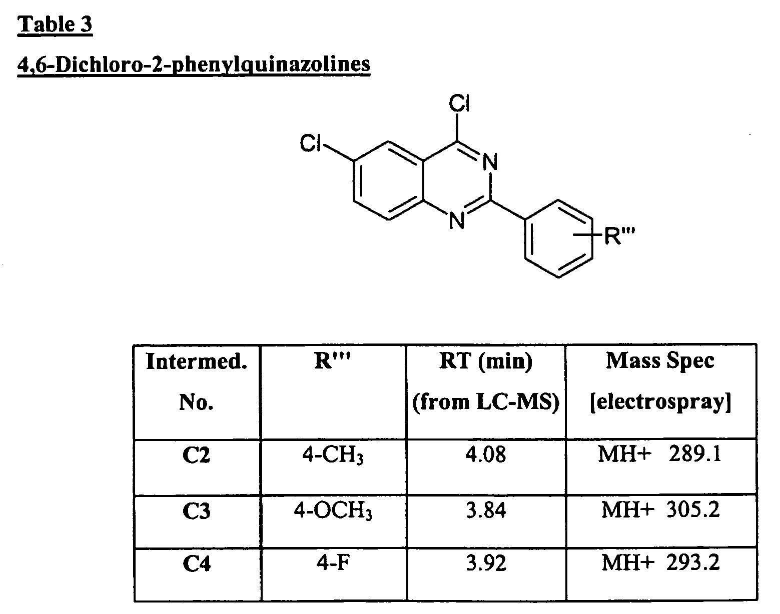

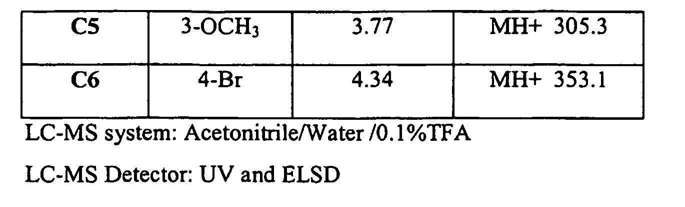

### Example 25

### Preparation of 7-chloro-N-(1H-indazol-5-yl)-2-(4-methylphenyl)-4-quinazolinamine

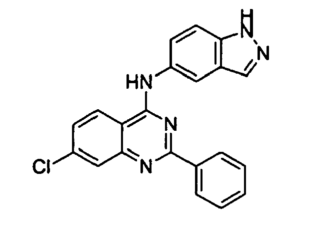

A mixture of 4,7-dichloro-2-phenylquinazoline (20 mg, 0.05mmol) and 5-aminoindazole (7.5 mg, 0.06mmol) in butanol (2.0ml) is heated to 100°C overnight. After removal of solvent in vacuo the crude product is purified by silica gel column chromatography (gradient from 20% to 80% ethyl acetate/ hexane) to afford Example **25** (15.2mg). HPLC/MS: (M+H)⁺ 372.4 *m*/*z*. Retention time (HPLC/MS) = 2.53 min.

Using the method described for Example **25** and using the appropriate substituted 4-chloro-2-arylquinazoline and 5-aminoindazole as starting materials, Examples **26-32** were similarly prepared and are summarized in Table 4 below:

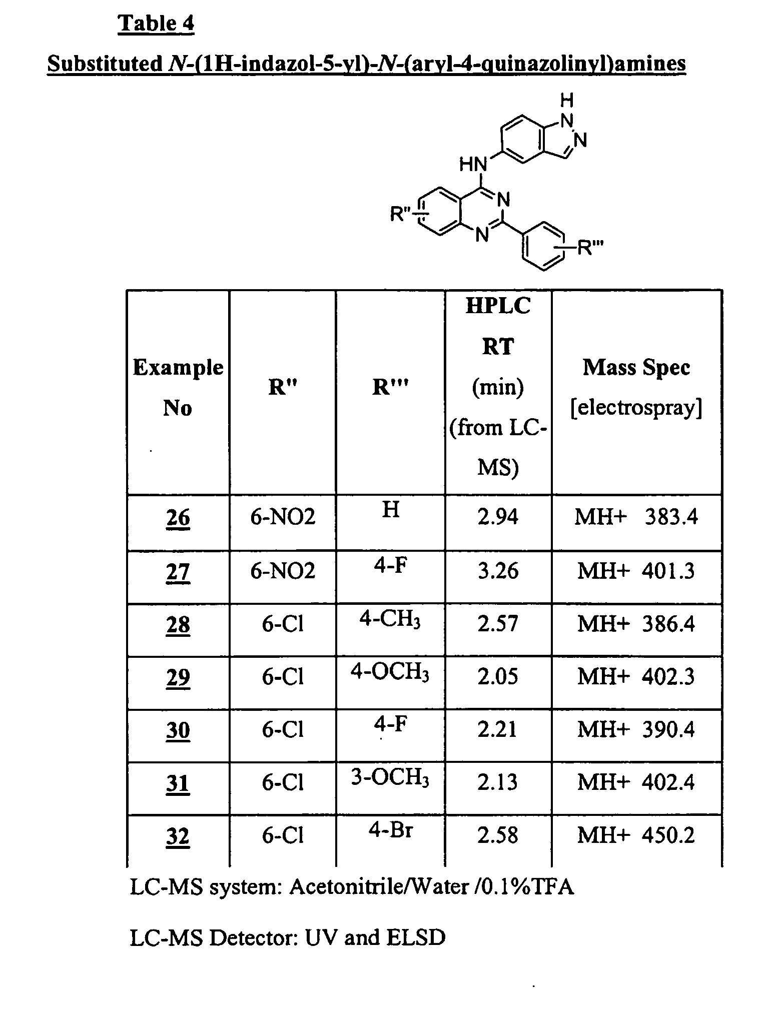

### General Synthetic Scheme for Example 33

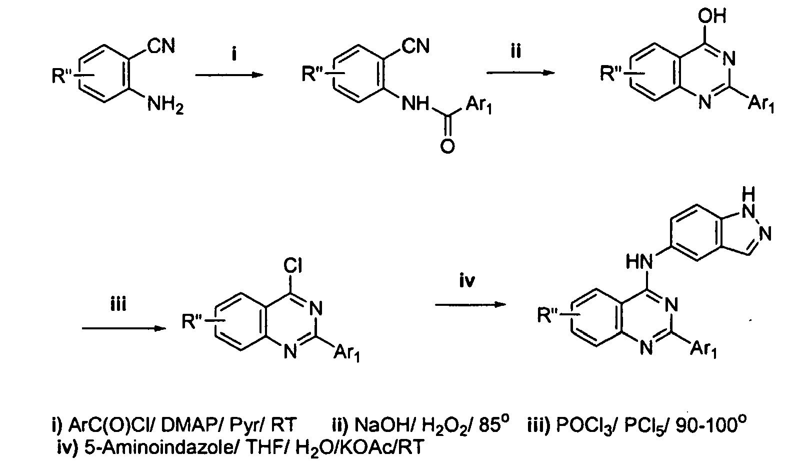

### Example 33

### Preparation of N-(1H-indazol-5-yl)-2-(2-quinoxalinyl)-4-quinazolinamine (1)

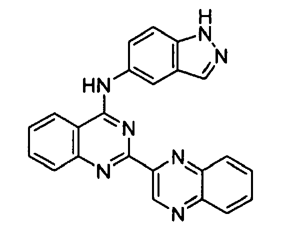

**Step 1:** To a solution of anthranilonitrile (7.58 mmol) in dry pyridine (30 mL) is added 2-quinoxaloyl chloride (9.11 mmol, 1.2 equivalent). The reaction mixture stirred at room temperature overnight and sodium hydroxide solution (2%, 50 mL) is added. The mixture is cooled and stirred for 30 min. The resulting white solid is collected by filtration, washed with brine and cold ether. A white solid product is obtained (1.51 g, 73%). HPLC/MS: (M+H)⁺=275, RT (HPLC/MS)=3.0 min.

**Step 2:** The amide prepared in Step 1(9.5 mmol, 1 equivalent) is suspended in dioxane (10 mL). NaOH solution (20%, 60 mL) and hydrogen peroxide solution (30%, 30 mL) is added in three portions. A vigorous release of gas is observed. The reaction mixture continued to stir and is cooled when necessary until the evolution of gas ceased. The reaction is brought to 120 °C (oil bath) and stirred overnight at this temperature. The reaction is neutralized with concentrated HCl to pH=7. A precipitate formed and is collected on a funnel, washed with water and dried *in vacuo*. A yellow solid is obtained and used in the next step without further purification. HPLC/MS: (M+H)⁺= 275, RT (HPLC/MS)=3.28.

Step 3: The quinazoline (10.9 mmol) is suspended in phosphorous oxychloride (214.6 mmol) containing PCl₅ (10.9 mmol) and stirred at 115 °C for 18 h. The resulting yellow solution is poured into 300 mL of ice and stirred. A gray precipitate formed and filtered and washed with cold water. The product is used in the next step without further purification. HPLC/MS: (M+H)⁺=293, RT (LC-MS)=3.40.

Step 4: A mixture of 4-chloroquinazoline, potassium acetate (14.25 mmol), and 5-aminoindazole (10.96 mmol) in THF/H₂O (70 mL/25 mL) is stirred at room temperature for 17 h. The resulting solid is collected by filtration and purified by silica gel column chromatography (gradient, 5-10% MeOH/CH₂Cl₂) to afford the product (1.19 g, 32%, 3 steps) as yellow powder. HPLC/MS: (M+H)⁺=390, RT (LC-MS)= 2.41.

### General Synthetic Scheme for Example 34

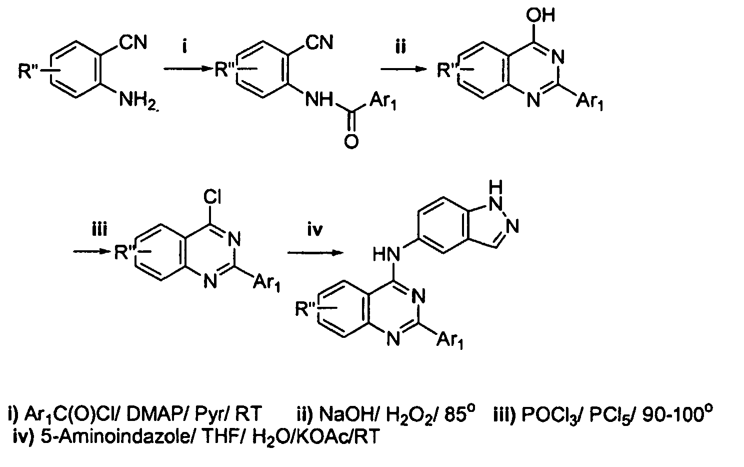

### Example 34

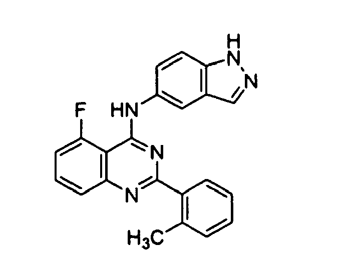

### Preparation of 5-Fluoro-N-(1H-indazol-5-yl)-2-(2-methylphenyl)-4-quinazolinamine

**Step 1:** To a solution of 6-fluoro-2-amino-benzonitrile (2mmol, 1 equivalent.) in pyridine (3mL) and CH₂Cl₂ (1mL) containing *N*-dimethylaminopyridine (3 mg) is added 2-toluoyl chloride (316 mL, 1.2 equivalent). The reaction mixture is shaken at room temperature for 48 h and poured into cold water (3mL) and shaken for 1 h. The resulting solid is filtered and washed with water to afford a white solid (90%). The LC-MS is consistent with the desired compound.

**Step 2:** The product is suspended in aqueous NaOH (20%, 2mL) and dioxane (1mL). Hydrogen peroxide (30%, 1mL) is added in potions to avoid vigorous formation of gas. The reaction is shaken at 85 °C for 20 h and then is neutralized with acetic acid to pH=7. The resulting precipitate is collected by filtration, washed with water and ether, and dried over P₂O₅ for two days. The product is suspended in P(O)Cl₃ (4mL) and shaken at 90 °C overnight. The POCl₃ is removed *in vacuo* and co-evaporated with toluene. The resulting yellow solid residue is dried in vacuo overnight and used in the next step without further purification

Step 3: The product (assumed to be 2 mmol), 5-aminoindazole (3mmol, 1.5 equivalent), and potassium carbonate (2mmol) were suspended in DMF (5mL) containing and shaken at 90 °C for 24 h. The reaction suspension is filtered and the filtrate is purified by HPLC, under the following conditions:

Column: YMC C18 Pro, 20x150m/m; Gradient: A= H2O, 0.1% TFA, B=CH₃CN, 0.1%TFA; Gradient over 10 min, flow: 30mL/min. A pale yellow solid product is obtained. (M+H)⁺= 370, RT (LC-MS)=2.19 min.

Using the methods described above for Examples 34 and substituting the appropriate starting materials, the compounds listed in Table 5 were also synthesized.

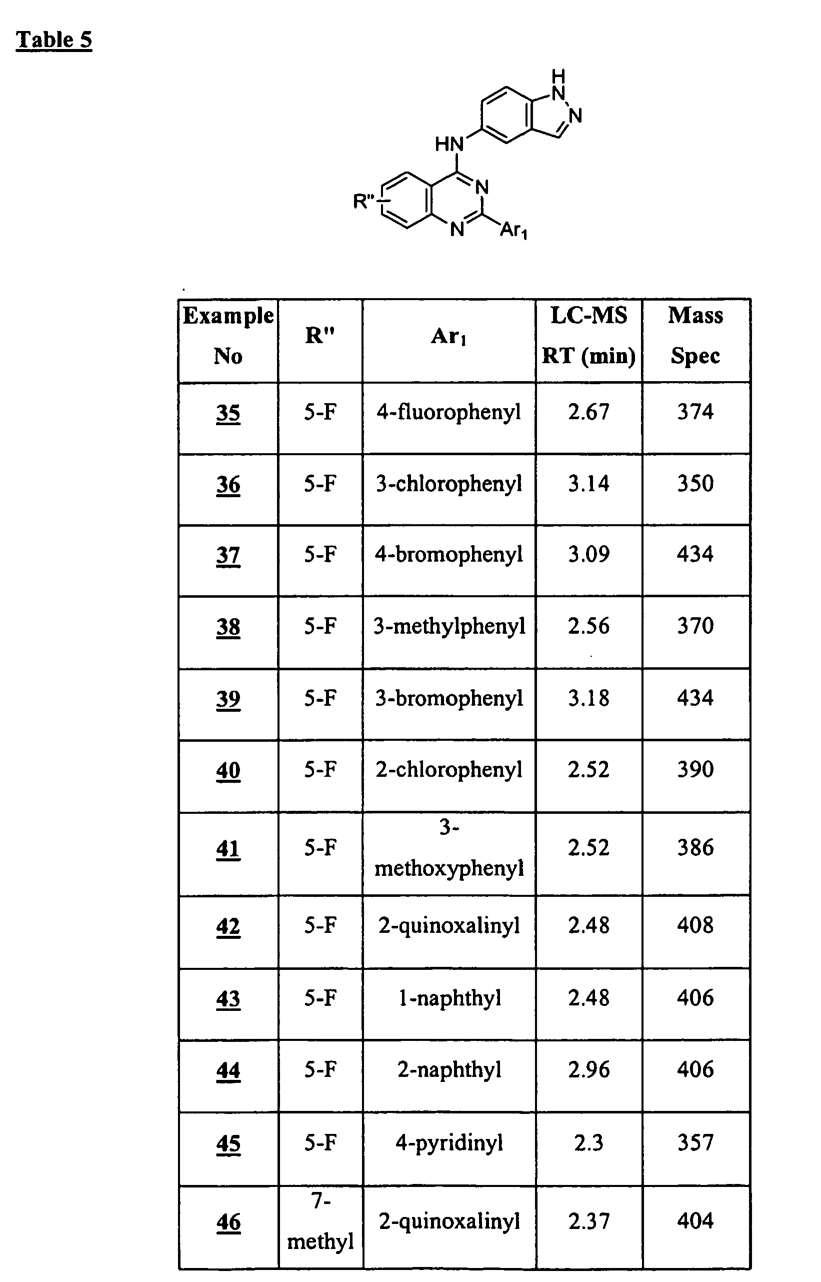

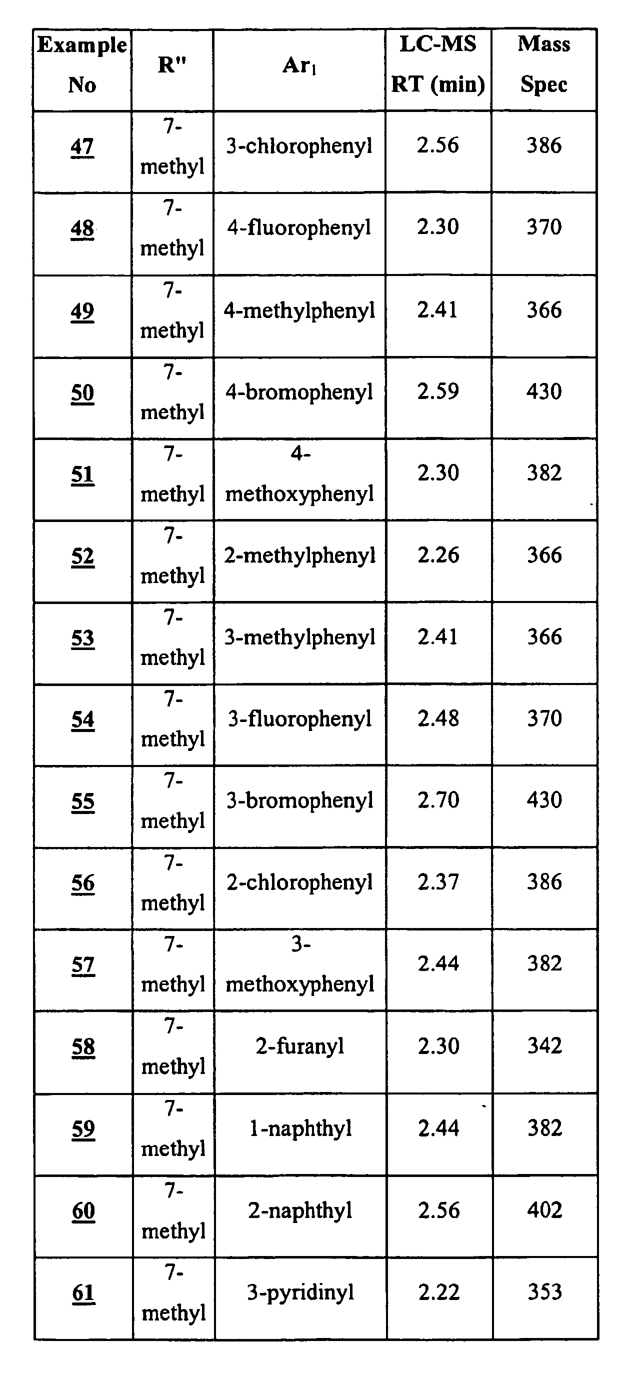

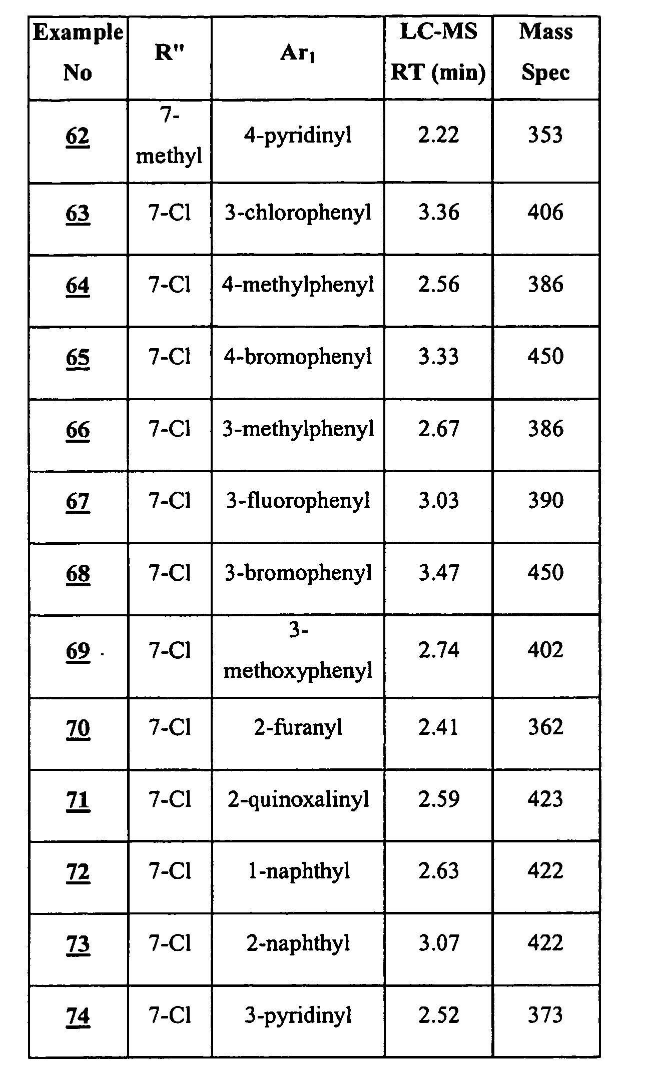

### General Synthetic Route to Examples 75-80

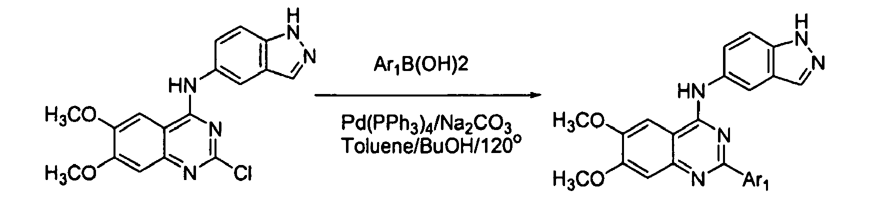

### Example 75

### Preparation of N-[2-(4-fluorophenyl)-6,7-dimethoxy-4-quinazolinyl]-N-(1H-indazol-5-yl)amine

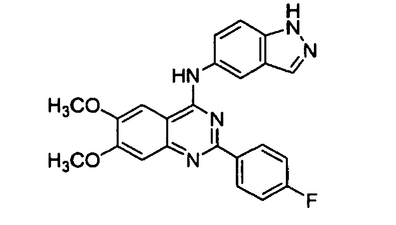

2-Chloro-*N*-(1*H*-indazol-5-yl)-6,7-dimethoxy-4-quinazolinamine (prepared from 3,4-dimethoxybenzoylurea by the method described for Example **1**, steps 1 and 2) (0.1 mmol) is suspended in toluene (1mL), *n*-BuOH (0.5 mL), and Na₂CO₃ (0.5 mL, 2M aqueous). The reaction mixture is degassed for 20 min with argon followed by the addition of 4-fluorophenyl boronic acid (0.4 mmol) and Pd-catalyst (0.05 mmol) The mixture is heated to reflux and stirred for 72 h. The solvent is removed in vacuo and the residue is purified by preparative silica gel TLC (5% MeOH /CH₂Cl₂) to obtain a yellow solid :HPLC/MS: (M+H)⁺ = 416, RT (HPLC/MS) = 2.96.

Using the method described above for Example **75** and substituting the appropriate starting materials Examples **76-80** similarly prepared and are shown in Table 6.

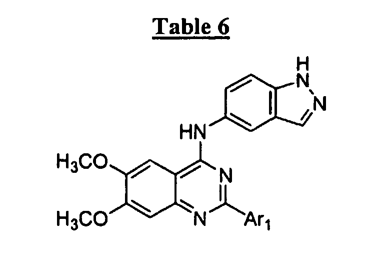

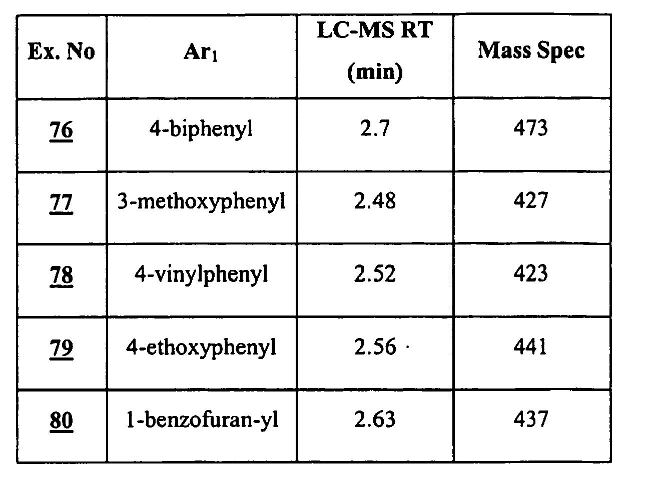

### General Synthetic Route to Examples 81-107

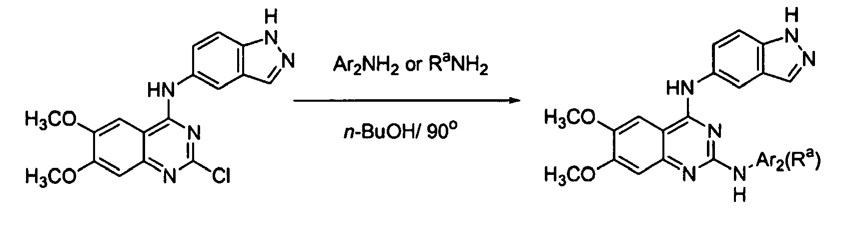

### Example 81

### Preparation of N2-(3-fluorophenyl)-N4-(1H-indazol-5-yl)-6,7-dimethoxy-2,4-quinazolinediamine

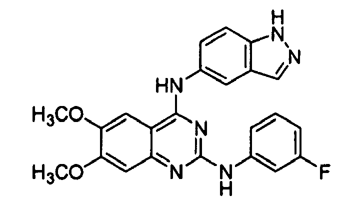

A suspension of 2-chloro-*N*-(1H-indazol-5-yl)-6,7-dimethoxy-4-quinazolinamine (0.1mmol) and 3-fluoroaniline (0.3 mmol) in n-butanol (1 mL) is shaken at 90°C for 72 h. The solvent is evaporated off and the residue is purified by HPLC to afford pure product. (M+H)⁺=431, RT (LC-MS)=2.94.

Using the method described above for Example **81**, and substituting the appropriate starting materials, Examples **82**-**107** were similarly prepared and are summarized below in Table 7.

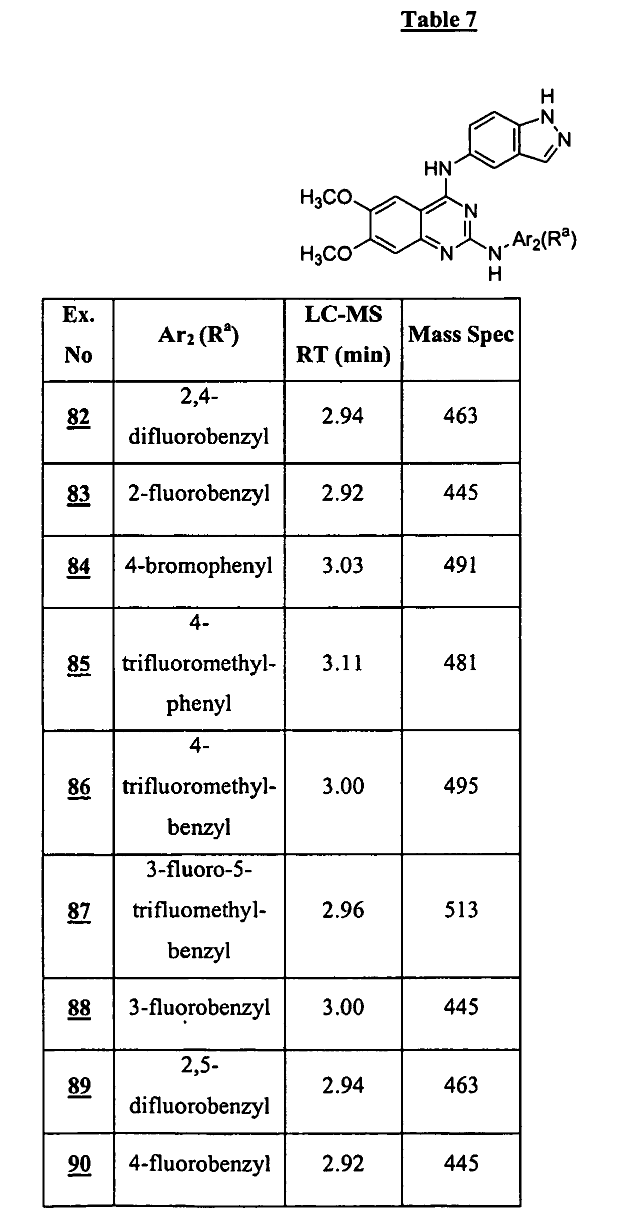

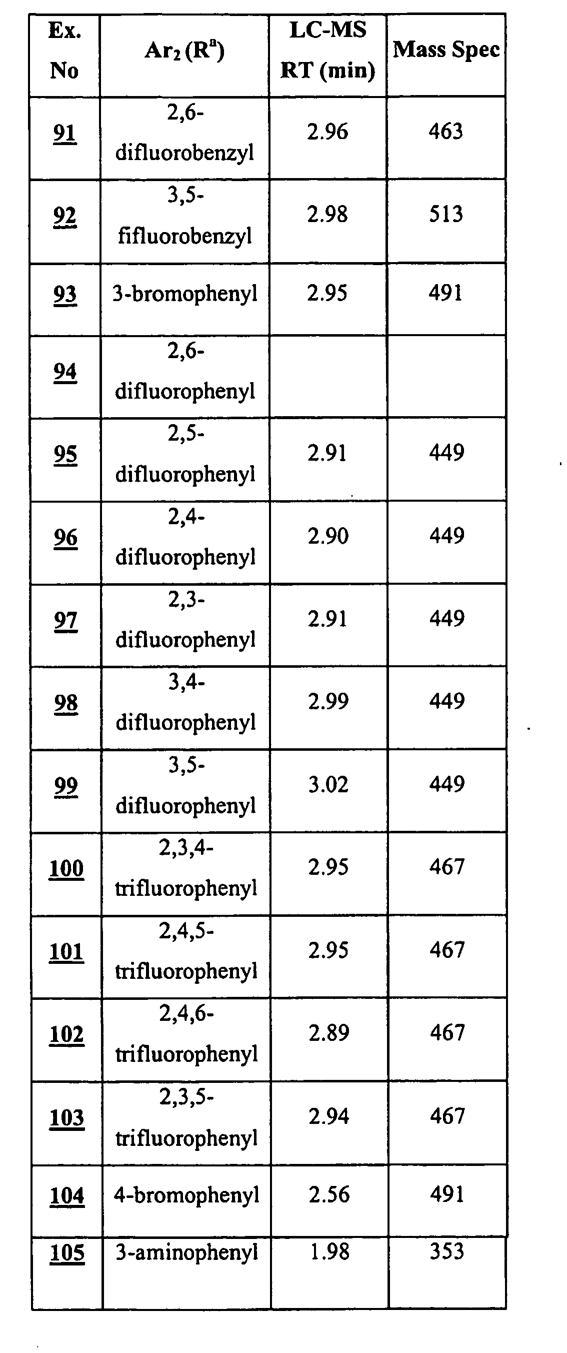

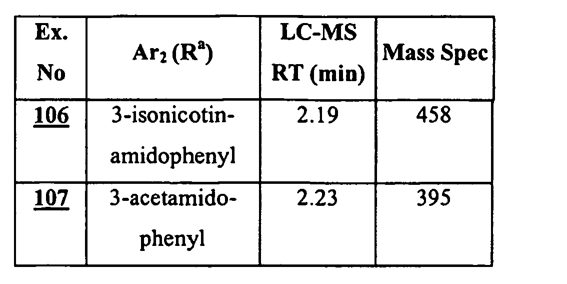

### General Synthesis Route to Examples 108-129

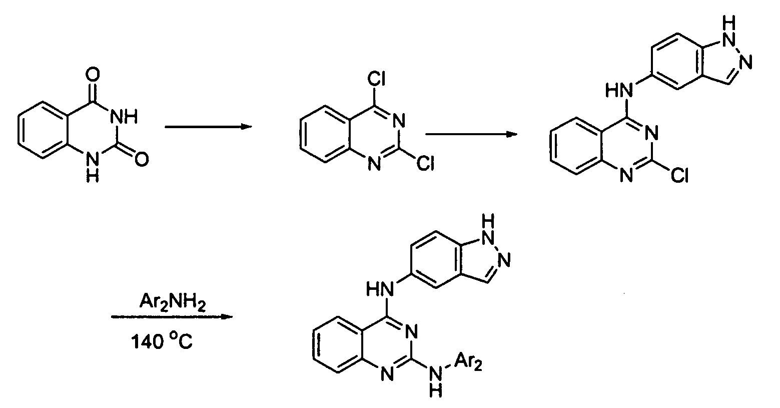

### Examples 108-136

### General Preparation of N2-(Substituted aryl)N4-(1H-indazol-5-yl)-2,4-quinaolinediamines

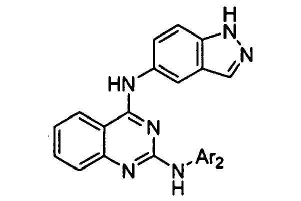

A mixture of 2-chloro-*N*-(1H-indazol-5-yl)-4-quinazolinamine (30 mg, 0.1 mmol) and a substituted aniline (2 mmol) is heated to 140 °C for 2 hrs. The mixture is cooled to rt and treated with ether to form precipitate which is washed with ether several times and dried under high vacuum to provide product. Alternatively, the product is purified by silica gel column chromatography by dissolving the solid in dichloromethane and loaded on to a column which is eluted (hexanes/ethyl acetate, gradient) to give desired product.

Using this method and substituting the appropriate aniline starting materials, Examples **108**-**129** were prepared and are summarized in Table 8 below:

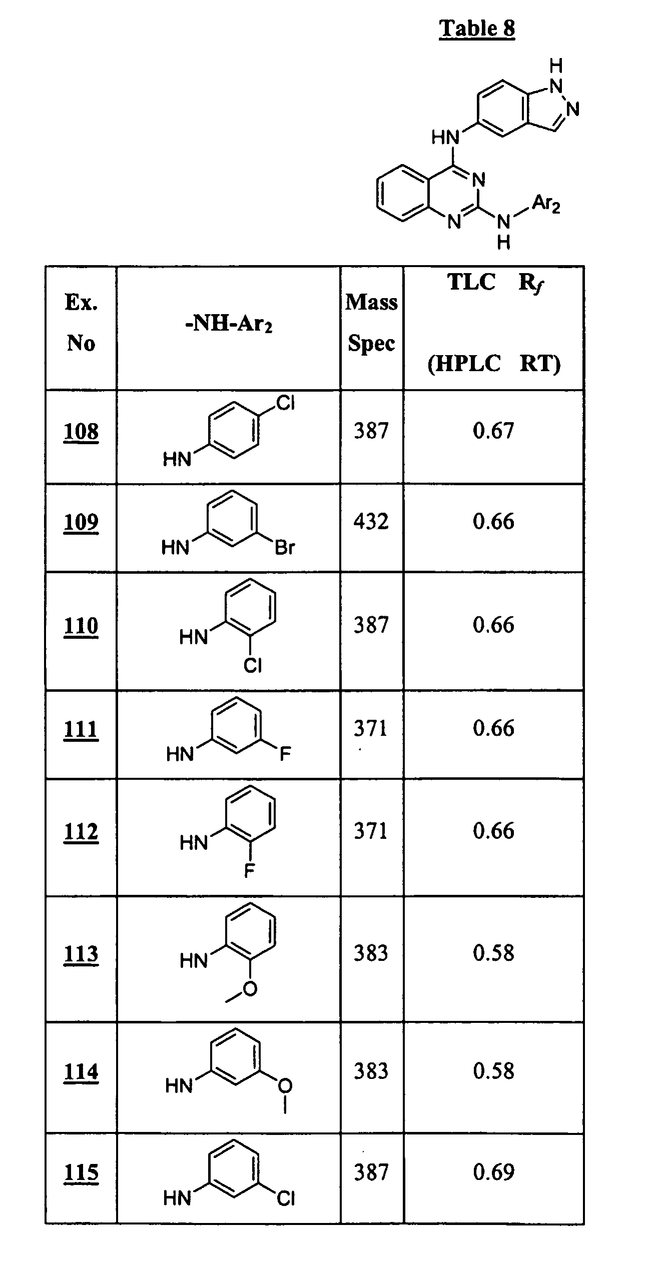

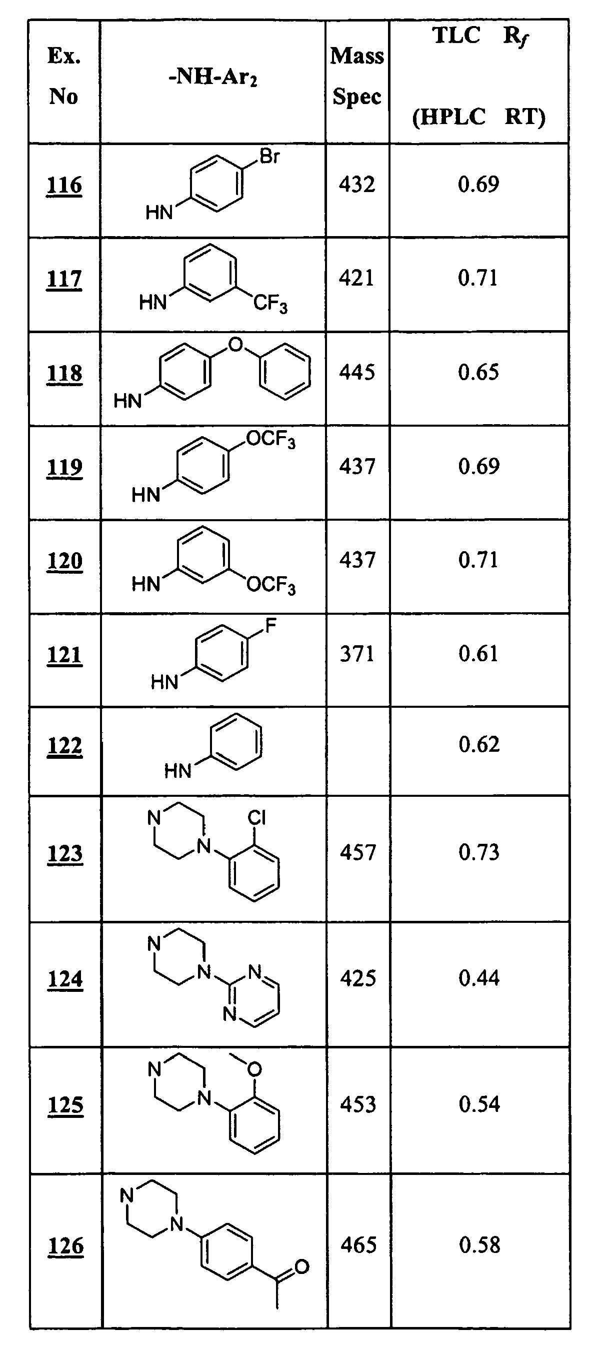

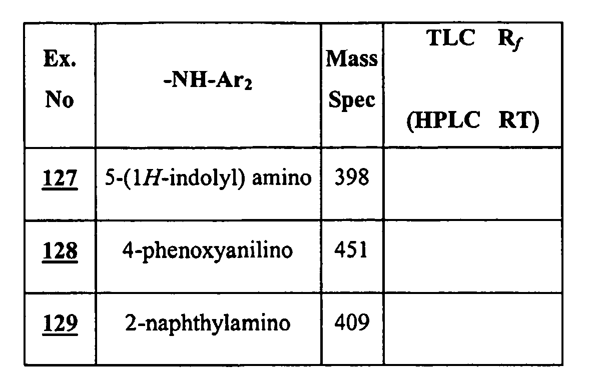

### Example 130

### Preparation of 4-(1H-indazol-5-ylamino)-2-quinazolinecarboxamide

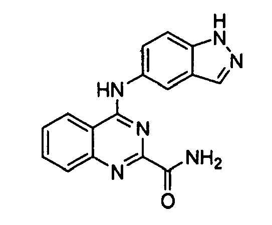

### Step 1: Preparation of ethyl 4-oxo-3,4-dihydro-2-quinazolinecarboxylate

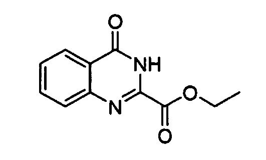

According to the method of Suesse, M.; Adler, F.; and Johne, *S.(Helv. Chim. Acta* **1986**, 69 1017), a mixture of 2-aminobenzamide (20 g, 147 mmol) and diethyl oxalate (39.9 mL, 42.9 g, 294 mmol) is warmed to 170 - 180 °C for 6 h. The mixture is cooled to rt and diluted with EtOH. The resultant precipitate is filtered and washed thoroughly with EtOH to afford a crude solid, which could be further purified by recrystallization from EtOH (21.1 g, 66%).

### Step 2: Preparation of ethyl 4-chloro-2-quinazolinecarboxylate

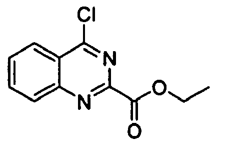

A mixture of material from Step 1 (1.0 g, 4.6 mmol), thionyl chloride (4.0 mL, 6.5 g, 55 mmol), and *N*,*N*-dimethylformamide (5 drops) in chloroform (10 mL) is heated to for 4 h. The mixture cooled to rt and the volatiles were removed under vacuum. The resultant crude solid is dried under vacuum overnight to afford the desired intermediate (1 g, 92 %) which is used in the next step without additional purification.

### Step 3: Preparation of ethyl 4-(1H-indazol-5-ylamino)-2-quinazolinecarboxylate hydrochloride

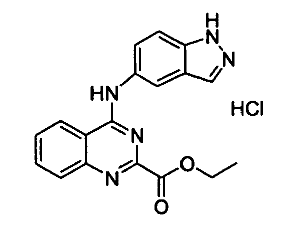

A mixture of compound from Step 2 (1 g, 4.23 mmol), 5-aminoindazole (0.560 mg, 4.23 mmol), HCl (15 mL, 0.12 N, aqueous) and *n*-BuOH (4.3 mL) is warmed to 100 °C for 4 h. The mixture cooled to rt and the resultant precipitate is removed by filtration. The solid is washed thoroughly with EtOAc and CH₂Cl₂, and is dried under vacuum overnight to afford the product as an orange solid (1.21 g, 77 %). mp (°C): 215-219; TLC Rf = 0.23 (90/10, CH₂Cl₂/MeOH)

### Step 4: Preparation of 4-(1H-indazol-5-ylamino)-2-quinazolinecarboxamide

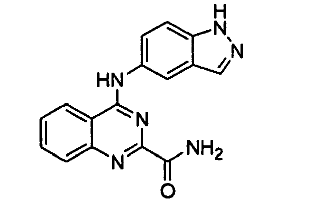

To a suspension of the Step 3 amine hydrochloride salt (0.11 g, 2.03 mmol) in toluene (5 mL) at rt is added the trimethylaluminum (1.00 mL, 2.0 M in heptanes, 2.0 mmol) dropwise. The mixture stirred until gas evolution ceased, approximately 1 hour. The newly formed solution of trimethylaluminum and ammonium chloride is added dropwise to a solution of product from Step3 (0.15 g, 0.41 mmol) in toluene (5 mL) at rt. The reaction mixture is heated to reflux, and stirred for 5 h. The reaction is cooled to rt, and quenched slowly with 5% aqueous HCl (2 mL). The biphasic mixture is filtered through Extrelut, and the filtering aid is washed thoroughly with EtOAc. The combined organic ishes and filtrates were concentrated, and the crude product is purified by reversed phase HPLC to afford Example **130** (0.032 g, 26 %). mp. (°C): 300; TLC Rf = 0.05 (90/10, CH₂Cl₂/MeOH) 0.05.

By using the above method and substituting the appropriate starting materials, Examples **131-138** were synthesized in analogous manner and are summarized in Table 9.

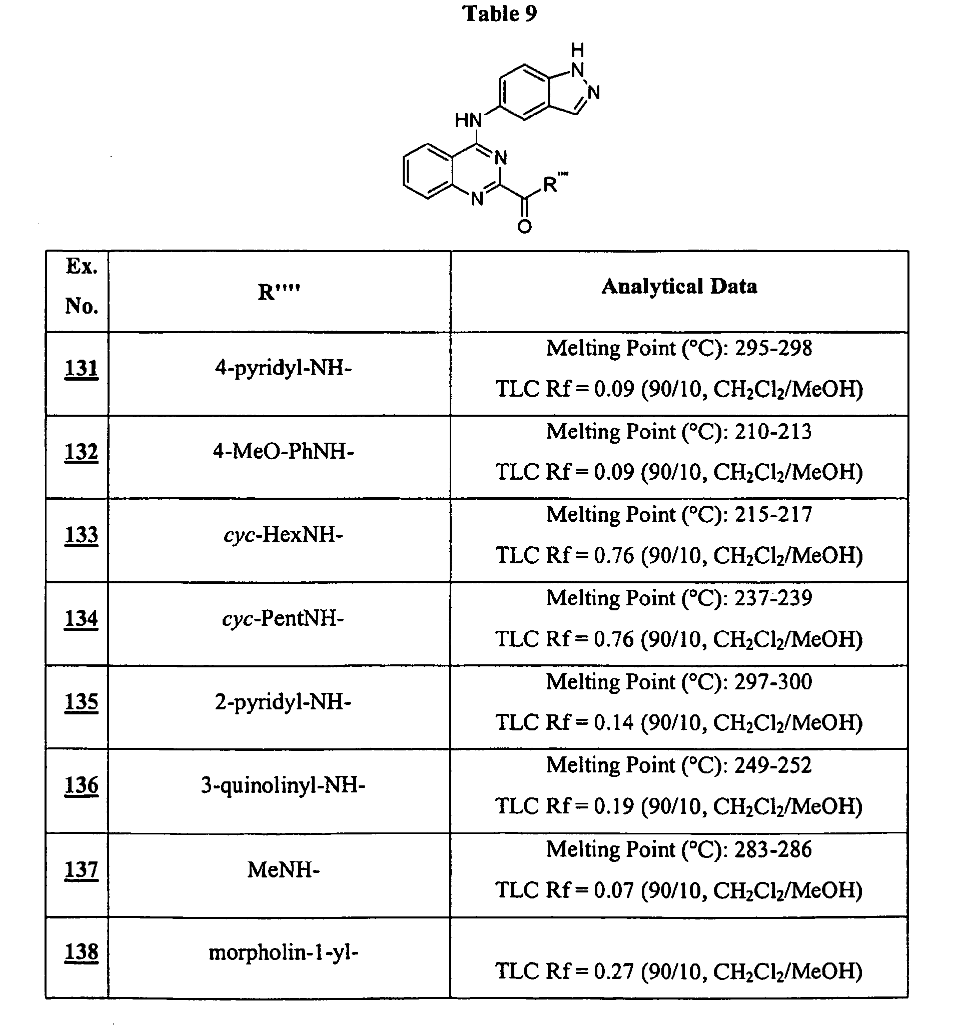

### Example 139

### Preparation of N-(1H-indazol-5-yl)-N-(2-methyl-4-quinazolinyl)amine

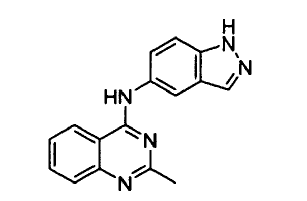

### Step 1: Preparation of 2-(acetylamino)benzamide

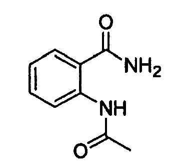

To a solution of anthranilamide (1.6 g, 11.6 mmol), pyridine (1.1 mL, 13.9 mmol) and CHCl₃ (55 mL) is added acetyl chloride (91 µL, 12.7 mmol), dropwise. The reaction stirred at room temperature for 2 h. The volatiles were removed by evaporation and the residue is partitioned between EtOAc and 1 N sodium carbonate. The resulting precipitate is collected by filtration. The layers of the filtrate were separated and the organic phase is washed with 1 N HCl, dried (MgSO₄), and evaporated. The filtered solid product and the evaporated solid were combined and dried under vacuum to afford the desired intermediate. (1.1 g, 6.2 mmol; 54% yield); Rf = 0.47 (EtOAc/hexanes, 50/50); ¹H NMR (DMSO-d₆) 11.55 (s, 1H), 8.39 (d, *J* = 8.2, 1H), 8.22 (s, 1H), 7.74 (m, 2H), 7.07 (m, 1H), 7.07 (m, 1H), 2.07 (s, 1H); ES MS (M+H)⁺= 179.

### Step 2: Preparation of 2-methyl-4-quinazolinol

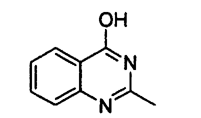

To a mixture of diamide from Step 1 (890 mg, 5.0 mmol) in EtOH (30 mL) is added 10 N NaOH (1.49 mL, 14.9 mmol). The reaction is heated to reflux for 4 h, cooled to room temperature and the volatiles were evaporated. The aqueous mixture is acidified to pH = 5 with concentrated HCl. The mixture is evaporated until a precipitate formed. The solids were collected by filtration, washed with hexanes and dried under vacuum to afford the desired intermediate (564 mg, 3.5 mmol; 71% yield); Rf = 0.10 (EtOAc/hexanes, 50/50); ¹H NMR (DMSO-d₆) 8.11 (dd, *J =* 1.0, 7.8, 1H), 7.89 (m, 1H), 7.74 (d, *J =* 8.1, 1H), 7.58 (m, 1H), 2.53 (s, 3H); ES MS (M+H)⁺= 161.

### Step 3: Preparation of N-(1H-indazol-5-yl)-2-methyl-4-quinazolinamine

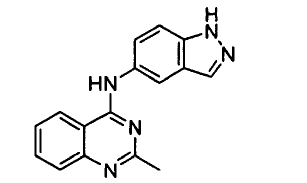

A thoroughly homogenized mixture of 5-aminoindazole (831 mg, 6.2 mmol), phosphorous pentoxide (886 mg, 6.2 mmol), and triethylamine hydrochloride (859 mg, 6.2 mmol) is heated at 200 °C to obtain a melt. After 1 h the hydroxyquinazoline from Step 2 (250 mg, 1.6 mmol) is added in one portion and the mixture is kept at 200 °C for 16 h. The mixture is cooled to 135 °C, 9:1 H₂O-MeOH (10 mL) is added, and mixture is sonicated. The mixture is decanted, adjusted to pH = 9 with concentrated ammonium hydroxide, and concentrated under vacuum. The residue is purified by flash chromatography (CH₂Cl₂₋MeOH, 100/0 - 90/10 gradient). The fractions containing product were combined and the volatiles were removed by evaporation. The residue is partitioned between 1 N NaOH and EtOAc. The organic layer is removed, dried (MgSO4), and evaporated. The residue is further purified by preparative TLC (CH₂Cl₂-MeOH, 95/5 - 90/10 gradient) and dried under vacuum to afford Example **139** (17 mg, 0.062 mmol, 4% yield); Rf = 0.45 (EtOAc/hexanes, 90/10); mp = 282 - 288 °C; ES MS (M+H)⁺= 276. 7 Hz), 1.40 (3H, t, J = 5.7 Hz).

### Example 140

### Preparation of 1H-indazol-5-yl[2-(3-fluoro-4-phenylphenyl)quinazolin-4-yl]amine

The following process can be used to prepare the single compound

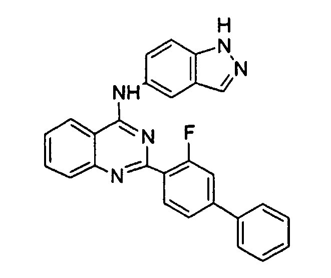

### Step 1: Preparation of 3-fluoro-4-phenylbenzoic acid

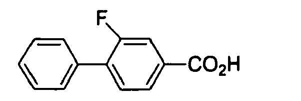

A suspension of magnesium (0.968 g, 3.98 mmol) and a few crystals of iodine in anhyd THF (200 mL) were treated with dropwise addition of 10 mL of a solution of 4-bromo-2-fluorobiphenyl (10.0 g, 3.98 mmol) in THF (100 mL). The mixture was heated to gentle reflux and a reaction ensued. At that time, the remaining solution of 4-bromo-2-fluorobiphenyl was added dropwise to the flask over a 3-minute period. The contents were then stirred at reflux under argon until no magnesium consumption was observed. The reaction mixture was subsequently cooled to -10°C and treated with dry ice (~70 g). The reaction mixture was quenched with 20% aqueous hydrochloric acid (50 mL), and the layers were separated. The aqueous phase was extracted with ethyl acetate (2 x 20 mL), and the combined organic layer was washed with brine (30 mL), dried over anhyd sodium sulfate and concentrated to about 1/3 of its original volume. The contents were treated with hexane (200 mL), and the precipitate was filtered and dried under high vacuum to afford 3-fluoro-4-phenylbenzoic acid (6.37 g, 74%) as a white, crystalline solid. ¹H-NMR (DMSO-*d*₆): δ 7.48 (m, 3H); 7.59 (m, 2H); 7.66 (dd, J = 8.1, 8.1 Hz, 1H); 7.76 (dd, J = 1.5, 11.6 Hz, 1H); 7.85 (dd, J = 1.5, 8.1 Hz, 1H); 13.30 (br s, 1H). Anal. Calcd for C₁₃H₉FO₂: C, 72.22; H, 4.20; F, 8.79. Found: C, 71.95; H, 4.11; F, 9.07.

### Step 2: Preparation of 2[(3-fluoro-4-phenylphenyl)carbonylamino]benzamide

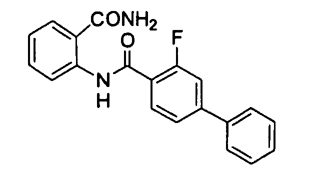

A suspension of the product of step 1 (0.5 g, 2.31 mmol) in oxalyl chloride (5 mL) was treated with one drop of DMF and the mixture was heated to 60°C for 45 min. The resulting, clear-yellow solution was concentrated to a yellow solid, which was dried under high vacuum for 60 min. The solid and anthranilamide (0.314 g, 2.31 mmol) were suspended in dry toluene (5 mL), treated with diisopropylethylamine (0.5 ml, 0.371 g, 2.87 mmol) and the contents were stirred at room temperature for 2 h, at which time TLC (silica gel 60, 10% methanol/dichloromethane, UV detection) analysis suggested complete reaction. The mixture was filtered, and the off-white solid was dissolved in ethyl acetate (50 mL). The organics were washed with brine (25 mL), 0.1 N aqueous hydrochloric acid (25 mL), and again with brine (25 mL). The organic layer was dried over anhyd sodium sulfate, concentrated and dried under high vacuum for 4 h to afford the product (0.59 g, 1.76 mmol, 76%) as an off-white solid. ¹H-NMR (DMSO-*d*₆): δ 7.22 (ddd, J = 1.2, 7.4, 7.8 Hz, 1H); 7.52 (m, 6H); 7.78 (m, 3H); 7.89 (m, 1H); 7.89, 8.47 (br s, 2H); 8.69 (dd, J = 1.2, 8.3 Hz, 1H); 13.12 (s, 1H). Anal. Calcd for C₂₀H₁₅N₂FO₂: C, 71.85; H, 4.52; N, 8.38. Found: C, 71.67; H, 4.47; N, 8.35. Mass spectrum (HPLC/ES, flow injection): m/e = 335 (M + 1).

### Step 3: Preparation of 2-(3-fluoro-1,1'-biphenyl-4-yl)-4(3H)-quinazolinone

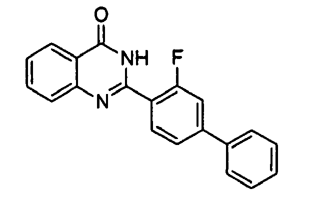

### Method A

A suspension of the product of step 2 (0.5 g, 2.31 mmol) in oxalyl chloride (5 mL) was treated with one drop of DMF and the mixture was heated to 60°C for 60 min. The resulting clear yellow solution was concentrated to a yellow solid, which was dried under high vacuum for 2 h. This solid and anthranilamide (0.314 g, 2.31 mmol) were dissolved in dry THF (5 mL), treated with diisopropylethylamine (0.5 ml, 0.371 g, 2.87 mmol) and the contents were stirred at room temperature for 90 min, at which time TLC (silica gel 60, 5% methanol/dichloromethane, UV detection) analysis suggested complete reaction. The mixture was treated with aqueous 1.0 N sodium hydroxide (10.0 mL, 10.0 mmol). The contents were heated to 50°C (complete dissolution occurred when the internal temperature reached 44°C) for 90 min and the organic solvent was removed by rotary evaporation. The aqueous suspension was treated with dropwise addition of aqueous 2.0 N hydrochloric acid (about 5 mL) until the pH was adjusted to about 2. The precipitate was filtered and the cake was washed with water (4 x 30 mL) and dried under high vacuum at 40°C for 18 h to provide the product (0.67 g, 2.12 mmol, 92%) as a white powder. ¹H-NMR (DMSO-*d*₆): δ 7.52 (m, 4H); 7.64 (m, 2H); 7.75 (m, 2H); 7.86 (ddd, J = 1.4, 6.9, 8.0 Hz, 1H); 8.16 (m, 3H); 12.63 (br s, 1H). Anal. Calcd for C₂₀H₁₃N₂FO: C, 75.94; H, 4.14; N, 8.86. Found: C, 75.66; H, 4.29; N, 8.77. Mass spectrum (HPLC/ES): m/e = 317 (M + 1).

### Method B.

A suspension of the product of step 1 (0.5 g, 2.31 mmol) in oxalyl chloride (5 mL) was treated with one drop of DMF and the mixture was heated to 60°C for 60 min. The resulting clear yellow solution was concentrated to a yellow solid, which was dried under high vacuum for 60 min. This solid and anthranilamide (0.314 g, 2.31 mmol) were suspended in dry toluene (5 mL), treated with diisopropylethylamine (0.5 ml, 0.371 g, 2.87 mmol) and the contents were stirred at room temperature for 2 h, at which time TLC (silica gel 60, 10% methanol/dichloromethane, UV detection) analysis suggested complete reaction. The mixture was filtered and dried under high vacuum for 2 h. The off-white solid was then dissolved in methanol (10 mL) and THF (5 mL), and the solution was treated with aqueous 1.0 N sodium hydroxide (10.0 mL, 10.0 mmol). The contents were heated to 45°C for 2 h and the organic solvents were removed by rotary evaporation. The aqueous suspension was treated with dropwise addition of aqueous 2.0 N hydrochloric acid until the pH was adjusted to about 2 (5 mL). The precipitate was filtered and the cake was washed with water (4 x 30 mL) and dried under high vacuum at 40°C for 3 h to provide product (0.66 g, 2.09 mmol, 90%) as a white powder. ¹H-NMR (DMSO-*d*₆): δ 7.52 (m, 4H, aromatic); 7.64 (m, 2H, aromatic); 7.75 (m, 2H); 7.86 (ddd, J = 1.4, 6.9, 8.0 Hz, 1H, aromatic); 8.16 (m, 3H, aromatic); 12.63 (br s, 1H, -NH). Anal. Calcd for C₂₀H₁₃N₂FO • 0.20 H₂O: C, 75.08; H, 4.22; N, 8.76. Found: C, 75.08; H, 4.03; N, 8.67. Mass spectrum (HPLC/ES): m/e = 317 (M + 1).

### Step 4: Preparation of 4-chloro-2-(3-fluoro-4-phenylphenyl)quinazoline

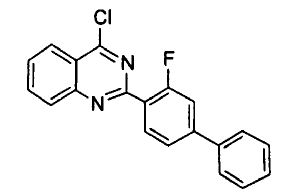

A solution of phosphorous oxychloride (3.0 mL) and anhyd DMF (2 mL) was stirred for 10 min before it was added to a flask containing the product of step 3 (0.300 g, 0.948 mmol). The resulting suspension was heated to gentle reflux under argon for 12 h. The dark solution was then cooled to 70°C and slowly added to vigorously-stirred water (100 mL) at 0°C. A solid precipitated, which was stirred for 10 min and filtered. The cake was washed with water (2 x 25 mL) and dried under high vacuum at 35°C for 2 h to provide product (0.285 g, 0.851 mmol, 90%) as a yellow solid. Part of this solid (0.125 g) was passed through a short plug of silica gel using 20% dichloromethane/hexane as eluant to afford the title compound (0.09 g) as white needles. ¹H-NMR (DMSO-*d*₆): δ 7.47 (m, 1H); 7.54 (m, 2H); 7.65 (m, 2H); 7.76 (dd, J = 8.4, 8.4 Hz, 1H); 7.87 (ddd, J = 2.9, 5.3, 8.3 Hz, 1H); 8.15 (m, 2H); 8.26 (m, 1H); 8.28 (m, 1H); 8.38 (dd, J = 1.9, 8.4 Hz, 1H). Anal. Calcd for C₂₀H₁₂N₂ClF: C, 71.75; H, 3.61; N, 8.37; Cl, 10.59. Found: C, 71.54; H, 3.48; N, 8.29; Cl, 10.61. Mass spectrum (HPLC/ES): m/e = 335 (M + 1). TLC (silica gel 60, 40% dichloromethane/hexane, UV detection): one spot, R_{*f*} = 0.50.

### Step 5: Preparation of 1H-indazol-5-yl[2-(3-fluoro-4-phenylphenyl)quinazolin-4-yl]amine

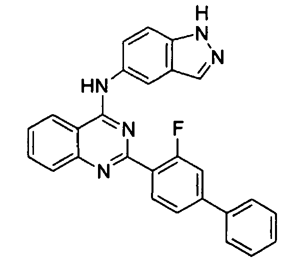

To a suspension of the product of step 4 (1.00 g, 2.99 mmol) and 5-aminoindazole (0.44 g, 3.29 mmol) in ethylene glycol dimethyl ether(DME, 10 mL) was added a solution of potassium acetate (0.44 g, 4.48 mmol) in water (2 mL). The contents were allowed to reflux for 16 h and then cooled to room temperature. The mixture was poured into water (200 mL) and the precipitate was filtered, washed with water (2 x 50 mL) and air-dried for 60 min. The solid was dissolved in THF (30 mL), and the solution was slowly poured into hexane (500 mL). The resulting precipitate was filtered and dried under high vacuum at 60°C for 18 h to afford the product (1.02 g, 2.36 mmol, 79%) as a yellow solid. ¹H-NMR (DMSO-*d*₆): δ 7.46 (m, 3H); 7.63 (m, 5H); 7.83 (dd, J = 1.9, 9.0 Hz, 1H); 7.87 (m, 2H); 8.13 (br s, 1H); 8.17 (dd, J = 1.6, 12.5 Hz, 1H); 8.22 (d, J = 1.9 Hz, 1H); 8.30 (dd, J = 1.6, 8.0 Hz, 1H); 8.58 (br d, J = 8.5 Hz, 1H); 10.04 (s, 1H, -NH); 13.13 (br s, 1H). Mass spectrum (HPLC/ES): m/e = 432 (M+1).

In order to prepare the *p*-toluene sulfonic acid (tosylate) salt, a suspension of the product (0.60 g, 1.39 mmol) in anhyd ethanol (12 mL) was treated with a solution of *p-*toluenesulfonic acid monohydrate (0.39 g, 2.09 mmol) in ethanol (8.5 mL) in one portion. The contents were stirred at 40°C for 60 min and the precipitate was filtered. The cake was washed with ethanol (3 x 15 mL) and dried under high vacuum at 40°C for 1.8 h to give the tosylate salt (0.71 g, 85%) as a pale-orange, crystalline solid. ¹H-NMR (DMSO-*d*₆): δ 2.27 (s, 3H); 7.09, 7.47 (AA'BB' quartet, J = 8.6 Hz, 4H); 7.48 (m, 2H); 7.52 (m, 2H); 7.62 (m, 2H); 7.73 (m, 2H); 7.84 (m, 2H); 8.10 (m, 5H); 8.20 (s, 1H); 8.74 (br d, J = 8.4 Hz, 1H); 11.50 (br s, 1H). Anal. Calcd for C₂₇H₁₈N₅F • CH₃C₆H₄SO₃H: C, 67.65; H, 4.34; N, 11.60. Found: C, 67.35; H, 4.46; N, 11.49. Mass spectrum (HPLC/ES): m/e = 432 (M + 1).

### General Synthetic Route for Examples 141

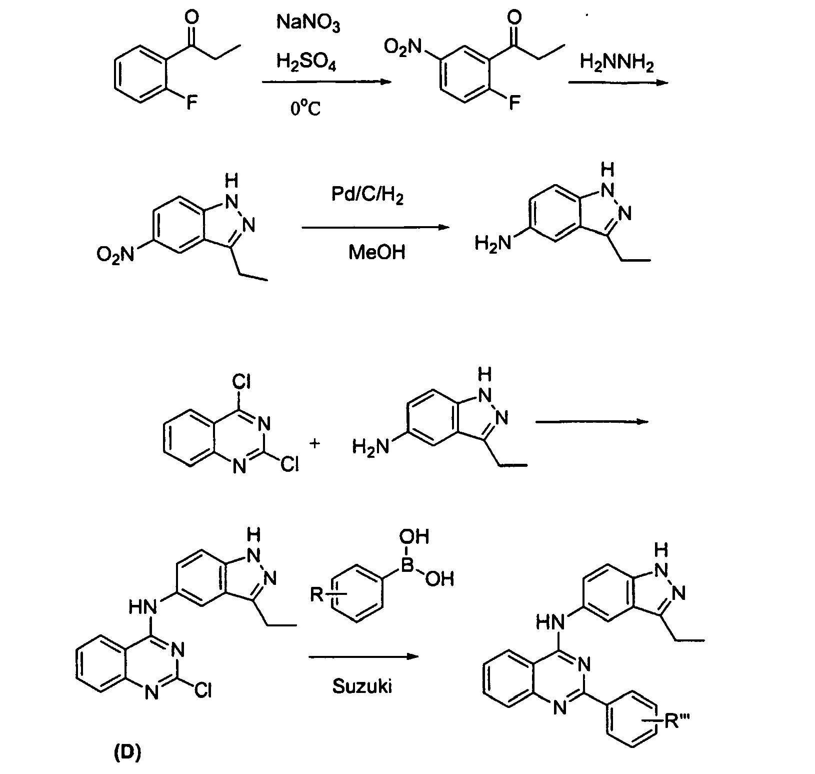

### Example 142

### Preparation of N-(3-ethyl-1H-indazol-5-yl)-2-(4-methoxyphenyl)-4-quinazolinamine

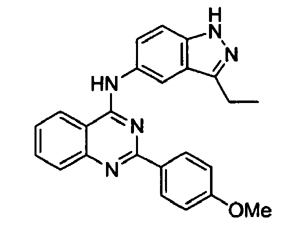

### Step 1: Preparation of 1-(2-fluoro-5-nitrophenyl)-1-propanone

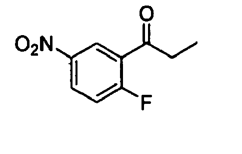

To a solution of 2-fluorophenyl ethyl ketone (4.41 g) in H₂SO₄ (10 mL) at 0°C is added a mixture of NaNO₃ (2.72 g) and H₂SO₄ (20 mL) dropwise to maintain the temperature. The reaction mixture warmed to room temperature slowly and stirred for 1 hour. The reaction mixture is poured over ice/water. The organic layer is washed with ice water (3 x 100 mL). The organic layer is dried over Na₂SO₄, filtered and evaporated under the reduced pressure. The crude product is purified by silica gel column chromatography (Hex/EtOAc, 4:1, Rf = 0.77) to afford pure product nitro ketone 1.83 g (34%): ¹H NMR (CDCl₃) δ 8.73 (1H, dd, J = 2.4, 4.8 Hz), 8.36-8.33 (1H, m), 7.29 (1H, t, J = 6.9 Hz), 3.00 (2H, q, J = 2.7 Hz), 1.20 (3H, t, J = 5.4 Hz).

### Step 2: Preparation of 3-ethyl-5-nitro-1H-indazole

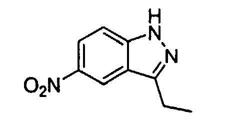

A solution of the compound prepared in step 1 (1.85 g, 9.34 mmol) hydrazine (0.33 mL, 10.3 mmol) in ethylene glycol (50 mL) is heated to 165 °C overnight. The reaction mixture cooled to room temperature and is extracted with EtOAc (3 x 150 mL). The combined organic layers were washed with H₂O (2 x 50 mL), and dried over Na₂SO₄. The solvent is removed under the reduced pressure and the crude product is purified by silica gel column chromatography (Hex/EtOAc, 2:1, Rf = 0.45) to afford the nitroindazole, 0.89 g (50%)..
1H NMR (CDCl3) δ 8.60 (1H, s), 8.16 (1H, dd, J = 1.5, 6.9 Hz), 7.38 (1H, d, J = 6.9 Hz). 2.95 (2H, t, J = 5.7 Hz), 1.33 (3H, t, J = 5.7 Hz).

### Step 3: Preparation of 3-ethyl-1H-indazol-5-amine

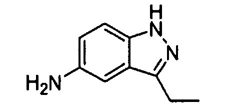

To a dry flask, purged with Argon, is added Pd/C followed by MeOH (20 mL). The nitro indazole of Step 2 is then added (0.89 g,) and the reaction is then charged with H₂ (1 atm). The reaction mixture is stirred for 4 h and then filtered through a Celite® plug. The solvent is evaporated under reduced pressure to give a yellow crude product. Purification of the crude product by silica gel column chromatography (Hex/EtOAc, 2:1 - 1:2) afforded pure product, 0.68 g (91%): ¹H NMR (CD₃OD) δ 7.16 (1H, d, J = 6.6 Hz), 6.90 (1H, d, J = 0.6 Hz), 6.85 (1H, dd, J = 12.6, 1.5 Hz). 2.80 (2H, t, J = 5.7 Hz), 1.23 (3H, t, J = 5.7 Hz).

### Step 4, Intermediate (D): Preparation of 2-chloro-N-(3-ethyl-1H-indazol-5-yl)-4-quinazolinamine

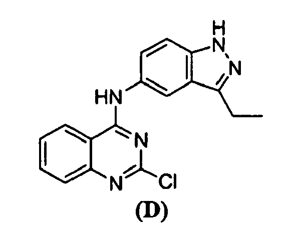

Reaction of the aminoindazole of Step 3 with 2,4-dichlorquinazoline in a manner analogous to Example **1,** Step 2 provided the desired Intermediate D which is used in the following steps without further purification.

### Step 5,: Preparation of N-(3-ethyl-1H-indazol-5-yl)-2-(4-methoxyphenyl)-4-quinazolinamine

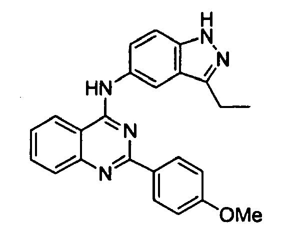

By following a procedure analogous to Example **1**, Step 3, and using intermediate **D** and the 4-methoxoyphenyl boronic acid as starting material, the product is prepared and characterized: 1H NMR (CD3OD, δ ppm) 8.58 (1H, d, J = 6.3 Hz), 8.44-8.39 (3H, m), 7.83-7.81 (2H, m), 7.75 (1H, dd, J = 1.5, 6.6 Hz), 7.56-7.53 (2H, m), 7.02 (2H, d, J = 5.1 Hz), 3.79 (3H, s), 2.79 (2H, q, J = 5.7 Hz), 1.20 (3H, t, J = 5.7 Hz).

The preceding examples can be repeated with similar success by substituting the generically or specifically described reactants and/or operating conditions of this invention for those used in the preceding examples. By so doing the following compounds are also prepared:

## Claims

1. A compound of Formula I 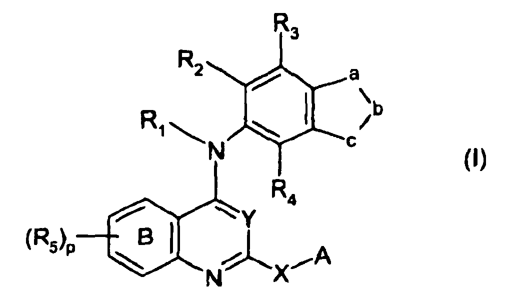 wherein Y is =N- or =CR₁₇,
X is -(CH₂)ₓ-, -O-(CH₂)ₙ-, -S-(CH₂)ₙ-, -NR₇-CO-(CH₂)ₙ-,
-NR₇-SO₂-(CH₂)ₙ-, -NR₇-(CH₂)ₙ-, or -(O)C-NR₇-,
each n is an integer which is independently 0, 1, 2 or 3,
x is 0-3
p is 0-3
a and c are each independently -CR5=, -N=, or -NR6-, wherein one of a or c is -NR6-, and b is -CR5= or -N=;
A is -CO-OR₈, -CO-R₈, cyano, -NR₈R₉, -CO-NR₈R₉, -NR₈-CO-R₉, -NR₈-CO-OR₉, -NR₈₋SO₂-R₉, -SR₈, -SO₂-R₈, -SO₂-NR₈R₉, NR₈-CO-NHR₉,
or
A is a 3-20 atom, cyclic or polycyclic moiety, containing 1-4 rings, which optionally contain 1-3 N, O or S atoms per ring, and may optionally be aryl or heteroaryl, which cyclic or polycyclic moiety may optionally be substituted up to 3 times by (i) C₁-C₁₀ alkyl or C₂-C₁₀-alkenyl, each optionally substituted with halogen up to perhalo; (ii) C₃-C₁₀ cycloalkyl; (iii) aryl; (iv) heteroaryl; said (iii) aryl or said (iv) heteroaryl being selected from the group consisting of : 2- or 3-furyl, 2- or 3-thienyl, 2- or 4-triazinyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or 5-yl, 1,2,4-triazol-1-, -3-or 5-yl, 1- or 5-tetrazolyl, 1,2,3-oxadiazol-4- or 5-yl, 1,2,4-oxadiazol-3- or 5-yl, 1,3,4-thiadiazol-2- or 5-yl, 1,2,4-oxadiazol-3- or 5-yl, 1,3,4-thiadiazol-2- or 5-yl, 1,3,4-thiadiazol-3- or 5-yl, 1,2,3-thiadiazol-4- or 5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-, 6- or 7-benzothiazolyl, 2-, 4-, 5-, 6- or 7-benzisothiazolyl, 2-, 4-, 5-, 6- or 7-benz-1,3-oxadiazolyl, 2-, 3-, 4-, 5-, 6-, 7- or 8-quinolinyl, 1-, 3-, 4-, 5-, 6-, 7-, 8- isoquinolinyl, 1-, 2-, 3-, 4- or 9-carbazolyl, 1-, 2-, 3-, 4-, 5-, 6-, 7-, 8- or 9-acridinyl, or 2-, 4-, 5-, 6-, 7- or 8-quinazolinyl, or additionally optionally substituted phenyl, 2- or 3-thienyl, 1,3,4-thiadiazolyl, 3-pyrryl, 3-pyrazolyl, 2-thiazolyl or 5-thiazolyl ; (v) halogen; (vi) -CO-OR₈; (vii) -CO-R₈; (viii) cyano; (ix) -NR₈R₁₃; (x) nitro; (xi) -CO-NR₈R₉; (xii) -C₁₋₁₀-alkyl-NR₈R₉; (xiii) -NR₈-CO-R₁₂; (xiv) -NR₈-CO-OR₉; (xv) -NR₈-SO₂-R₉; (xvi) -SR₈; (xvii) -SO₂-R₈; (xviii) -SO₂-NR₈R₉; or (xix) NR₈-CO-NHR₉;
Ring **B** is optionally independently substituted up to 3 times in any position by R₅,
R₁, and R₆-R₁₁ are each independently hydrogen or C₁₋₆ alkyl,
R₂-R₅ are each independently (i) hydrogen, (ii) C₁₋₁₀ alkyl or C₂₋₁₀-alkenyl each optionally substituted by amino, N-lower alkylamino, N,N-dilower alkylamino, N-lower alkanoylamino, hydroxy, cyano, -COOR₁₀, -COR₁₄, -OCOR₁₄, -OR₁₀, C₅₋₁₀₋heteroaryl, C₅₋₁₀-heteroaryloxy, or C₅₋₁₀-heteroaryl-C₁₋₁₀-alkoxy, halogen up to perhalo; (iii) C₃-C₁₀ cycloalkyl, in which 1-3 carbon atoms are optionally independently replaced by O, N or S; (iv) C₃₋₁₀-cycloalkenyl; (v) partially unsaturated C₅₋₁₀-heterocyclyl; (vi) aryl; (vii) heteroaryl; said (iii) aryl or said (iv) heteroaryl being selected from the group consisting of : 2- or 3-furyl, 2- or 3-thienyl, 2- or 4-triazinyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or 5-yl, 1,2,4-triazol-1-, -3-or 5-yl, 1- or 5-tetrazolyl, 1,2,3-oxadiazol-4- or 5-yl, 1,2,4-oxadiazol-3- or 5-yl, 1,3,4-thiadiazol-2- or 5-yl, 1,2,4-oxadiazol-3- or 5-yl, 1,3,4-thiadiazol-2- or 5-yl, 1,3,4-thiadiazol-3- or 5-yl, 1,2,3-thiadiazol-4- or 5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-, 6- or 7-benzothiazolyl, 2-, 4-, 5-, 6- or 7-benzisothiazolyl, 2-, 4-, 5-, 6- or 7-benz-1,3-oxadiazolyl, 2-, 3-, 4-, 5-, 6-, 7- or 8-quinolinyl, 1-, 3-, 4-, 5-, 6-, 7-, 8- isoquinolinyl, 1-, 2-, 3-, 4- or 9-carbazolyl, 1-, 2-, 3-, 4-, 5-, 6-, 7-, 8- or 9-acridinyl, or 2-, 4-, 5-, 6-, 7- or 8-quinazolinyl, or additionally optionally substituted phenyl, 2- or 3-thienyl, 1,3,4-thiadiazolyl, 3-pyrryl, 3-pyrazolyl, 2-thiazolyl or 5-thiazolyl ; (viii) halogen; (ix) -CO-OR₁₀; (x) -OCOR₁₀; (xi) -OCO₂R₁₀; (xii) -CHO; (xiii) cyano; (xiv) -OR₁₆; (xv) -NR₁₀R₁₅; (xvi) nitro; (xvii) -CO-NR₁₀R₁₁; (XVIII) -NR₁₀-CO-R₁₂; (xix) -NR₁₀-CO-OR₁₁; (xx) -NR₁₀-SO₂-R₁₂; (xxi) -SR₁₆; (xxii) -SOR₁₆; (xxiii) -SO₂-R₁₆; (xxiv) -SO₂-NR₁₀R₁₁; (xxv) NR₁₀-CO-NHR₁₁; (xxvi) amidino; (xxvii) guanldino; (xxviii) sulfo; (xxix) -B(OH)₂; (xxx)
-OCON(R₁₀)₂; or (xxxi) -NR₁₀CON(R₁₀)₂;
R₁₂ is H, C₁₋₆-alkyl or C₅₋₁₀-aryl,
R₁₃ is H, C₁₋₆-alkyl or C₁₋₆-alkoxy,
R₁₄ Is C₁₋₆ alkyl or phenyl;
R₁₅ is C₁₋₆ alkyl, halogen, amino, N-lower alkyl amino, N,N-dilower alkylamino, N-lower alkanoylamino, OH, CN, COOR₁₀, -COR₁₄ or -OCOR₁₄;
R₁₆ is hydrogen, C₁₋₆-alkyl optionally substituted by halogen, up to perhalo, or C₅₋₁₀₋heteroaryl; and
R₁₇ is H, C₁₋₆ alkyl or CN,
or a pharmaceutically acceptable salt thereof,
with the proviso that Formula I is not

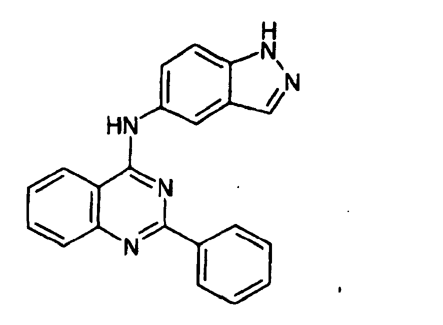

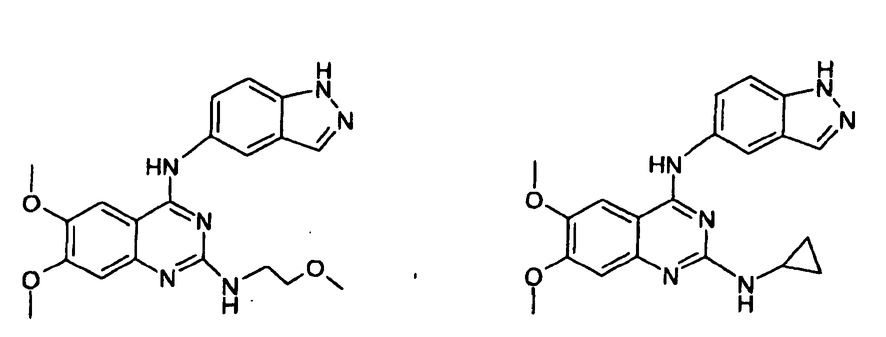

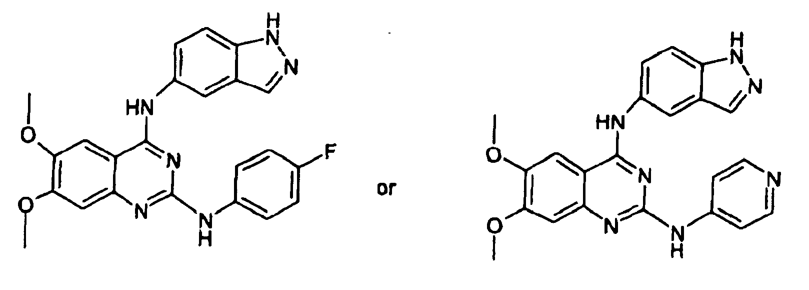

2. A compound according to claim 1, wherein A is a 5-12 carbon-atom aromatic ring or ring system containing 1-3 rings, at least one of which is aromatic, in which 1-4 carbon atoms in one or more of the rings is optionally replaced by oxygen, nitrogen or sulfur atoms.

3. A compound according to claim 1 or 2, wherein each ring in A has 3-7 atoms.

4. A compound according to any of claims 1 to 3, wherein A is 2- or 3-furyl, 2- or 3-thienyl, 2- or 4-triazinyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or 5-yl, 1,2,4-triazol-1-, -3- or 5-yl, 1- or 5-tetrazolyl, 1,2,3-oxadiazol-4- or 5-yl, 1,2,4-oxadiazol-3- or 5-yl, 1,3,4-thiadiazol-2- or 5-yl, 1,2,4-oxadiazol-3- or 5-yl, 1,3,4-thiadiazol-2- or 5-yl, 1,3,4-thiadiazol-3- or 5-yl, 1,2,3-thiadlazol-4- or 5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6-or 7-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-; 6- or 7-benzothiazolyl, 2-, 4-, 5-, 6- or 7-benzisothlazolyl, 2-, 4-, 5-, 6- or 7-benz-1,3-oxadiazolyl, 2-, 3-, 4-, 5-, 6-, 7- or 8-quinolinyl, 1-, 3-, 4-, 5-, 6-, 7-, 8- isoquinolinyl, 1-, 2-, 3-, 4- or 9-carbazolyl, 1-, 2-, 3-, 4-, 5-, 6-, 7-, 8- or 9-acridinyl, or 2-, 4-, 5-, 6-, 7- or 8-quinazolinyl, 2- or 3-thlenyl, 1,3,4-thiadiazolyl, 3-pyrryl, 3-pyrazolyl, 2-thiazolyl or 5-thiazolyl.

5. A compound according to claim 1, wherein A is cyclohexyl; or C₅₋₁₂-aryl or C₅₋₁₂-heteroaryl each independently optionally substituted up to three times by (I) C₁₋C₁₀-alkyl or C₂₋₁₀-alkenyl each optionally substituted with halogen up to perhalo; (ii) C₃-C₁₀ cycloalkyl; (iii) C₅₋₁₂-aryl optionally substituted by 1-3 halogen atoms; (iv) C₅₋₁₂-heteroaryl; (v) halogen; (vi) -CO-OR₈; (vii) -CO-R₈; (viii) cyano; (ix) -NR₈R₁₃; (x) nitro; (xi) -CO-NR₈R₉; (xii) -C₁₋₁₀-alkyl-NR₈R₉; (xiii) -NR₈-CO-R₁₂; (xiv) -NR₈-CO-OR₉; (xv) -NR₈-SO₂-R₉; (xvi) -SR₈; (xvii) -SO₂-R₈; (xviii) -SO₂-NR₈R₉, or (xix) NR₈-CO-NHR₉.

6. A compound according to any one of claims 1 to 3, wherein A is phenyl, pyridyl, pyrimidinyl, oxazolyl, furyl, thienyl, pyrrolyl, imidazolyl, isoxazolyl and pyrazinyl, each independently substituted up to three times by halogen, C₁₋₁₀-alkyl, C₁₋₁₀-alkoxyphenyl, naphthyl, -OR₁₀, 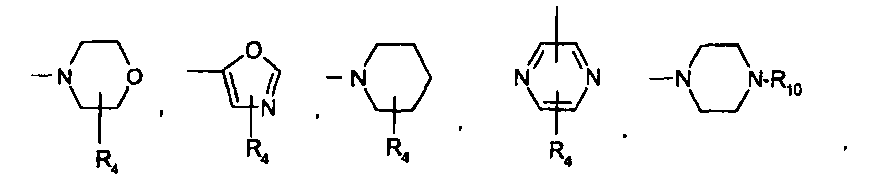 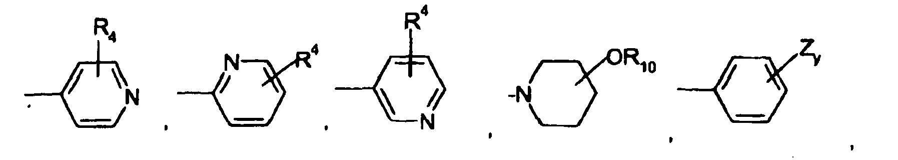 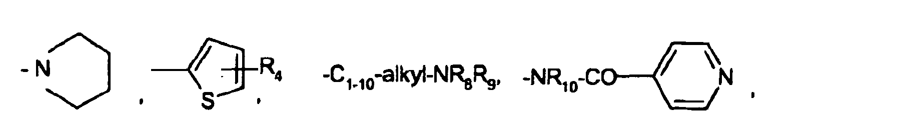 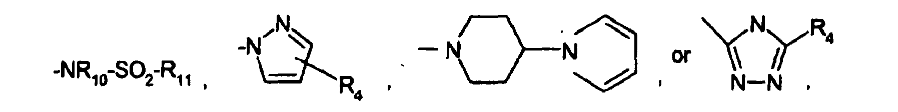 wherein each Z independently is halogen, hydroxy, hydroxy-C₁₋₁₀-alkyl, -CN, -NO₂, C₁₋₁₀-alkoxycarboxyl, -NR₁₀-CO-R₁₁, or -NR₁₀-CO-OR₁₁, and
y is 1-3.

7. A compound according to claim 1 or 3, wherein A is 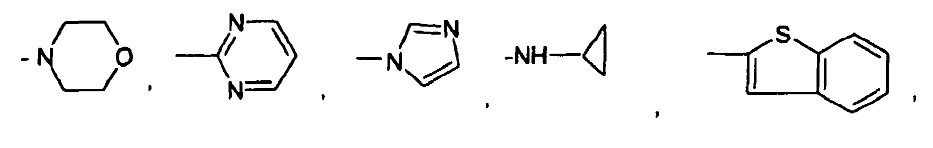 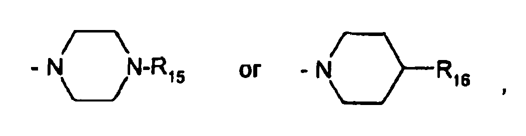 wherein R₁₅ is H; phenyl optionally substituted by C₁₋₁₀-alkyl, C₁₋₁₀-alkoxy, C₁₋₁₀₋alkylcarboxyl, or halogen; benzyl; pyrimidyl or pyridyl; and R₁₆ is H, phenyl, -COOR₁₀,

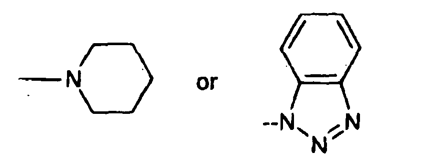

8. A compound according to claim 1, wherein R⁵ in a, b or c is hydrogen or C₁₋₁₀₋alkyl or C₂₋₁₀-alkyl optionally substituted by amino, N-lower alkylamino, N,N-dilower alkylamino, N-lower alkanoylamino, hydroxy, cyano, -COOR₁₀, -COR₁₄, -OCOR₁₄, -OR₁₀, C₅₋₁₀-heteroaryl, C₅₋₁₀-heteroaryloxy, or C₅₋₁₀-heteroaryl-C₁₋₁₀-alkoxy, halogen up to perhalo; (iii) C₃-C₁₀ cycloalkyl, in which 1-3 carbon atoms are optionally independently replaced by O, N or S; (iv) C₃₋₁₀-cycloalkenyl; (v) partially unsaturated C₅₋₁₀-heterocyclyl; (vi) aryl; (vii) heteroaryl; (viii) halogen; (ix) -CO-OR₁₀; (x) -OCOR₁₀; (xi) -OCO₂R₁₀; (xii) -CHO; (xiii) cyano; (xiv) -OR₁₆; (xv) -NR₁₀R₁₅; (xvi) nitro; (xvii) -CO-NR₁₀R₁₁; (xviii) -NR₁₀-CO-R₁₂; (xix) -NR₁₀-CO-OR₁₁; (xx) -NR₁₀-SO₂₋R₁₂; (xxi) -SR₁₆; (xxii) -SOR₁₆; (xxiii) -SO₂-R₁₆; (xxiv) -SO₂-NR₁₀R₁₁; (xxv) NR₁₀-CO-NHR₁₁; (xxvi) amidino; (xxvii) guanidino; (xxviii) sulfo; (xxix) -B(OH)₂; (xxx) -OCON(R₁₀)₂; or (xxxi) -NR₁₀CON(R₁₀)₂.

9. A compound according to claim 1, wherein Y is N and R₁ is H.

10. A compound according to claim 9, wherein a is -NR₆-, R₆ is H, and c is -N=.

11. A compound according to claim 10, wherein p is 0 and R₁₋₄ are H.

12. A compound according to claim 11, wherein X is -(CH₂)ₓ- and x is 0.

13. A compound according to claim 12, wherein A is biphenyl optionally substituted by halogen.

14. A compound according to claim 1, of the formula:
2-(2,4-dichlorophenyl)-*N*-(1*H*-indazol-5-yl)-4-quinazolinamine, 2-(4-chlorophenyl)-*N-*(1*H*-indazol-5-yl)-4-quinazolinamine, 1-{4-[4-(1*H*-indazol-5-ylamino)-2-quinazolinyl]phenyl}ethanone, *N*-(1*H*-indazol-5-yl)-2-[4-(trifluoromethyl)phenyl]-4-quinazolinamine, 2-(3-chloro-4-fluorophenyl)-*N*-(1*H*-indazol-5-yl)-4-quinazolinamine, 2-(1,3-benzodioxol-5-yl)-*N*-(1*H*-indazol-5-yl)-4-quinazolinamine, *N*-(1*H*-indazol-5-yl)-2-(4-methylphenyl)-4-quinazolinamine, 2-(3,4-dichlorophenyl)-*N*-(1*H*-indazol-5-yl)-4-quinazolinamine, *N*-(1*H*-indazol-5-yl)-2-(1-naphthyl)-4-quinazolinamine, *N*-(1*H-*indazol-5-yl)-2-(3,4,5-trimethoxyphenyl)-4-quinazolinamine, 2-(1-benzofuran-2-yl)-*N-*(1*H*-indazol-5-yl)-4-quinazolinamine, *N*-(1*H*-indazol-5-yl)-2-(2-thienyl)-4-quinazolinamine, *N*-(1*H*-indazol-5-yl)-2-(3-thienyl)-4-quinazolinamine, 2-(3,5-dimethyl-4-isoxazolyl)-*N*-(1*H*-indazol-5-yl)-4-quinazolinamine, 2-(1,1'-biphenyl-4-yl)-*N*-(1*H*-indazol-5-yl)-4-quinazolinamine, 2-[4-(dimethylamino)phenyl]-*N*-(1*H*-indazol-5-yl)-4-quinazolinamine, 2-(1-benzothie *N*-2-yl)-*N*-(1*H*-indazol-5-yl)-4-quinazolinamine, 4-[4-(1*H*-indazol-5-ylamino)-2-quinazolinyl]phenol, 2-dibenzo[b,d]furan-1-yl-*N*-(1*H*-indazol-5-yl)-4-quinazolinamine, 2-(2-fluoro-1,1'-biphenyl-4-yl)-*N*-(1*H*-indazol-5-yl)-4-quinazolinamine, 7-chloro-*N*-(1*H*-indazol-5-yl)-2-phenyl-4-quinazolinamine, *N*-(1*H*-indazol-5-yl)-6-nitro-2-phenyl-4-quinazolinamine, 2-(4-fluorophenyl)-*N*-(1*H*-indazol-5-yl)-6-nitro-4-quinazolinamine, 6-chloro-*N*-(1*H-*indazol-5-yl)-2-(4-methylphenyl)-4-quinazolinamine, 6-chloro-2-(4-fluorophenyl)-*N*-(1*H*-indazol-5-yl)-4-quinazolinamine, 2-(4-bromophenyl)-6-chloro-*N*-(1*H*-indazol-5-yl)-4-quinazolinamine, *N*-(1*H*-indazol-5-yl)-2-(2-quinoxalinyl)-4-quinazolinamine, 5-fluoro-*N*-(1*H*-indazol-5-yl)-2-(2-methylphenyl)-4-quinazolinamine, 5-fluoro-2-(4-fluorophenyl)-*N*-(1*H*-indazol-5-yl)-4-quinazolinamine, 2-(3-chlorophenyl)-5-fluoro-*N*-(1*H*-indazol-5-yl)-4-quinazolinamine, 2-(4-bromophenyl)-5-fluoro-*N*-(1*H*-indazol-5-yl)-4-quinazolinamine, 5-fluoro-*N*-(1*H*-indazol-5-yl)-2-(3-methylphenyl)-4-quinazolinamine hydrochloride, 2-(3-bromophenyl)-5-fluoro-*N*-(1*H*-indazol-5-yl)-4-quinazolinamine hydrochloride, 2-(2-chlorophenyl)-5-fluoro-*N*-(1*H*-indazol-5-yl)-4-quinazolinamine, 5-fluoro-*N*-(1*H*-indazol-5-yl)-2-(2-quinoxalinyl)-4-quinazolinamine tris(trifluoroacetate), 5-fluoro-*N*-(1*H*-indazol-5-yl)-2-(1-naphthyl)-4-quinazolinamine bis(trifluoroacetate), 5-fluoro-*N*-(1*H*-indazol-5-yl)-2-(2-naphthyl)-4-quinazolinamine bis(trifluoroacetate), 5-fluoro-*N*-(1*H*-indazol-5-yl)-2-(4-pyridinyl)-4-quinazolinamine tris(trifluoroacetate), *N-*(1*H*-indazol-5-yl)-7-methyl-2-(2-quinoxalinyl)-4-quinazolinamine, 2-(3-chlorophenyl)-*N*-(1*H*-indazol-5-yl)-7-methyl-4-quinazolinamine, 2-(4-fluorophenyl)-*N*-(1*H*-Indazol-5-yl)-7-methyl-4-quinazolinamine, *N*-(1*H*-indazol-5-yl)-7-methyl-2-(4-methylphenyl)-4-quinazolinamine", 2-(4-bromophenyl)-*N*-(1*H*-indazol-5-yl)-7-methyl-4-quinazolinamine, *N*-(1*H*-indazol-5-yl)-7-methyl-2-(2-methylphenyl)-4-quinazolinamine bis(trifluoroacetate), *N*-(1*H*-indazol-5-yl)-7-methyl-2-(3-methylphenyl)-4-quinazolinamine bis(trifluoroacetate), *N*-[2-(3-fluorophenyl)-7-methyl-4-quinazolinyl]-*N*-(1*H*-indazol-5-yl)amine bis(trifluoroacetate), 2-(3-bromophenyl)-*N*-(1*H*-indazol-5-yl)-7-methyl-4-quinazolinamine bis(trifluoroacetate), *N*-[2-(2-chlorophenyl)-7-methyl-4-quinazolinyl]-*N*-(1*H*-indazol-5-yl)amine bis(trifluoroacetate), 2-(3-furyl)-*N-*(1*H*-indazol-5-yl)-7-methyl-4-quinazolinamine bis(trifluoroacetate), *N*-(1*H*-indazol-5-yl)-7-methyl-2-(1-naphthyl)-4-quinazolinamine bis(trifluoroacetate), *N*-(1*H*-indazol-5-yl)-7-methyl-2-(2-naphthyl)-4-quinazolinamine bis(trifluoroacetate), *N*-(1*H*-indazol-5-yl)-7-methyl-2-(3-pyridinyl)-4-quinazolinamine tris(trifluoroacetate), *N*-(1*H*-indazol-5-yl)-7-methyl-2-(4-pyridinyl)-4-quinazolinamine tris(trifluoroacetate), 7-chloro-2-(3-chlorophenyl)-*N*-(1*H*-indazol-5-yl)-4-quinazolinamine, 7-chloro-*N*-(1*H*-indazol-5-yl)-2-(4-methylphenyl)-4-quinazolinamine, 2-(4-bromophenyl)-7-chloro-*N*-(1*H*-indazol-5-yl)-4-quinazolinamine, 7-chloro-*N*-(1*H*-indazol-5-yl)-2-(3-methylphenyl)-4-quinazolinamine hydrochloride, 7-chloro-2-(3-fluorophenyl)-*N*-(1*H*-indazol-5-yl)-4-quinazolinamine bis(trifluoroacetate), 2-(3-bromophenyl)-7-chloro-*N*-(1*H*-indazol-5-yl)-4-quinazolinamine bis(trifluoroacetate), *N*-[7-chloro-2-(2-furyl)-4-quinazolinyl]-*N*-(1*H*-indazol-5-yl)amine bis(trifluoroacetate), 7-chloro-*N*-(1*H*-indazol-5-yl)-2-(2-quinoxalinyl)-4-quinazolinamine tris(trifluoroacetate), 7-chloro-*N*-(1*H-*indazol-5-yl)-2-(1-naphthyl)-4-quinazolinamine bis(trifluoroacetate), 7-chloro-*N*-(1*H-*indazol-5-yl)-2-(2-naphthyl)-4-quinazolinamine bis(trifluoroacetate), 7-chloro-*N*-(1*H-*indazol-5-yl)-2-(3-pyridinyl)-4-quinazolinamine tris(trifluoroacetate), 2-(4-fluorophenyl)-*N*-(1*H*-indazol-5-yl)-6,7-dimethoxy-4-quinazolinamine, 2-(1,1'-biphenyl-4-yl)-*N*-(1*H*-indazol-5-yl)-6,7-dimethoxy-4-quinazolinamine, *N*-(1*H*-indazol-5-yl)-6,7-dimethoxy-2-(4-vinylphenyl)-4-quinazolinamine, *N*-cyclopentyl-4-(1*H*-indazol-5-ylamino)-2-quinazolinecarboxamide, *N*-(3-fluorophenyl)-*N*-[4-(1*H*-indazol-5-ylamino)-6,7-dimethoxy-2-quinazolinyl]amine, *N*-(2,4-difluorobenzyl)-*N*-[4-(1*H*-indazol-5-ylamino)-6,7-dimethoxy-2-quinazolinyl]amine, *N*-(2-fluorobenzyl)-*N*-[4-(1*H*-indazol-5-ylamino)-6,7-dimethoxy-2-quinazolinyl]amine, *N*-(4-bromophenyl)-*N*-[4-(1*H*-indazol-5-ylamino)-6,7-dimethoxy-2-quinazolinyl]amine, *N*-(6,7-dimethoxy-2-{[4-(trifluoromethyl)phenyl]amino}-4-quinazolinyl)-*N*-(1*H*-indazol-5-yl)amine, *N*-(6,7-dimethoxy-2-{[4-(trifluoromethyl)benzyl]amino}-4-quinazolinyl)-*N*-(1*H*-indazol-5-yl)amine, *N*-[3-fluoro-5-(trifluoromethyl)benzyl]-*N*-[4-(1*H*-indazol-5-ylamino)-6,7-dimethoxy-2-quinazolinyl]amine, *N*-(3-fluorobenzyl)-*N*-[4-(1*H*-indazol-5-ylamino)-6,7-dimethoxy-2-quinazolinyl]amine, *N*-(2,4-difluorobenzyl)-*N*-[4-(1*H*-indazol-5-ylamino)-6,7-dimethoxy-2-quinazolinyl]amine, *N*-(4-fluorobenzyl)-*N*-[4-(1*H*-indazol-5-ylamino)-6,7-dimethoxy-2-quinazolinyl]amine, *N*-(2,6-difluorobenzyl)-*N*-[4-(1*H*-indazol-5-ylamino)-6,7-dimethoxy-2-quinazolinyl]amine, *N*-(3,5-difluorobenzyl)-*N*-[4-(1*H-*indazol-5-ylamino)-6,7-dimethoxy-2-quinazolinyl]amine, *N*-(3-bromophenyl)-*N*-[4-(1*H*-indazol-5-ylamino)-6,7-dimethoxy-2-quinazolinyl]amine, *N*-(2,6-difluorophenyl)-*N*-[4-(1*H*-indazol-5-ylamino)-6,7-dimethoxy-2-quinazolinyl]amine, *N*-(2,5-difluorophenyl)-*N*-[4-(1*H*-indazol-5-ylamino)-6,7-dimethoxy-2-quinazolinyl]amine, *N-*(2,4-difluorophenyl)-*N*-[4-(1*H*-indazol-5-ylamino)-6,7-dimethoxy-2-quinazolinyl]amine, *N*-(2,3-difluorophenyl)-*N*-[4-(1*H*-indazol-5-ylamino)-6,7-dimethoxy-2-quinazolinyl]amine, *N*-(3,4-difluorophenyl)-*N*-[4-(1*H*-indazol-5-ylamino)-6,7-dimethoxy-2-quinazolinyl] amine, *N*-(3,5-difluorophenyl)-*N*-[4-(1*H*-indazol-5-ylamino)-6,7-dimethoxy-2-quinazolinyl] amine, *N*-{6,7-dimethoxy-2-[(2,3,4-trifluorophenyl)amino]-4-quinazolinyl}-*N*-(1*H*-indazol-5-yl)amine, *N*-{6,7-dimethoxy-2-[(2,4,5-trifluorophenyl)amino]-4-quinazolinyl}-*N*-(1H-indazol-5-yl)amine, *N*-{6,7-dimethoxy-2-[(2,4,6-trifluorophenyl)amino]-4-quinazolinyl}-*N*-(1*H*-indazol-5-yl)amine, *N*-{6,7-dimethoxy-2-[(2,3,6-trifluorophenyl)amino]-4-quinazolinyl}-*N*-(1*H-*indazol-5-yl)amine, *N*-(4-bromophenyl)-*N*-[4-(1*H*-indazol-5-ylamino)-6,7-dimethoxy-2-quinazolinyl]amine, 2-(3-aminophenyl)-*N*-(1*H*-indazol-5-yl)-4-quinazolinamine, *N-*{3-[4-(1*H*-indazol-5-ylamino)-2-quinazolinyl]phenyl}isonicotinamide, *N*-{3-[4-(1*H-*indazol-5-ylamino)-2-quinazolinyl]phenyl}acetamide, *N*-(4-chlorophenyl)-*N*-[4-(1*H-*indazol-5-ylamino)-2-quinazolinyl]amine, *N*-(3-bromophenyl)-*N*-[4-(1*H*-indazol-5-ylamino)-2-quinazolinyl]amine, *N*-(2-chlorophenyl)-*N*-[4-(1*H*-indazol-5-ylamino)-2-quinazolinyl]amine, *N*-(3-fluorophenyl)-*N*-[4-(1*H*-indazol-5-ylamino)-2-quinazolinyl]amine, *N*-(2-fluorophenyl)-*N*-[4-(1*H*-indazal-5-ylamino)-2-quinazolinyl]amine, *N*-(3-chlorophenyl)-*N*-[4-(1*H*-indazol-5-ylamino)-2-quinazolinyl]amine, *N*-(4-bromophenyl)-*N*-[4-(1*H*-indazol-5-ylamino)-2-quinazolinyl]amine, *N*-(1*H*-indazol-5-yl)-*N*-(2-{[3-(trifluoromethyl)phenyl]amino}-4-quinazolinyl)amine, *N*-(1*H*-indazol-5-yl)-*N*-{2-[(4-phenoxyphenyl)amino]-4-quinazolinyl}amine, *N*-(1*H*-indazol-5-yl)-*N*-(2-{[4-(trifluoromethoxy)phenyl]amino}-4-quinazolinyl)amine, *N*-(1*H*-indazol-5-yl)-*N*-(2-{[3-(trifluoromethoxy)phenyl]amino}-4-quinazolinyl)amine, *N*-(4-fluorophenyl)-*N*-[4-(1*H*-indazol-5-ylamino)-2-quinazolinyl]amlne, *N*-(2-anilino-4-quinazolinyl)-*N*-(1*H*-indazol-5-yl)amine, 2-[4-(2-chlorophenyl)-1-piperazinyl]-*N*-(1*H*-indazol-5-yl)-4-quinazolinamine, *N*-(1*H*-indazol-5-yl)-2-[4-(2-pyrimidinyl)-1-piperazinyl]-4-quinazolinamine, *N*-(1*H*-indazol-5-yl)-2-[4-(2-methoxyphenyl)-1-piperazinyl]-4-quinazolinamine, 1-(4-{4-[4-(1*H*-indazol-5-ylamino)-2-quinazolinyl]-1-piperazinyl}phenyl)ethanone, 4-(1*H*-indazol-5-ylamino)-2-quinazolinecarboxamide", 4-(1*H*-indazol-5-ylamino)-*N*-(4-pyridinyl)-2-quinazolinecarboxamide, 4-(1*H*-indazol-5-ylamino)-*N*-(4-methoxyphenyl)-2-quinazolinecarboxamide, *N*-cyclohexyl-4-(1*H*-indazol-5-ylamino)-2-quinazolinecarboxamide, *N*-cyclopentyl-4-(1*H*-indazol-5-ylamino)-2-quinazolinecarboxamide, 4-(1*H*-indazol-5-ylamino)-*N*-(2-pyridinyl)-2-quinazolinecarboxamide, 4-(1*H*-indazol-5-ylamino)-*N*-(3-quinolinyl)-2-quinazolinecarboxamide, 4-(1*H*-indazol-5-ylamino)-*N*-methyl-2-quinazolinecarboxamide, *N*-(1*H*-indazol-5-yl)-2-(4-morpholinylcarbonyl)-4-quinazolinamine, 2-(2,3-dihydro-1-benzofuran-5-yl)-*N*-(1*H*-indazol-5-yl)-4-quinazolinamine, 2-cyclopropyl-*N*-(1*H*-indazol-5-yl)-4-quinazolinamine, 2-(2-fluoro-1,1'-biphenyl-4-yl)-*N*-(1*H*-indazol-5-yl)-4-quinazolinamine dihydrochloride, 2-(2-fluoro-1,1'-biphenyl-4-yl)-*N*-(1*H*-indazol-5-yl)-4-quinazolinamine dimethanesulfonate, 2-(2-fluoro-1,1'-biphenyl-4-yl)-*N*-(1*H*-indazol-5-yl)-4-quinazolinamine benzenesulfonate, 2-(2-fluoro-1,1'-biphenyl-4-yl)-*N*-(1*H*-indazol-5-yl)-4-quinazolinamine 4-methylbenzenesulfonate, or 2-dibenzo[*b,d*]furan-1-yl-*N*-(1*H*-indazol-5-yl)-4-quinazolinamine trifluoroacetate, 2-(3-fluoro-1,1-biphenyl-4-yl)-*N*-(1*H*-indazol-5-yl)-4-quinazolinamine; 2-(2-furyl)-*N*-(1*H*-indazol-5-yl)-7-methyl-4-quinazolinamine; 2-(1-benzofuranyl)-*N*-(1*H*-indazol-5-yl)-6,7-dimethoxy-4-quinazolinamine; *N*-(2,5-difluorobenzyl)-*N*-[4-(1*H*-indazol-5-ylamino)-6,7-dimethoxy-2-quinazolinyl]amine; *N-*{6,7-dimethoxy-2-[(2,3,5-trifluorophenyl)amino]-4-quinazolinyl}-*N*-(1*H*-indazol-5-yl)amine; *N*-(1*H*-indazol-5-yl)-2-[5-(1*H*-indolyl)amino]-4-quinazolinamine; *N*-(1*H-*indazol-5-yl)-2-[4-phenoxyanilino]-4-quinazolinamine; *N*-(1*H*-indazol-5-yl)-2-[2-naphthylamino]-4-quinazolinamine.

15. A compound of formula :
*N*-(1*H*-indazol-5-yl)-2-(trifluoromethyl)-4-quinazolinamine ; 2-chloro-*N*-(3-ethyl-1*H-*indazol-5-yl)-4-quinazolinamine ; 2-chloro-*N*-(1*H*-indazol-5-yl)-4-quinazolinamine.

16. A process for the preparation of a compound of claim 1, comprising
(a) reacting a compound of formula II 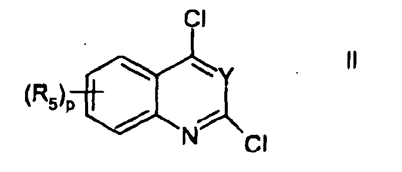 with a compound of formula III 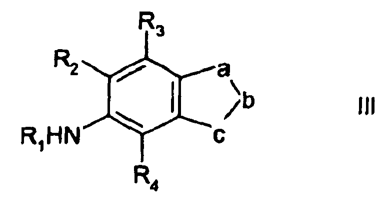 in the presence of a base, to produce a compound of formula IV 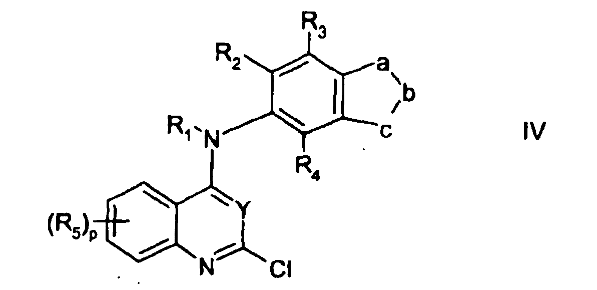 and optionally further reacting IV with arylboronic acid or A-NH₂, or
(b) reacting a substituted benzoyl chloride with dimethylamine to produce a compound of formula V 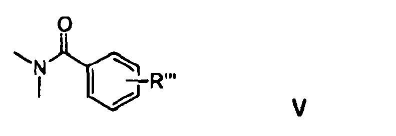
wherein R''' is (i) C₁-C₁₀ alkyl or C₂-C₁₀-alkenyl, each optionally substituted with halogen up to perhalo; (ii) C₃-C₁₀ cycloalkyl; (iii) aryl; (iv) heteroaryl; (v) halogen; (vi) -CO-OR₈; (vii) -CO-R₈; (viii) cyano; (ix) -OR₈, (x) (x) -NR₈R₁₃; (xi) nitro; (xii) -CO-NR₈R₉; (xiii) -C₁₋₁₀-alkyl-NR₈R₉;(xiv) -NR₈-CD-R₁₂; (xv) -NR₈-CO-OR₉; (xvi) -NR₈₋SO₂-R₉; (xvii) -SR₈; (xviii) -SO₂-R₈; (xix) -SO₂-NR₈R₉; or (xx) NR₈-CO-NHR₉, reacting V with chloro-2-amino-benzonitrile to produce a compound of formula VI 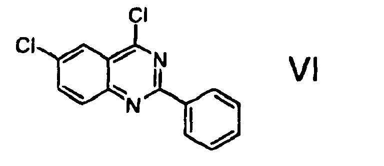 and reacting VI with aminoindazole.

17. A process for preparing 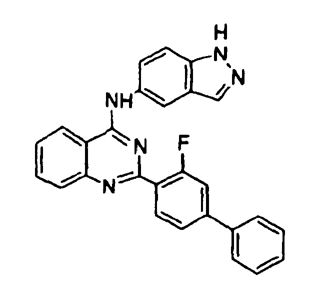 comprising reacting 3-fluoro-4-phenylbenzoic acid 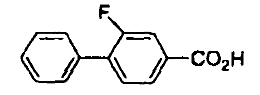 with 4-bromo-2-fluorobiphenyl to produce 2[(3-fluoro-4-phenylphenyl)carbonylamino]benzamide 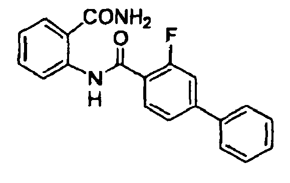 cyclizing to produce 2-(3-fluoro-1,1'-biphenyl-4-yl)-4(3*H*)-quinazolinone 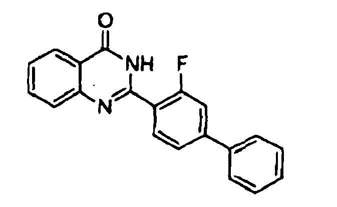 reacting to produce 4-chloro-2-(3-fluoro-4-phenylphenyl)quinazoline 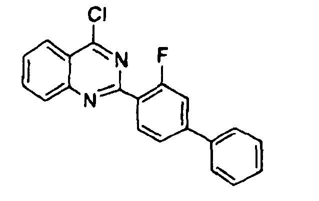 and then reacting with aminoindazole to produce

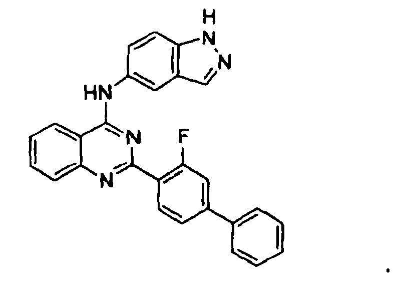

18. A pharmaceutical composition, **characterised in that** it includes one or more compounds according to any one of claims 1 to 17, in association with one or more non-toxic pharmaceutically acceptable carriers, and, optionally, other active ingredients.

19. Use of a compound of any one of claims 1 to 18 for the preparation of a medicament for treating an indication mediated by Rho-kinase.

20. Use of a compound of claim 13 for the preparation of a medicament for treating an indication mediated by Rho-kinase.

21. Use of a compound of claim 14 for the preparation of a medicament for treating an indication mediated by Rho-kinase.

22. Use of a compound of claim 15 for the preparation of a medicament for treating an indication mediated by Rho-kinase.

23. Use of a compound according to any one of claims 1 to 18 for the preparation of a medicament for treating hypertension, atherosclerosis, restenosis, cerebral ischemia, cerebral vasospasm, neuronal degeneration, spinal cord injury, cancer of the breast, colon, prostate, ovaries, brain or lung, thrombotic disorders, asthma, glaucoma, osteoporosis or erectile dysfunction.

24. Use of a compound according to claim 13 for the preparation of a medicament for treating hypertension, atherosclerosis, restenosis, cerebral ischemia, cerebral vasospasm, neuronal degeneration, spinal cord injury, cancer of the breast, colon, prostate, ovaries, brain or lung, thrombotic disorders, asthma, glaucoma, osteoporosis or erectile dysfunction.

25. Use of a compound according to claim 14 for the preparation of a medicament for treating hypertension, atherosclerosis, restenosis, cerebral ischemia, cerebral vasospasm, neuronal degeneration, spinal cord injury, cancer of the breast, colon, prostate, ovaries, brain or lung, thrombotic disorders, asthma, glaucoma and osteoporosis or erectile dysfunction.

26. Use of a compound according to claim 15 for the preparation of a medicament for treating hypertension, atherosclerosis, restenosis, cerebral ischemia, cerebral vasospasm, neuronal degeneration, spinal cord injury, cancer of the breast, colon, prostate, ovaries, brain or lung, thrombotic disorders, asthma, glaucoma and osteoporosis or erectile dysfunction.

27. Use according to any one of claims 18 to 26, wherein the host is a human.

## Patentansprüche

1. Verbindung mit Formel I 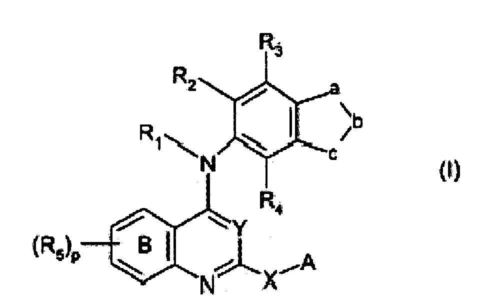 worin Y =N- oder =CR₁₇ ist,
X -(CH₂)x-, -O-(CH₂)n-, -S-(CH₂)n-, -NR₇-CO-(CH₂)n-,
-NR₇-SO₂-(CH₂)n-, -NR₇-(CH₂)n-, oder -(O)C-NR₇- ist,
jedes n eine ganze Zahl ist, die unabhängig 0,1,2 oder 3 ist,
x 0-3 ist,
p 0-3 ist,
a und c jedes unabhängig -CR₅=,-N=, oder -NR₆- ist, worin eines von a oder c -NR6-, und b -CR5= oder -N= ist;
A H, Halogen,-CO-OR₈, -CO-R₈, Cyano, -NR₈R₉, -CO-NR₈R₉, -NR₈-CO-R₉, -NR₈-CO-OR₉, -NR₈-SO₂-R₉, -SR₈, -SO₂-R₈, -SO₂-NR₈R₉,NR₈-CO-NHR₉ ist, oder A eine zyklische oder polyzyklische Einheit mit 3-20 Atomen ist, die 1-4 Ringe enthält, welche optional 1-3 N- ,O- oder S-Atome pro Ring enthält und optional Aryl oder Heteroaryl sein kann, welche zyklische oder polyzyklische Einheit optional bis zu dreimal substituiert sein kann durch (i) C₁-C₁₀-Alkyl oder C₂-C₁₀₋Alkenyl, jedes optional substituiert mit Halogen bis Perhalogen; (ii) C₃-C₁₀₋Zykloalkyl ; (iii) Aryl; (iv) Heteroaryl;
wobei das (iii) Aryl oder das (iv) Heteroaryl gewählt ist aus der Gruppe, die besteht aus:
2- oder 3-Furyl, 2- oder 3-Thienyl, 2- oder 4-Triazinyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4 Pyridyl, 2-, 4-, 5- oder 6-Pyrimidinyl, 1,2,3-Triazol-1-, -4- oder 5-yl, 1,2,4-Triazol-1-, -3- oder 5-yl, 1 oder 5-Tetrazolyl, 1,2,3-Oxadiazol-4- oder 5-yl, 1,2,4-Oxadiazol-3- oder 5-yl, 1,3,4-Thiadiazol-2- oder 5-yl, 1,2,4-Oxadiazol-3- oder 5-yl, 1,3,4-Thiadiazol-2- oder 5-yl, 1,3,4-Thiadiazol-3- oder 5-yl, 1,2,3-Thiadiazol-4- oder 5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-6- oder 7-Benzisoxazolyl, 1-, 3-, 4-, 5 , 6- oder 7-Benzothiazolyl, 2-, 4-, 5-, 6- oder 7-Benzisothiazolyl, 2-, 4-, 5-, 6- oder 7-Benz-1,3-Oxadiazolyl, 2-, 3-, 4-, 5-, 6-, 7- oder 8-Quinolinyl, 1-, 3-, 4-, 5-, 6-, 7-, 8-Isoquinolinyl, 1-, 2-, 3-, 4- oder 9-Carbazolyl, 1-, 2-, 3-, 4-, 5-, 6-, 7-, 8- oder 9-Acridinyl, oder 2-, 4-, 5-, 6-, 7- oder 8-Quinazolinyl oder zusätzlich optional substituiertes Phanyl, 2- oder 3-Thienyl, 1,3,4-Thiadiazolyl, 3-Pyrryl, 3-Pyrazolyl, 2-Thiazolyl oder 5-Thiazolyl; (v) Halogen; (vi)-CO-OR₈; (vii)-CO-R₈; (viii) Cyano; (ix) -NR₈R₁₃; (x) Nitro; (xi) -CO-NR₈R₉; (xii) -C₁₋₁₀-Alkyl-NR₈R₉ ;(xiii) -NR₈-CO-R₁₂ ; (xiv) NR₈-CO-OR₉; (xv) -NR₈-SO₂-R₉ ;(xvi) -SR₈; (xvii) -SO₂-R₈; (xviii) -SO₂-NR₈R₉; oder (xix) NR₈-CO-NHR₉;
Ring B optional unabhängig bis zu dreimal substituiert ist in jeder Position durch R₅,
R₁, und R₆-R₁₁ jedes unabhängig Wasserstoff oder C₁₋₆-Alkyl sind,
R₂-R₅ jedes unabhängig (i) Wasserstoff, (ii) C₁₋₁₀-Alkyl oder C₂₋₁₀-Alkenyl jedes optional substituiert mit Amino, N-Niederalkylamino, N,N-Diniederalkylamino, N-Niederalkanoylamino, Hydroxy, Cyano, -COOR₁₀, -COR₁₄, -OCOR₁₄, -OR₁₀, C₅₋₁₀-Heteroaryl, C₅₋₁₀-Heteroaryloxy, oder
C₅₋₁₀-Heteroaryl-C₁₋₁₀-Alkoxy, Halogen bis zu Perhalogen; (iii) C₃-C₁₀-Zykloalkyl, in welchem 1-3 Kohlenstoffatome optional unabhängig ersetzt werden durch O, N oder S; (iv) C₃₋₁₀-Zykloalkenyl ; (v) teilweise ungesättigtes C₅₋₁₀₋Heterocyclyl ; (vi) Aryl; (vii) Heteroaryl;
wobei das (iii) Aryl oder (iv) Heteroaryl gewählt ist aus der Gruppe, die besteht aus:
2- oder 3-Furyl, 2- oder 3-Thienyl, 2- oder 4-Triazinyl, 1-, 2- oder 3-Pyrrolyl,1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4 Pyridyl, 2-, 4-, 5- oder 6-Pyrimidinyl, 1,2,3-Triazol-1-, -4- oder 5-yl, 1,2,4-Triazol-1-, -3- oder 5-yl, 1- oder 5-Tetrazolyl, 1,2,3-Oxadiazol-4- oder 5-yl, 1,2,4-Oxadiazol-3- oder 5-yl, 1,3,4-Thiadiazol-2- oder yl, 1,2,4-Oxadiazol-3- oder 5-yl, 1,3,4-Thiadiazol-2- oder 5-yl, 1,3,4-Thiadiazol-3- oder 5-yl, 1,2,3-Thiadiazol-4- oder 5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-6- oder 7-Benzisoxazolyl, 1-, 3-, 4-, 5 , 6- oder 7-Benzothiazolyl, 2-, 4-, 5-, 6- oder 7-Benzisothiazolyl, 2-, 4-, 5-, 6- oder 7-Benz-1,3-Oxadiazolyl, 2-, 3-, 4-, 5-, 6-, 7- oder 8-Quinolinyl, 1-, 3-, 4-, 5-, 6-, 7-, 8-Isoquinolinyl, 1-, 2-, 3-, 4- oder 9-Carbazolyl, 1-, 2-, 3-, 4-, 5-, 6-, 7-, 8- oder 9-Acridinyl, oder 2-, 4-, 5-, 6-, 7- oder 8-Quinazolinyl oder zusätzlich optinal substituiertes Phenyl, 2- oder 3-Thienyl, 1,3,4-Thiadiazolyl, 3-Pyrryl, 3-Pyrazolyl, 2-Thiazolyl oder 5-Thiazolyl; (viii) Halogen; (ix) -CO-OR₁₀; (x) -OCOR₁₀; (xi) -OCO₂R₁₀; (xii)-CHO; (xiii) Cyano; (xiv) -OR₁₆; (xv) -NR₁₀R₁₅; (xvi) Nitro; (xvii) -CO-NR₁₀R₁₁; (xviii) -NR₁₀-CO-R₁₂; (xix) -NR₁₀-CO-OR₁₁; (xx) -NR₁₀₋SO₂-R₁₂ ; (xxi) -SR₁₆; (xxii) -SOR₁₆; (xxiii) -SO₂-R₁₆; (xxiv) -SO₂-NR₁₀R₁₁; (xxv) NR₁₀-CO-NHR₁₁; (xxvi) Amidino; (xxvii) Guanidino; (xxviii) Sulfo; (xxix) -B(OH)₂; (xxx)
-OCON(R₁₀)₂; oder (xxxi) -NR₁₀CON(R₁₀)₂;
R₁₂ H, C₁₋₆-Alkyl oder C₅₋₁₀-Aryl ist,
R₁₃ H, C₁₋₆-Alkyl oder C₁₋₆-Alkoxy ist,
R₁₄ C₁₋₆-Alkyl oder Phenyl;
R₁₅ C₁₋₆-Alkyl, Halogen, Amino, N-Niederalkylamino, N,N-Diniederalkylamino, N-Niederalkanoylamino, OH, CN, COOR₁₀-, -COR₁₄ oder -OCOR₁₄ ist;
R₁₆ Wasserstoff, C₁₋₆-Alkyl, optional substituiert mit Halogen bis zu Perhalogen oder C₅₋₁₀-Heteroaryl ist; und
R₁₇ H, C₁₋₆-Alkyl oder CN ist,
oder ein pharmazeutisch verträgliches Salz davon,
mit der Bedingung, dass Formel I nicht ist:

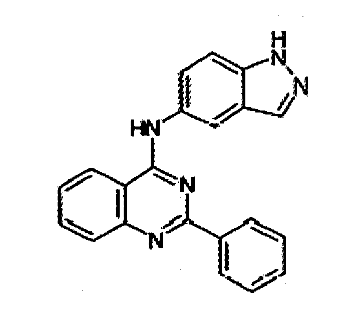

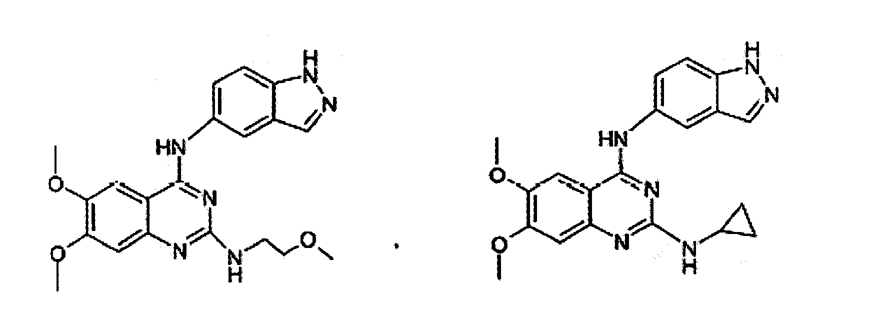

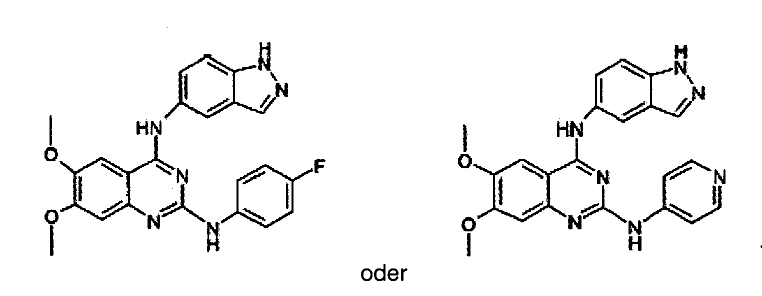

2. Verbindung nach Anspruch 1, worin A ein aromatischer 5-12-Kohlenstoffatomring oder Ringsystem ist, das 1-3 Ringe enthält, von denen wenigstens einer aromatisch ist, in welchen 1-4 Kohlenstoffatome in einem der mehreren der Ringe optional durch Sauerstoff-, Stickstoff- oder Schwefelatome ersetzt sind.

3. Verbindung nach Anspruch 2, worin jeder Ring in A 3-7 Atome hat.

4. Verbindung nach einem der Ansprüche 1 bis 3, worin A 2- oder 3-Furyl, 2- oder 3-Thienyl, 2- oder 4-Triazinyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4 Pyridyl, 2-, 4-, 5- oder 6-Pyrimidinyl, 1,2,3-Triazol-1-, -4- oder 5-yl, 1,2,4-Triazol-1-, -3- oder 5-yl, 1 oder 5-Tetrazolyl, 1,2,3-Oxadiazol-4- oder 5-yl, 1,2,4-Oxadiazol-3- oder 5-yl, 1,3,4-Thiadiazol-2- oder 5-yl, 1, 2, 4-Oxadiazol-3- oder 5-yl, 1,3,4-Thiadiazol-2- oder 5-yl, 1,3,4-Thiadiazol-3- oder 5-yl, 1,2,3-Thiadiazol-4- oder 5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-6- oder 7-Benzisoxazolyl, 1-, 3-, 4-, 5 , 6- oder 7-Benzothiazolyl, 2-, 4-, 5-, 6- oder 7-Benzisothiazolyl, 2-, 4-, 5-, 6- oder 7-Benz-1,3-Oxadiazolyl, 2-, 3-, 4-, 5-, 6-, 7- oder 8-Quinolinyl, 1-, 3-, 4-, 5-, 6-, 7-, 8-Isoquinolinyl, 1-, 2-, 3-, 4- oder 9-Carbazolyl, 1-, 2-, 3-, 4-, 5-, 6-, 7-, 8- oder 9-Acridinyl, oder 2-, 4-, 5-, 6-, 7- oder 8-Quinazolinyl, 2- oder 3-Thienyl, 1,3,4-Thiadiazolyl, 3-Pyrryl, 3-Pyrazolyl, 2-Thiazolyl oder 5-Thiazolyl ist.

5. Verbindung nach Anspruch 1, worin A Cyclohexyl oder C₅₋₁₂-Aryl oder C₅₋₁₂₋Heteroaryl ist, jedes unabhängig optional bis zu dreimal substituiert durch (i) C₁-C₁₀-Alkyl oder C₂₋₁₀-Alkenyl, jedes optional substituiert mit Halogen bis Perhalogen; (ii) C₃-C₁₀- Cycloalkyl; (iii) C₅₋₁₂-Aryl optional substituiert mit 1-3 Halogenatomen; (iv) C₅₋₁₂-Heteroaryl; (v) Halogen; (vi) -CO-OR₈; (vii) -CO-R₈; (viii) Cyano; (ix) NR₈R₁₃; (x) Nitro; (xi) -CO-NR₈R₉; (xii) -C₁₋₁₀-Alkyl-NR₈R₉; (xiii) -NR₈-CO-R₁₂; (xiv) -NR₈-CO-OR₉; (xv) -NR₈-SO₂-R₉ ; (xvi)-SR₈ ; (xvii) -SO₂-R₈; (xviii) -SO₂-NR₉R₉, oder (xix) NR₈-CO-NHR₉.

6. Verbindung nach Anspruch 1, worin A Phenyl, Pyridyl, Pyrimidinyl, Oxazolyl, Furyl, Thienyl, Pyrrolyl, Imidazolyl, Isoxazolyl und Pyrazinyl, jedes unabhängig bis zu dreimal substituiert durch Halogen, C₁₋₁₀-Alkyl, C₁₋₁₀-Alkoxyphenyl, Naphthyl, -OR₁₀, 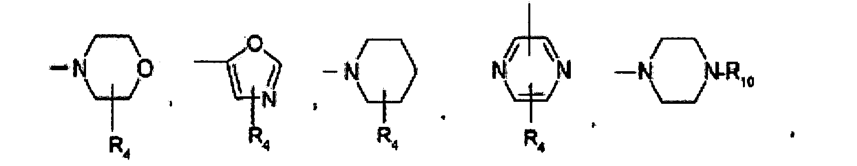 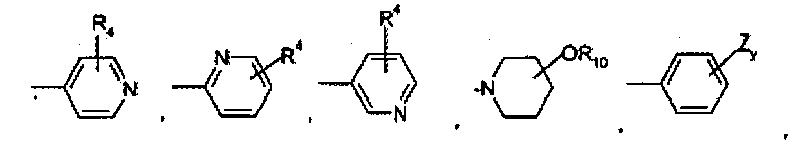 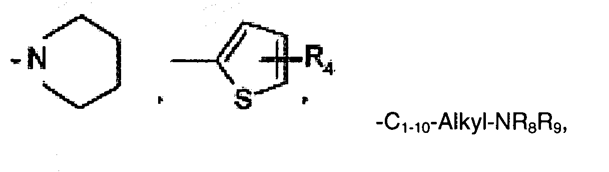 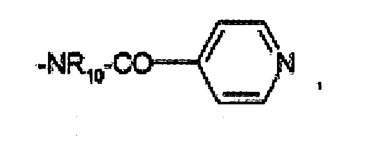 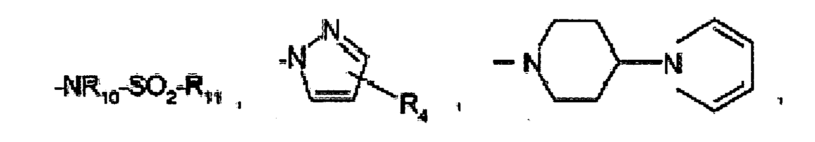 oder 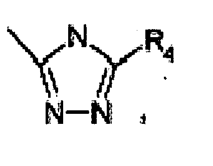 ist, worin jedes Z unabhängig Halogen, Hydroxy, Hydroxy-C₁₋₁₀-Alkyl, -CN, -NO₂, -C₁₋₁₀-Alkoxycarbonyl, -NR₁₀-CO-R₁₁, oder -NR₁₀-CO-OR₁₁, und y 1-3 ist.

7. Verbindung nach Anspruch 1 oder 3, worin A 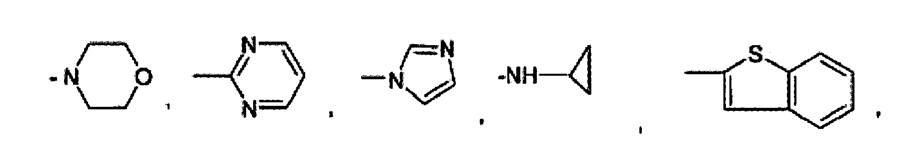 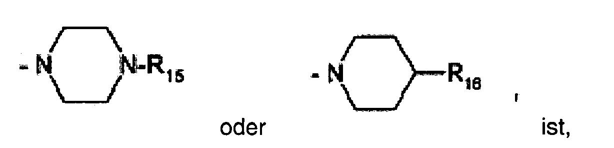 worin R₁₅ H; Phenyl optional substituiert mit C₁₋₁₀-Alkyl, C₁₋₁₀-Alkoxy, C₁₋₁₀₋Alkylcarboxyl, oder Halogen; Benzyl ; Pyrimidyl oder Pyridyl ist und R₁₆ H, Phenyl, -COOR₁₀,

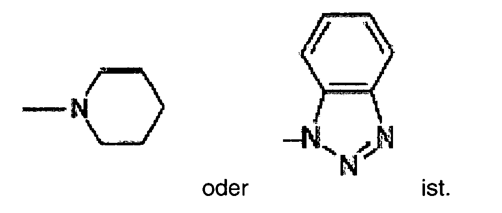

8. Verbindung nach Anspruch 1, worin R₅ in a, b oder c Wasserstoff oder C₁₋₁₀₋Alkyl oder C₂₋₁₀-Alkyl ist, optional substituiert mit Amino, N-Niederalkylamino, N,N-Diniederalkylamino, N-Niederalkanoylamino, Hydroxy, Cyano, -COOR₁₀, -COR₁₄, -OCOR₁₄, - OR₁₀, C₅₋₁₀-Heteroaryl, C₅₋₁₀-Heteroaryloxy, oder C₅₋₁₀₋Heteroaryl-C₁₋₁₀-Alkoxy, Halogen bis Perhalogen; (iii) C₃-C₁₀ Cycloalkyl, in welchem 1-3 Kohlenstoffatome optional unabhängig ersetzt sind durch O, N oder S; (iv) C₃₋₁₀-Zykloalkenyl; (v) teilweise ungesättigtes C₅₋₁₀-Heterocyclyl ; (vi) Aryl; (vii) Heteroaryl; (viii) Halogen;(ix) -CO-OR₁₀; (x) -OCOR₁₀ ; (xi) -OCO₂R₁₀; (xii) -CHO; (xiii) Cyano; (xiv) -OR₁₆; (xv) -NR₁₀R₁₅ ; (xvi) Nitro; (xvii) -CO-NR₁₀R₁₁; (xviii) -NR₁₀-CO-R₁₂; (xix) -NR₁₀-CO- OR₁₁; (xx) -NR₁₀-SO₂-R₁₂; (xxi)-SR₁₆; (xxii) -SOR₁₆; (xxiii)-SO₂-R₁₆; (xxiv) -SO₂-NR₁₀R₁₁; (xxv) NR₁₀-CO-NHR₁₁; (xxvi)-Amidino; (xxvii) Guanidino; (xxviii) Sulfo; (xxix) -B(OH)₂; (xxx) -OCON(R₁₀)₂; oder (xxxi) -NR₁₀CON (R₁₀)₂ ist.

9. Verbindung nach Anspruch 1, worin Y N ist und R₁ H ist.

10. Verbindung nach Anspruch 9, worin a -NR₆- ist, R₆ H ist, und c -N= ist.

11. Verbindung nach Anspruch 10, worin p 0 ist und R₁₋₄ H ist.

12. Verbindung nach Anspruch 11, worin X -(CH₂)ₓ- ist und x 0 ist.

13. Verbindung nach Anspruch 12, worin A Biphenyl, optional substituiert mit Halogen ist.

14. Verbindung nach Anspruch 1 mit der Formel: 2-(2,4-Dichlorphenyl)-N-(1H-Indazol-5-yl)-4-Quinazolinamin, 2-(4-Chlorphenyl)-N-(1H-Indazol-5-yl)-4-Quinazolinamin, 1-{-4[4-(1H-Indazol-5-ylamino)-2-Quinazolinyl]-Phenyl}-Ethanon, N-(1H-Indazol-5-yl)-2-[-(Trifluormethyl)-Phenyl]-4-Quinazolinamin, 2-(3-Chlor-4-Fluorphenyl)-N(1H-Indazol-5-yl)-4-Quinazolinamin, 2-(1,3-Benzodioxol-5yl)-N-(1H-Indazol-5-yl)-4-Quinazolinamin, N-(1H-Indazol-5-yl)-2-(4-Methylphenyl)-4-Quinazolinamin, 2-(3, 4-Dichlorphenyl)-N-(H-Indazol-5-yl)-4-Quinazolinamin,N-(1H-Indazol-5-yl)-2-(1-Naphthyl)-4-Quinazolinamin, N-(1 H-Indazol-5-yl)-2-(3,4,5-Trimethoxyphenyl)-4-Quinazolinamin, 2-(1-Benzofuran-2-yl)-N-(1H-Indazol-5yl)-4-Quinazolinamin, N-(1H-Indazol-5-yl)-2-(2-Thienyl)-4-Quinazolinamin, N-(1H-Indazol-5-yl)-2-(3-Thienyl)-4-Quinazolinamin, 2-(3,5-Dimethyl-4-Isoxazolyl)-N-(1H-Indazol-5-yl)-4-Quinazolinamin, 2-(1,1'-Biphenyl-4-yl)-N-(1H-Indazol-5-yl)-4-Quinazolinamin, 2-[4-(Dimethylamino)Phenyl]-N-(1H-Indazol-5-yl)-4-Quinazolinamin, 2-(1-Benzothien-2-yl)-N-(H-Indazol-5-yl)-4-Quinazolinamin, 4-[4-(1H-Indazol-5-ylamino)-2-Quinazolinyl]-Phenol, 2-Dibenzo-[b,d]Furan-1-yl-N-(1H-Indazol-5-yl)-4-Quinazolinamin, 2-(2-Fluor-1,1'-Biphenyl-4-yl)-N-(1H-Indazol-5-yl)-4-Quinazolinamin, 7-Chlor-N-(1H-Indazol- 5-yl)-2-Phenyl-4-Quinazolinamin, N-(1H-Indazol-5-yl)-6-Nitro-2-Phenyl-4-Quinazolinamin, 2-(4-Fluorphenyl)-N-(1H-Indazol-5-yl)-6-nitro-4-Quinazolinamin, 6-Chlor-N-(1 H-Indazol- 5-yl)-2-(4-Methylphenyl)-4-Quinazolinamin, 6-Chlor-2-(4-Fluorphenyl)-N-(1H-Indazol-5-yl)-4Quinazolinamin, 2 (4-Bromphenyl)-6-Chlor-N-(1H-Indazol-5-yl)-4-Quinazolinamin, N-(1H-Indazol-5-yl)-2-(2-Quinoxalinyl)-4-Quinazolinamin, 5-Fluor-N-(1H-Indazol-5-yl)-2-(2-Methylphenyl)-4-Quinazolinamin, 5-Fluor-2-(4-Fluorphenyl)-N-(1H-Indazol-5-yl)-4-Quinazolinamin, 2-(3-Chlorphenyl)-5-Fluor-N-(1H-Indazol-5-yl)-4-Quinazolinamin, 2-(4-Bromphenyl)-5-Fluor-N-(1H-Indazol-5-yl)-4-Quinazolinamin, 5-Fluor-N-(1H-Indazol-5-yl)-2-(3-Methylphenyl)-4-Quinazolinaminhydrochlorid, 2-(3-Bromphenyl)-5-Fluor-N (1H-Indazol-5-yl)-4-Quinazolinaminhydrochlorid, 2-(2-Chlorphenyl)-5-Fluor-N-(1H-Indazol-5-yl)-4-Quinazolinamin, 5-Fluor-N-(1H-Indazol-5-yl)-2-(2-Quinoxalinyl)-4-Quinazolinamin-Tris-(Trifluoracetat), 5-Fluor-N-(1H-Indazol-5-yl)-2-(1-Naphthyl)-Quinazolinamin-Bis-(Trifluoracetat), 5-Fluor-N-(1H-Indazol-5-yl)-2-(2-Naphthyl)-4-Quinazolinamin-Bis-(Trifluoracetat), 5-Fluor-N-(1H-Indazol-5-yl)-2-(4-Pyridinyl)-4-Quinazolinamin-Tris-(Trifluoracetat), N-(1H-Indazol-5-yl)-7-Methyl-2-(2-Quinoxalinyl)-4-Quinazolinamin, 2-(3-Chlorphenyl)-N-(1 H-Indazol-5-yl)-7-Methyl-4-Quinazolinamin, 2-(4-Fluorphenyl)-N-(1H-Indazol-5-yl)-7-Methyl-4-Quinazolinamin, N-(1H-Indazol-5-yl)-7-Methyl-2-(4-Methylphenyl)-4-Quinazolinamin", 2-(4-Bromphenyl)-N-(1H-Indazol-5-yl)-7-Methyl-4-Quinazolinamin, N-(1H-Indazol-5-yl)-7-Methyl-2-(2-Methylphenyl)-4-Quinazolinamin-Bis-(Trifluoracetat), N-(1H-Indazol-5-yl)-7-Methyl-2-(3-Methylphenyl)-4-Quinazolinamin-Bis-(Trifluoracetat),N-[2-(3-Fluorphenyl)-7-Methyl-4-Quinazolinyl]-N-(1H-Indazol-5-yl)-Amin-Bis-(Trifluoracetat), 2-(3-Bromphenyl)-N-(1H-Indazol-5-yl)-7-Methyl-4-Quinazolinamin-Bis-(Trifluoracetat), N-[2-(2-Chlorphenyl)-7-Methyl-4-Quinazolinyl]-N-(1 H-Indazol-5-yl)-Amin-Bis-(Trifluoracetat), 2-(3-Furyl)-N-(1H-Indazol-5-yl)-7-Methyl-4-Quinazolinamin-Bis-(Trifluoracetat), N-(1H-Indazol-5-yl)-7-Methyl-2-(1-Naphthyl)-4-Quinazolinamin-Bis-(Trifluoracetat), N-(1H-Indazol-5-yl)-7-Methyl-2-(2-Naphthyl)-4-Quinazolinamin-Bis-(Trifluoracetat), N-(1H-Indazol-5-yl)-7-Methyl-2-(3-Pyridinyl)-4-Quinazolinamin-Tris-(Trifluoracetat), N-(1H-Indazol-5-yl)-7-Methyl-2-(4-pyridinyl)-Quinazolinamin-Tris-(Trifluoracetat), 7-Chlor-2-(3-Chlorphenyl)-N-(1H-Indazol-5-yl)-4-Quinazolinamin, 7-Chlor-N-(1H-Indazol-5-yl)-2-(4-Methylphenyl)-4-Quinazolinamin, 2-(4-Bromphenyl)-7-Chlor-N-(1H-Indazol-5-yl)-4-Quinazolinamin, 7-Chlor-N-(1H-Indazol-5-yl)-2-(3-Methylphenyl)-4-Quinazolinaminhydrochlorid, 7-Chlor-2-(3-Fluorphenyl)-N-(1H-Indazol-5-yl)-4-Quinazolinamin-Bis-(Trifluoracetat), 2-(3-Bromophenyl)-7-Chlor-N-(1 H-Indazol-5-yl)-4-Quinazolinamin-Bis-(Trifluoracetat), N-[7-Chlor-2- (2-furyl)-4-Quinazolinyl]-N (1H-Indazol-5-yl)-Amin-Bis-(Trifluoracetat), 7-Chlor-N-(1 H-Indazol-5-yl)-2-(2-Quinoxalinyl)-4-Quinazolinamin-Tris-(Trifluoracetat), 7-Chlor-N-(1H-Indazol-5-yl)-2-(1-Naphthyl)-4-Quinazolinamin-Bis-(Trifluoracetat), 7-Chlor-N-(1H-Indazol-5-yl)-2-(2-Naphthyl)-4-Quinazolinamin-Bis-(Trifluoracetat), 7-Chlor-N-(1H-Indazol-5-yl)-2-(3-Pyridinyl)-4-Quinazolinamin-Tris-(Trifluoracetat), 2-(4-Fluorphenyl)-N-(1H-Indazol-5-yl)-6,7-Dimethoxy-4-Quinazolinamin, 2-(1,1'-Biphenyl-4-yl)-N-(1H-Indazol- 5-yl)-6,7-Dimethoxy-4-Quinazolinamin, N-(1H-Indazol-5-yl)-6,7-Dimethoxy-2-(4-Vinylphenyl)4-Quinazolinamin, N-Zyklopentyl-4-(1H-Indazol-5-ylamino)-2-Quinazolincarboxamid, N-(3-Fluorphenyl)-N-[4-(1H-Indazol-5-ylamino)-6,7-Dimethoxy-2-Quinazolinyl]-Amin, N-(2,4-Difluorbenzyl)-N-[4-(1H-Indazol-5-ylamino)-6,7-Dimethoxy-2-Quinazolinyl]-Amin, N-(2-Fluorbenzyl)-N-[4-(1 H-Indazol-5-ylamino)-6,7-Dimethoxy-2-Quinazolinyl]-Amin, N-(4-Bromophenyl)-N-[4-(1H-Indazol-5-ylamino)-6,7-Dimethoxy-2-Quinazolinyl]-Amin, N-(6,7-Dimethoxy-2-{[4-(Trifluormethyl)Phenyl]-Amino}-4-Quinazolinyl)-N-(1H-Indazol-5-yl)-Amin,
N-(6,7-Dimethoxy-2-{[4-(Trifluormethyl)Benzyl]-Amino}-4-Quinazolinyl)-N-(1H-Indazol-5-yl)-Amin, N-[3-Fluor-5-(Trifluormethyl)-Benzyl]-N-[4-(1H-Indazol-5-ylamino)-6,7-Dimethoxy-2-Quinazolinyl]-Amin, N-(3-Fluorbenzyl)-N-[4-(1 H-Indazol-5-ylamino)-6,7-Dimethoxy-2-Quinazolinyl]-Amin, N-(2,4-Difluorbenzyl)-N-[4-(1H indazol-5-ylamino)-6,7-Dimethoxy-2-Quinazolinyl]-Amin, N-(4-Fluorbrenzyl)-N-[4-(1H-Indazol-5-ylamino)-6, Dimethoxy-2-Quinazolinyl]-Amin,
N-(2,6-Difluorbenzyl)-N-[4-(1H-Indazol-5-ylamino)-6,7-Dimethoxy-2-Quinazolinyl]-Amin, N-(3, 5-Difluorbenzyl)-N [4-(1H-Indazol-5-ylamino)-6,7-Dimethoxy-2-Quinazolinyl]-Amin, N-(3-Bromphenyl)-N-[4-(1H-Indazol-5-ylamino)-6,7-Dimethoxy-2-Quinazolinyl]-Amin, N-(2,6-Difluorphenyl)-N-[4-(1H-Indazol-5-ylamino)-6,7-Dimethoxy-2-Quinazolinyl]-Amin, N-(2, 5-Difluorphenyl)-N-[4-(1H-Indazol-5-ylamino)-6,7-Dimethoxy-2-Quinazolinyl]-Amin, N-(2, 4-Difluorphenyl)-N-[4-(1H-Indazol-5-ylamino)-6,7-Dimethoxy-2-Quinazolinyl]-Amin, N (2,3-Difluorphenyl)-N-[4-(1H-Indazol-5-ylamino)-6,7-Dimethoxy-2-Quinazolinyl]-Amin, N-(3,4-Difluorphenyl)-N-[4-(1H-Indazol-5-ylamino)-6,7-Dimethoxy-2-Quinazolinyl]-Amin, N-(3,5-Difluorphenyl)-N-4-(1H-Indazol-5-ylamino)-6,7-Dimethoxy-2-Quinazolinyl]-Amin, N-{6,7-Dimethyoxy-2-[(2,3,4-Trifluorphenyl)-Amino]-4Quinazolinyl}-N-(1H-Indarazol-5-ylamin, N-{6,7-Dimethoxy-2-[(2,4,5-Trifluorphenyl)-Amino]-4-Quinazolinyl}-N-(1H-Indazol-5-yl)-Amin, N-{6,7-Dimethoxy-2-[(2, 4,6-Trifluorphenyl)-Amino]-4-Quinazolinyl}-N-(1H-Indazol-5-yl)-Amin, N-{6,7-Dimethoxy-2-[(2,3,6-Trifluorphenyl)-Amino]-4-Quinazolinyl}-N-(1H-Indazol-5-yl)-Amin, N-(4-Bromphenyl)-N-[4-(1H-Indazol-5-ylamino)-6,7-Dimethoxy-2-Quinazolinyl]-Amin, 2-(3-Aminophenyl)-N-(1H-Indazol-5-yl)-4-Quinazolinamin, N-{3-(4-(1H-Indazol-5-ylamino)-2-Quinazolinyl]-Phenyl}Isonicotatiamid, N-{3-[4-(1H-Indazol-5-ylamino)-2-Quinazolinyl]Phenyl}-Acetamid, N-(4-Chlorphenyl)-N-[4-(1H-Indazol-5-ylamino)-2-Quinazolinyl]-Amin,
N-(3-Bromphenyl)-N [4-(1H-Indazol-5-ylamino)-2-Quinazolinyl]-Amin, N-(2-Chlorphenyl)-N-[4-(1H-Indazol-5-ylamino)-2-Quinazolinyl]-Amin, N-(3-Fluorphenyl)-N-[4-(1H-Indazol-5-ylamino)-2-Quinazolinyl]-Amin, N-(2-Fluorphenyl)-N [4-(H-Indazol-5-ylamino)-2-Quinazolinyl]-Amin, N-(3-Chlorphenyl)-N- [4-(1H-Indazol-5-ylamino)-2-Quinazolinyl]-Amin, N-(4-Bromphenyl)-N-[4-(1H-Indazol-5-ylamino)-2-Quinazolinyl]-Amin,N-(1H-Indazol-5- yl)-N-(2-{[3-(Trifluormethyl)Phenyl]Amino}-4-Quinazolinyl)-Amin, N-(1H-Indazol-5-yl)-{2-[(4-Phenoxyphenyl)-Amino]-4-Quinazolinyl} Amin, N-(1H-Indazol-5-yl)-N-(2-{[4-(Trifluormethoxy)Phenyl]-Amino}-4-Quinazolinyl)-Amin, N-(1H-Indazol-5-yl)-N-(2-{[3-(Trifluormethoxy)phenyl]-Amino}-4-Quinazolinyl)-Amin, N-(4-Fluorphenyl)-N-[4-(1H-Indazol-5-ylamino)-2-Quinazolinyl]-Amin, N-(2-Anilino-4-Quinazolinyl)-N-(H-Indazol-5 yl)-Amin, 2-[4-(2-Chlorphenyl)-1-Piperazinyl]-N-(1H-Indazol-5-yl)-4-Quinazolinamin, (1H-Indazol-5-yl)-2-[4-(2-Pyrimidinyl)-1-Piperazinyl]-4-Quinazolinamin, N-(1H-Indazol-5-yl)-2- [4-(2-Methoxyphenyl)-1-Piperazinyl]-4-Quinazolinamin, 1-(4-{4-[4-(1H-Indazolylamino)-2-Quinazolinyl]-1-Piperazinyl}-Phenyl)-Ethanon, 4-(1H-Indazol-5-ylamino)-2-Quinazolincarboxamid", 4-(1H-Indazol-5-ylamino)-N-(4-Pyridinyl)-2-Quinazolincarboxamid, 4-(1H-Indazol-5-ylamino)-N-(4-Methoxyphenyl)-2-Quinazolincarboxamid, N-Zyklohexyl-4-(1H-Indazol-5-ylamino)-2-Quinazolincarboxamid,N-Zyklopentyl-4-(1H-Indazol-5-ylamino)-2-Quinazolincarboxamid, 4-(1H-Indazol-5-ylamino)-N-(2-Pyridinyl)-2-Quinazolincarboxamid,4-(1H-Indazol-5-ylamino)-N-(3-Quinolinyl)-2-Quinazolincarboxamid,4-(1H-Indazol-5-ylamino)-N-Methyl-2-Quinazolincarboxamid, N-(1H-Indazol-5-yl)-2-(4-Morpholinylcarbonyl)-4-Quinazolinamin, 2-(2,3-Dihydro-1-Benzofuran-5-yl)-N-(1H-Indazol-5-yl)-4-Quinazolinamin, 2-Zyklopropyl-N-(1H-Indazol-5-yl)-4-Quinazolinamin, 2-(2-Fluor-1,1'-Biphenyl-4-yl)-N-(1H-Indazol-5-yl)-4-Quinazolinamindihydrochlorid, 2-(2-Fluor-1,1'-Biphenyl-4-yl)-N-(1H-Indazol-5-yl)-4Quinazolinamindimethanesulfonat, 2-(2-Fluor-1,1'-Biphenyl-4-yl)-N-(1H-Indazol-5-yl)-4- Quinazolinaminbenzenesulfonat, 2-(2-Fluor-1, 1'-Biphenyl-4-yl)-N-(1H-Indazol-5-yl)-4-Quinazolinamin-4-Methylbenzenesulfonat oder 2-Dibenzo[b,d]furan-1-yl-N-(1H-Indazol-5yl)-4-Quinazolinamintrifluoracetat, 2-(3-Fluor-1,1-Biphenyl-4-yl)-N-(1H-Indazol-5-yl)-4-Quinazolinamin; 2-(2-(Furyl)-N-(1H-Indazol-5-yl)-7-Methyl-4-Quinazolinamin; 2-(1-Benzofuranyl)-N-(1H-Indazol-5-yl)-6,7-Dimethoxy-4-Quinazolinamin; N-(2,5-Difluorbenzyl)-N-[4-(1H-Indazol-5-ylamino)-6,7-Dimethoxy-2-Quinazolinyl]-Amin; N-{6,7-Dimethoxy-2-[(2,3,5-Trifluorphenyl)-Amino]-4-Quinazolinyl}-N-(1H-Indazol-5-yl)-Amin; N-(1 H-Indazol-5-yl)-2-[5-(1H-Indolylamino]-4-Quinazolinamin; N-(1H-Indazol-5-yl)-2-[4-Phenoxyanilino]-4-Quinazolinamin; N-(1H-Indazol-5-yl)-2-[2-Naphtylamino]-4-Quinazolinamin.

15. Verbindung der Formel:
N-(1H-Indazol-5-yl)-2-(Trifluormethyl)-4-Quinazolinamin; 2-Chlor-N-(3-Ethyl-1H-Indazol-5-yl)-4-Quinazolinamin; 2-Chlor-N-(1H-Indazol-5-yl)-4-Quinazolinamin.

16. Verfahren zur Herstellung einer Verbindung nach Anspruch 1, umfassend (a) Reagieren einer Verbindung mit Formel II 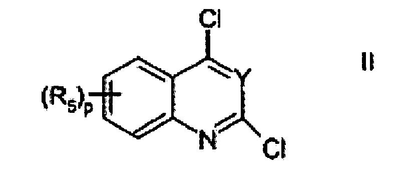 mit einer Verbindung der Formel III 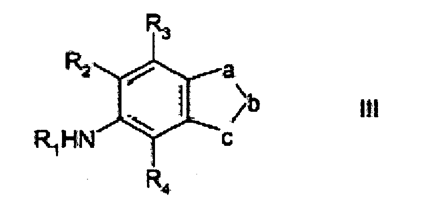 in Gegenwart einer Base, um eine Verbindung der Formel IV 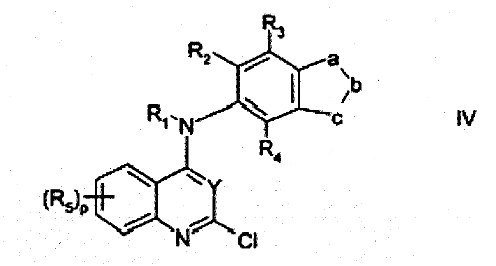 herzustellen
und optional weiter Reagieren von IV mit Arylborsäure oder A-NH₂, oder
(b) Reagieren eines substituierten Benzoylchlorids mit Dimethylamin, um eine Verbindung der Formel V herzustellen 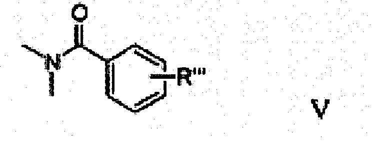 worin R"' (i)C₁-C₁₀-Alkyl oder C -C₁₀-Alkenyl, jedes optional substituiert mit Halogen bis zu Perhalogen; (ii) C₃-C₁₀-Zykloalkyl; (iii) Aryl; (iv) Heteroaryl; (v) Halogen; (vi) -CO-OR₈; (vii) CO-R₈; (viii) Cyano; (ix) -OR₈, (x) (x) -NR₈R₁₃; (xi) Nitro; (xii) -CO-NR₈R₉; (xiii) -C₁₋₁₀-Alkyl-NR₉R₉; (xiv) -NR₈-CO-R₁₂; (xv) -NR₈₋CO-OR₉; (xvi) -NR₈SO₂-R₉; (xvii) -SR₈ (xviii) -SO₂-R₈;(xix)-SO₂-NR₈R₉; oder (xx) NR₈-CO-NHR₉, Reagieren von V mit Chlor-2-Aminobenzonitril, um eine Verbindung der Formel VI herzustellen 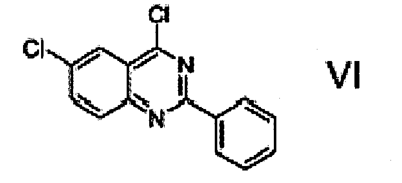 und Reagieren von VI mit Aminoindazol.

17. Verfahren zum herstellen von 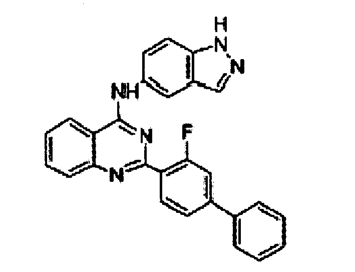 umfassend Reagieren von 3-Fluor-4-Phenylbenzoesäure 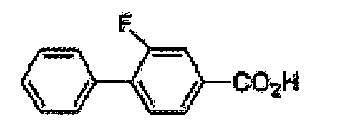 mit 4-Brom-2-Fluorbiphenyl, um 2-[(3-Fluor-4-Phenylphenyl)Carbonylamino]Benzamid herzustellen 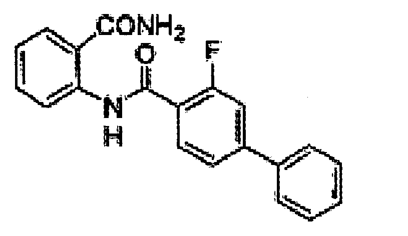 Ringschluss, um 2-(3-Fluor-1,1'-Biphenyl-4-yl)-4(3H)-Quinazolinon herzustellen 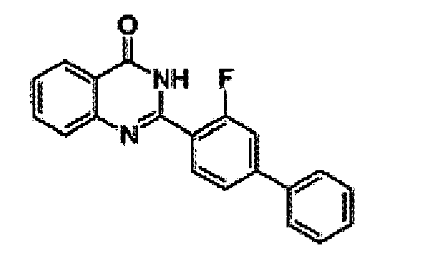 Reagieren, um 4-Chlor-2-(3-Fluor-4-Penylphenyl)Quinazolin herzustellen. 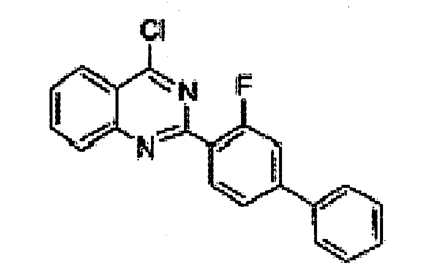 und dann Reagieren mit Aminoindazol, um 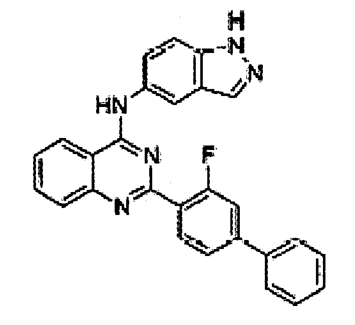 herzustellen.

18. Pharmazeutische Zusammensetzung, **dadurch gekennzeichnet, dass** sie eine oder mehrere Verbindungen nach einem der Ansprüche 1 bis 17 umfasst, in Verbindung mit einem oder mehreren pharmazeutisch verträglichen Trägern und optional andere Wirkstoffe.

19. Verwendung einer Verbindung nach einem der Ansprüche 1 bis 18 zur Herstellung eines Medikaments zur Behandlung einer Indikation, die mit Rho-Kinase medikamentös behandelt wird.

20. Verwendung einer Verbindung nach Anspruch 13 zur Herstellung eines Medikaments zur Behandlung einer Indikation, die mit Rho-Kinase medikamentös behandelt wird.

21. Verwendung einer Verbindung nach Anspruch 14 zur Herstellung eines Medikaments zur Behandlung einer Indikation, die mit Rho-Kinase medikamentös behandelt wird.

22. Verwendung einer Verbindung nach Anspruch 15 zur Herstellung eines Medikaments zur Behandlung einer Indikation, die mit Rho-Kinase medikamentös behandelt wird.

23. Verwendung einer Verbindung nach einem der Ansprüche 1 bis 18 zur Herstellung eines Medikaments zur Behandlung von Bluthochdruck, Atherosclerose, Restenosis, cerebraler Ischämie, cerebralem Vasospasmas, neuronaler Degeneration, Rückenmarkverletzung, Brust-, Darm-, Prostata-, Eierstock-, Hirn- oder Lungenkrebs, Thrombotischen Störungen, Asthma, Glaukom, Osteoporose oder Erektionsstörung.

24. Verwendung einer Verbindung nach Anspruch 13 zur Herstellung eines Medikaments zur Behandlung von Bluthochdruck, Atherosclerose, Restenosis, cerebraler Ischämie, cerebralem Vasospasmas, neuronaler Degeneration, Rückenmarkverletzung, Brust-, Darm-, Prostata-, Eierstock-, Hirn- oder Lungenkrebs, Thrombotischen Störungen, Asthma, Glaukom, Osteoporose oder Erektionsstörung.

25. Verwendung einer Verbindung nach Anspruch 14 zur Herstellung eines Medikaments zur Behandlung von Bluthochdruck, Atherosclerose, Restenosis, cerebraler Ischämie, cerebralem Vasospasmas, neuronaler Degeneration, Rückenmarkverletzung, Brust-, Darm-, Prostata-, Eierstock-, Hirn- oder Lungenkrebs, Thrombotischen Störungen, Asthma, Glaukom, Osteoporose oder Erektionsstörung.

26. Verwendung einer Verbindung nach Anspruch 15 zur Herstellung eines Medikaments zur Behandlung von Bluthochdruck, Atherosclerose, Restenosis, cerebraler Ischämie, cerebralem Vasospasmas, neuronaler Degeneration, Rückenmarkverletzung, Brust-, Darm-, Prostata-, Eierstock-, Hirn- oder Lungenkrebs, Thrombotischen Störungen, Asthma, Glaukom, Osteoporose oder Erektionsstörung.

27. Verwendung nach einem der Ansprüche 18 bis 26, worin der Wirt ein Mensch ist.

## Revendications

1. Composé de formule 1 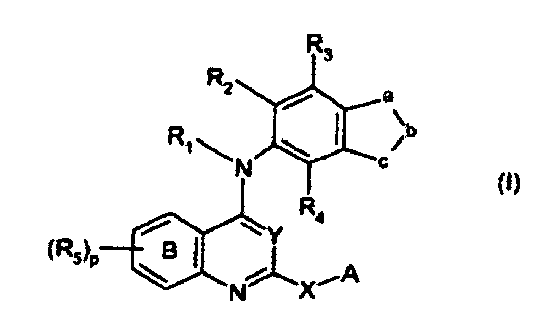 dans laquelle Y est =N- ou =CR₁₇,
X est -(CH₂)ₓ-, -O-(CH₂)ₙ-, -S-(CH₂)ₙ-, -NR₇-CO-(CH₂)ₙ-,
-NR₇-SO₂-(CH₂)ₙ-, -NR₇-(CH₂)ₙ- ou -(O)C-NR₇-,
chaque n est un entier qui vaut indépendamment 0, 1, 2 ou 3,
x vaut 0 à 3
p vaut 0 à 3
a et c sont chacun indépendamment -CR5=, -N=, ou -NR6-, dans lesquels l'un de a ou c est -NR6-, et b est -CR5= ou -N= ;
A est -CO-OR₈, -CO-R₈, un groupe cyano, -NR₈R₉, -CO-NR₈R₉, -NR₈-CO-R₉, -NR₈-CO-OR₉, -NR₈-SO₂-R₉, -SR₈, -SO₂-R₈, -SO₂-NR₈R₉, NR₈-CO-NHR₉,
ou
A est une fraction cyclique ou polycyclique à 3 à 20 atomes, contenant 1 à 4 cycles, qui contient facultativement 1 à 3 atomes de N, O ou S par cycle, et peut facultativement être un groupe aryle ou hétéroaryle, laquelle fraction cyclique ou polycyclique peut facultativement être substituée jusqu'à 3 fois par (i) un groupe alkyle en C_{1 à} C₁₀ ou alcényle en C₂ à C₁₀, chacun facultativement substitué par un atome d'halogène jusqu'à un perhalogéno ; (ii) un groupe cycloalkyle en C₃ à C₁₀ ; (iii) un groupe aryle ; (iv) un groupe hétéroaryle ; ledit (iii) groupe aryle ou ledit (iv) groupe hétéroaryle étant choisi dans le groupe constitué par : le 2- ou 3-furyle, le 2- ou 3-thiényle, le 2- ou 4-triazinyle, le 1-, 2- ou 3-pyrrolyle, le 1-, 2-, 4- ou 5-imidazolyle, le 1-, 3-, 4- ou 5-pyrazolyle, le 2-, 4- ou 5-oxazolyle, le 3-, 4- ou 5-isoxazolyle, le 2-, 4- ou 5-thiazolyle, le 3-, 4- ou 5-isothiazolyle, le 2-, 3- ou 4-pyridyle, le 2-, 4-, 5- ou 6-pyrimidinyle, le 1,2,3-triazol-1-, -4- ou 5-yle, le 1,2,4-triazol-1-, -3- ou 5-yle, le 1- ou 5-tétrazolyle, le 1,2,3-oxadiazol-4- ou 5-yle, le 1,2,4-oxadiazol-3- ou 5-yle, le 1,3,4-thiadiazol-2- ou 5-yle, le 1,2,4-oxadiazol-3- ou 5-yle, le 1,3,4-thiadiazol-2- ou 5-yle, le 1,3,4-thiadiazol-3- ou 5-yle, le 1,2,3-thiadiazol-4- ou 5-yle, le 2-, 3-, 4-, 5- ou 6-2H-thiopyranyle, le 2-, 3- ou 4-4H-thiopyranyle, le 3- ou 4-pyridazinyle, le pyrazinyle, le 2-, 3-, 4-, 5-, 6- ou 7-benzofuryle, le 2-, 3-, 4-, 5-, 6- ou 7-benzothiényle, le 1-, 2-, 3-, 4-, 5-, 6- ou 7-indolyle, le 1-, 2-, 4- ou 5-benzimidazolyle, le 1-, 3-, 4-, 5-, 6- ou 7-benzopyrazolyle, le 2-, 4-, 5-, 6- ou 7-benzoxazolyle, le 3-, 4-, 5-, 6- ou 7-benzisoxazolyle, le 1-, 3-, 4-, 5-, 6- ou 7-benzothiazolyle, le 2-, 4-, 5-, 6- ou 7-benzisothiazolyle, le 2-, 4-, 5-, 6- ou 7-benz-1,3-oxadiazolyle, le 2-, 3-, 4-, 5-, 6-, 7- ou 8-quinoléinyle, le 1-, 3-, 4-, 5-, 6-, 7-, 8-isoquinoléinyle, le 1-, 2-, 3-, 4- ou 9-carbazolyle, le 1-, 2-, 3-, 4-, 5-, 6-, 7-, 8- ou 9-acridinyle, ou le 2-, 4-, 5-, 6-, 7- ou 8-quinazolinyle, ou de plus un groupe phényle 2- ou 3-thiényle, 1,3,4-thiadiazolyle, 3-pyrryle, 3-pyrazolyle, 2-thiazolyle ou 5-thiazolyle facultativement substitué ; (v) un atome d'halogène ; (vi) -CO-OR₈ ; (vii) -CO-R₈ ; (viii) un groupe cyano ; (ix) -NR₈R₁₃ ; (x) un groupe nitro ; (xi) -CO-NR₈R₉ ; (xii) C-(alkyl en C_{1 à 10})-NR₈R₉ ; (xiii) -NR₈-CO-R₁₂ ; (xiv) -NR₈-CO-OR₉ ; (xv) -NR₈-SO₂-R₉ ; (xvi) -SR₈ ; (xvii) -SO₂-R₈ ; (xviii) -SO₂-NR₈R₉ ; ou (xix) NR₈-CO-NHR₉ ;
le cycle B est facultativement indépendamment substitué jusqu'à 3 fois en toute position par R₅,
R₁ et R₆ à R₁₁ sont chacun indépendamment un atome d'hydrogène ou un groupe alkyle en C_{1 à 6},
R₂ à R₅ sont chacun indépendamment (i) un atome d'hydrogène, (ii) un groupe alkyle en C_{1 à 10} ou alcényle en C_{2 à 10} chacun facultativement substitué par un groupe amino, N-alkyl inférieur amino, N,N-dialkyl inférieur amino, N-alcanoyl inférieur amino, hydroxy, cyano, -COOR₁₀, -COR₁₄, -OCOR₁₄, -OR₁₀, hétéroaryle en C₅ à ₁₀, hétéroaryloxy en C_{5 à 10} ou hétéroaryle en C_{5 à 10}-alcoxy en C_{1 à 10}, un atome d'halogène jusqu'à un perhalogéno ; (iii) un groupe cycloalkyle en C₃ à C₁₀, dans lequel 1 à 3 atomes de carbone sont facultativement indépendamment remplacés par O, N ou S ; (iv) un groupe cycloalcényle en C_{3 à 10} ; (v) un groupe hétérocyclyle en C_{5 à 10} partiellement insaturé ; (vi) un groupe aryle ; (vii) un groupe hétéroaryle ; ledit (iii) groupe aryle ou ledit (iv) groupe hétéaryle étant choisi dans le groupe constitué par : le 2- ou 3-furyle, le 2- ou 3-thiényle, le 2- ou 4-triazinyle, le 1-, 2- ou 3-pyrrolyle, le 1-, 2-, 4- ou 5-imidazolyle, le 1-, 3-, 4- ou 5-pyrazolyle, le 2-, 4- ou 5-oxazolyle, le 3-, 4- ou 5-isoxazolyle, le 2-, 4- ou 5-thiazolyle, le 3-, 4- ou 5-isothiazolyle, le 2-, 3- ou 4-pyridyle, le 2-, 4-, 5- ou 6-pyrimidinyle, le 1,2,3-triazol-1-, -4- ou 5-yle, le 1,2,4-triazol-1-, -3- ou 5-yle, le 1- ou 5-tétrazolyle, le 1,2,3-oxadiazol-4- ou 5-yle, le 1,2,4-oxadiazol-3- ou 5-yle, le 1,3,4-thiadiazol-2- ou 5-yle, le 1,2,4-oxadiazol-3- ou 5-yle, le 1,3,4-thiadiazol-2- ou 5-yle, le 1,3,4-thiadiazol-3- ou 5-yle, le 1,2,3-thiadiazol-4- ou 5-yle, le 2-, 3-, 4-, 5- ou 6-2H-thiopyranyle, le 2-, 3- ou 4-4H-thiopyranyle, le 3- ou 4-pyridazinyle, le pyrazinyle, le 2-, 3-, 4-, 5-, 6- ou 7-benzofuryle, le 2-, 3-, 4-, 5-, 6- ou 7-benzothiényle, le 1-, 2-, 3-, 4-, 5-, 6- ou 7-indolyle, le 1-, 2-, 4- ou 5-benzimidazolyle, le 1-, 3-, 4-, 5-, 6- ou 7-benzopyrazolyle, le 2-, 4-, 5-, 6- ou 7-benzoxazolyle, le 3-, 4-, 5-, 6- ou 7-benzisoxazolyle, le 1-, 3-, 4-, 5-, 6- ou 7-benzothiazolyle, le 2-, 4-, 5-, 6- ou 7-benzisothiazolyle, le 2-, 4-, 5-, 6- ou 7-benz-1,3-oxadiazolyle, le 2-, 3-, 4-,5-, 6-, 7- ou 8-quinoléinyle, le 1-, 3-, 4-, 5-, 6-, 7-, 8-isoquinoléinyle, le 1-, 2-, 3-, 4- ou 9-carbazolyle, le 1-, 2-, 3-, 4-, 5-, 6-, 7-, 8- ou 9-acridinyle, ou le 2-, 4-, 5-, 6-, 7- ou 8-quinazolinyle, ou de plus un groupe phényle, 2- ou 3-thiényle, 1,3,4-thiadiazolyle, 3-pyrryle, 3-pyrazolyle, 2-thiazolyle ou 5-thiazolyle facultativement substitué ; (viii) un atome d'halogène ; (ix) -CO-OR₁₀ ; (x) -OCOR₁₀ ; (xi) -OCO₂R₁₀ ; (xii) -CHO ; (xiii) un groupe cyano ; (xiv) -OR₁₆ ; (xv) -NR₁₀R₁₅ ; (xvi) un groupe nitro ; (xvii) -CO-NR₁₀R₁₁ ; (xviii) -NR₁₀-CO-R₁₂ ; (xix) -NR₁₀-CO-OR₁₁ ; (xx) -NR₁₀-SO₂-R₁₂ ; (xxi) -SR₁₆ ; (xxii) -SOR₁₆ ; (xxiii) -SO₂-R₁₆ ; (xxiv) -SO₂-NR₁₀R₁₁ ; (xxv) NR₁₀-CO-NHR₁₁ ; (xxvi) un amidino ; (xxvii) un groupe guanidino ; (xxviii) un groupe sulfo ; (xxix) -B(OH)₂ ; (xxx) -OCON(R₁₀)₂ ; ou (xxxi) -NR₁₀CON(R₁₀)₂ ;
R₁₂ est H, un groupe alkyle en C_{1 à 6} ou aryle en C_{5 à 10},
R₁₃ est H, un groupe alkyle en C_{1 à 6} ou alcoxy en C_{1 à 6},
R₁₄ est un groupe alkyle en C_{1 à 6} ou phényle ;
R₁₅ est un groupe alkyle en C_{1 à 6}, un atome d'halogène, un groupe amino, N-alkyl inférieur amino, N,N-dialkyl inférieur amino, N-alcanoyl inférieur amino, OH, CN, COOR₁₀, -COR₁₄ ou -OCOR₁₄ ;
R₁₆ est un atome d'hydrogène, un groupe alkyle en C_{1 à 6} facultativement substitué par un atome d'halogène, jusqu'au perhalogéno, ou un groupe hétéroaryle en C_{5 à 10} ; et
R₁₇ est H, un groupe alkyle en C_{1 à 6} ou CN,
ou un sel pharmaceutiquement acceptable de celui-ci,
à condition que la formule I ne soit pas

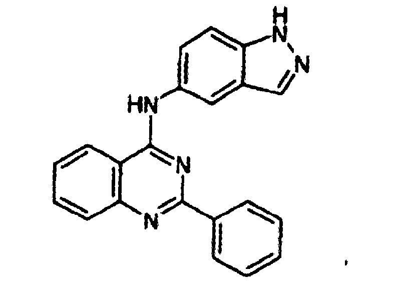

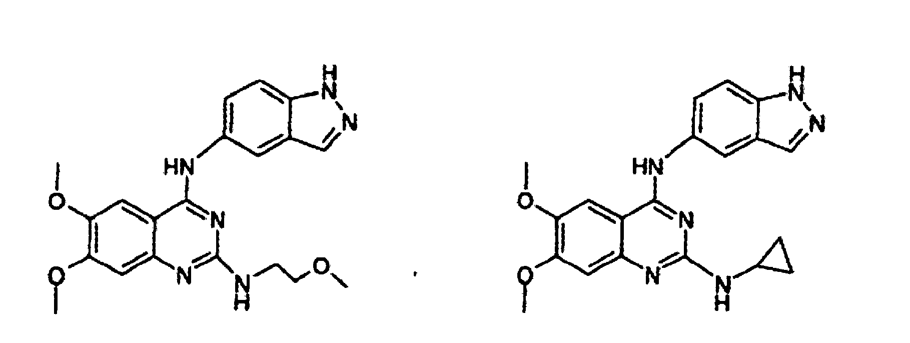

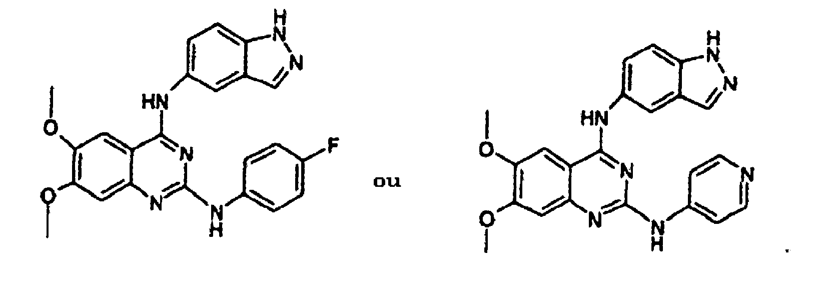

2. Composé selon la revendication 1, dans lequel A est un cycle ou système de cycle aromatique à 5 à 12 atomes de carbone contenant 1 à 3 cycles, dont au moins l'un est aromatique, dans lequel 1 à 4 atomes de carbone dans un ou plusieurs des cycles sont facultativement remplacés par des atomes d'oxygène, d'azote ou de soufre.

3. Composé selon la revendication 1 ou 2, dans lequel chaque cycle dans A comporte 3 à 7 atomes.

4. Composé selon l'une quelconque des revendications 1 à 3, dans lequel A est le 2- ou 3-furyle, le 2- ou 3-thiényle, le 2- ou 4-triazinyle, le 1-, 2- ou 3-pyrrolyle, le 1-, 2-, 4- ou 5-imidazolyle, le 1-, 3-, 4- ou 5-pyrazolyle, le 2-, 4- ou 5-oxazolyle, le 3-, 4- ou 5-isoxazolyle, le 2-, 4- ou 5-thiazolyle, le 3-, 4- ou 5-isothiazolyle, le 2-, 3- ou 4-pyridyle, le 2-, 4-, 5- ou 6-pyrimidinyle, le 1,2,3-triazol-1-, -4- ou 5-yle, le 1,2,4-triazol-1-, -3- ou 5-yle, le 1- ou 5-tétrazolyle, le 1,2,3-oxadiazol-4- ou 5-yle, le 1,2,4-oxadiazol-3- ou 5-yle, le 1,3,4-thiadiazol-2- ou 5-yle, le 1,2,4-oxadiazol-3- ou 5-yle, le 1,3,4-thiadiazol-2- ou 5-yle, le 1,3,4-thiadiazol-3- ou 5-yle, le 1,2,3-thiadiazol-4- ou 5-yle, le 2-, 3-, 4-, 5- ou 6-2H-thiopyranyle, le 2-, 3- ou 4-4H-thiopyranyle, le 3- ou 4-pyridazinyle, le pyrazinyle, le 2-, 3-, 4-, 5-, 6- ou 7-benzofuryle, le 2-, 3-, 4-, 5-, 6- ou 7-benzothiényle, le 1-, 2-, 3-, 4-, 5-, 6- ou 7-indolyle, le 1-, 2-, 4- ou 5-benzimidazolyle, le 1-, 3-, 4-, 5-, 6- ou 7-benzopyrazolyle, le 2-, 4-, 5-, 6- ou 7-benzoxazolyle, le 3-, 4-, 5-, 6- ou 7-benzisoxazolyle, le 1-, 3-, 4-, 5-, 6- ou 7-benzothiazolyle, le 2-, 4-, 5-, 6- ou 7-benzisothiazolyle, le 2-, 4-, 5-, 6- ou 7-benz-1,3-oxadiazolyle, le 2-, 3-, 4-, 5-, 6-, 7- ou 8-quinoléinyle, le 1-, 3-, 4-, 5-, 6-, 7-, 8-isoquinoléinyle, le 1-, 2-, 3-, 4- ou 9-carbazolyle, le 1-, 2-, 3-, 4-, 5-, 6-, 7-, 8- ou 9-acridinyle, ou le 2-, 4-, 5-, 6-, 7- ou 8-quinazoléinyle, le 2- ou 3-thiényle, le 1,3,4-thiadiazolyle, le 3-pyrryle, le 3-pyrazolyle, le 2-thiazolyle ou 5-thiazolyle.

5. Composé selon la revendication 1, dans lequel A est un groupe cyclohéxyle ; ou un groupe aryle en C_{5 à 12} ou hétéroaryle en C_{5 à 12} chacun indépendamment facultativement substitué jusqu'à trois fois par (i) un groupe alkyle en C₁ à C₁₀ ou alcényle en C_{2 à 10} chacun facultativement substitué par un atome d'halogène jusqu'au perhalogéno ; (ii) un groupe cycloalkyle en C₃ à C₁₀ ; (iii) un groupe aryle en C_{5 à 12} facultativement substitué par 1 à 3 atomes d'halogène ; (iv) un groupe hétéroaryle en C_{5 à 12}; (v) un atome d'halogène ; (vi) -CO-OR₈ ; (vii) -CO-R₈ ; (viii) un groupe cyano ; (ix) -NR₈R₁₃ ; (x) un groupe nitro ; (xi) -CO-NR₈R₉ ; (xii) -(alkyl en C_{1 à 10})-NR₈R₉ ; (xiii) -NR₈-CO-R₁₂ ; (xiv) -NR₈-CO-OR₉ ; (xv) -NR₈-SO₂-R₉ ; (xvi) -SR₈ ; (xvii) -SO₂-R₈ ; (xviii) -SO₂-NR₈R₉ ; ou (xix) NR₈-CO-NHR₉.

6. Composé selon l'une quelconque des revendications 1 à 3, dans lequel A est le phényle, le pyridyle, le pyrimidinyle, l'oxazolyle, le furyle, le thiényle, le pyrrolyle, l'imidazolyle, l'isoxazolyle et le pyrazinyle, chacun indépendamment substitué jusqu'à trois fois par un atome d'halogène, un groupe alkyle en C_{1 à 10}, (alcoxy en C_{1 à 10})phényle, naphthyle, -OR₁₀, 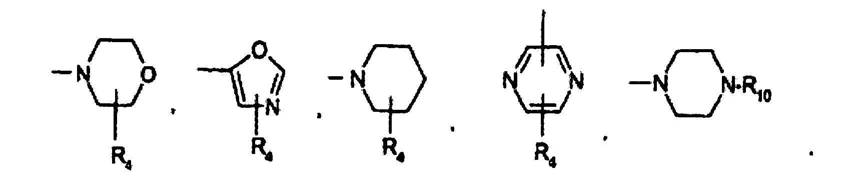 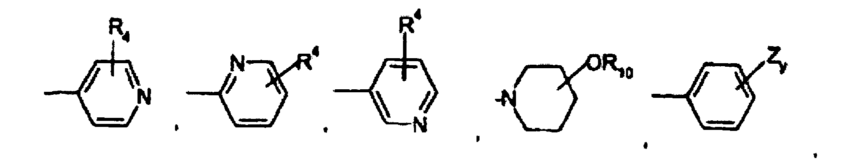 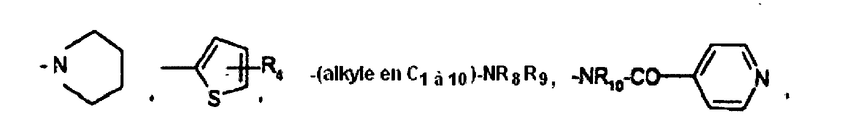 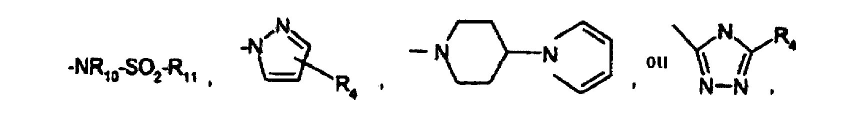 où chaque Z est indépendamment un atome d'halogène, un groupe hydroxy, hydroxy-alkyle en C_{1 à 10}, -CN, -NO₂, alcoxy en C_{1 à 10} carboxyle, -NR₁₀-CO-R₁₁ ou -NR₁₀-CO-OR₁₁, et
y vaut 1 à 3.

7. Composé selon la revendication 1 ou 3, dans lequel A est 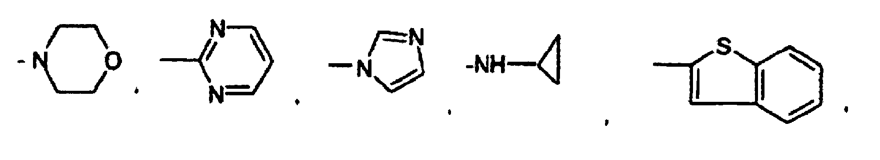 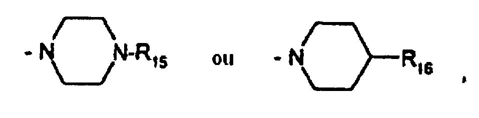 où R₁₅ est H ; un groupe phényle facultativement substitué par un groupe alkyle en C_{1 à 10}, alcoxy en C_{1 à 10}, alkylcarboxyle en C_{1 à 10}, ou un atome d'halogène ; un groupe benzyle ; pyrimidyle ou pyridyle ; et R₁₆ est H, un groupe phényle, -COOR₁₀,

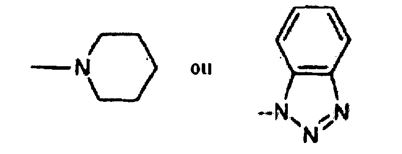

8. Composé selon la revendication 1, dans lequel R⁵ dans a, b ou c est un atome d'hydrogène ou un groupe alkyle en C_{1 à 10} ou alkyle en C_{2 à 10} facultativement substitué par un groupe amino, N-alkyl inférieur amino; N,N-dialkyl inférieur amino, N-alcanoyl inférieur amino, hydroxy, cyano, -COOR₁₀, -COR₁₄, -OCOR₁₄, -OR₁₀, hétéroaryle en C_{5 à 10}, hétéroaryloxy en C_{5 à 10} ou hétéroaryl en C_{5 à 10}- alcoxy en C_{1 à 10}, un atome d'halogène jusqu'au perhalogéno ; (iii) un groupe cycloalkyle en C₃ à C₁₀, dans lequel 1 à 3 atomes de carbone sont facultativement indépendamment remplacé par O, N ou S ; (iv) un groupe cycloalcényle en C_{3 à 10} ; (v) un groupe hétérocyclyle en C_{5 à 10} partiellement insaturé ; (vi) un groupe aryle ; (vii) un groupe hétéroaryle ; (viii) un atome d'halogène ; (ix) -CO-OR₁₀ ; (x) -OCOR₁₀ ; (xi) -OCO₂R₁₀ ; (xii) -CHO ; (xiii) un groupe cyano ; (xiv) -OR₁₆; (xv) -NR₁₀R₁₅ ; (xvi) un groupe nitro ; (xvii) -CO-NR₁₀R₁₁ ; (xviii) -NR₁₀-CO-R₁₂ ; (xix) -NR₁₀₋CO-OR₁₁ ; (xx) -NR₁₀-SO₂-R₁₂ ; (xxi) -SR₁₆ ; (xxii) -SOR₁₆ ; (xxiii) -SO₂-R₁₆ ; (xxiv) -SO₂₋NR₁₀R₁₁ ; (xxv) NR₁₀-CO-NHR₁₁ ; (xxvi) un amidino ; (xxvii) un groupe guanidino ; (xxviii) un groupe sulfo ; (xxix) -B(OH)₂ ; (xxx) -OCON(R₁₀)₂ ; ou (xxxi) -NR₁₀CON(R₁₀)₂.

9. Composé selon la revendication 1, dans lequel Y est N et R₁ est H.

10. Composé selon la revendication 9, dans lequel a est -NR₆-, R₆ est H et c est -N=.

11. Composé selon la revendication 10, dans lequel p vaut 0 et R_{1 à 4} sont H.

12. Composé selon la revendication 11, dans lequel X est -(CH₂)ₓ- et x vaut 0.

13. Composé selon la revendication 12, dans lequel A est un groupe biphényle facultativement substitué par un atome d'halogène.

14. Composé selon la revendication 1, de formule :
2-(2,4-dichlorophényl)-*N*-(1*H*-indazol-5-yl)-4-quinazolinamine, 2-(4-chlorophényl)-*N*-(1*H*-indazol-5-yl)-4-quinazolinamine, 1-{4-[4-(1*H*-indazol-5-ylamino)-2-quinazolinyl]phényl}éthanone, *N*-(1*H*-indazol-5-yl)-2-[4-(trifluorométhyl)phényl]-4-quinazolinamine, 2-(3-chloro-4-fluorophényl)-*N*-(1*H*-indazol-5-yl)-4-quinazolinamine, 2-(1,3-benzodioxol-5-yl)-*N*-(1*H*-indazol-5-yl)-4-quinazolinamine, *N*-(1*H*-indazol-5-yl)-2-(4-méthylphényl)-4-quinazolinamine, 2-(3,4-dichlorophényl)-*N*-(1*H*-indazol-5-yl)-4-quinazolinamine, *N*-(1*H*-indazol-5-yl)-2-(1-naphthyl)-4-quinazolinamine, *N*-(1*H*-indazol-5-yl)-2-(3,4,5-triméthoxyphényl)-4-quinazolinamine, 2-(1-benzokran-2-yl)-*N*-(1*H*-indazol-5-yl)-4-quinazolinamine, *N*-(1*H*-indazol-5-yl)-2-(2-thiényl)-4-quinazolinamine, *N*-(1*H*-indazol-5-yl)-2-(3-thiényl)-4-quinazolinamine, 2-(3,5-diméthyl-4-isoxazolyl)-*N*-(1*H*-indazol-5-yl)-4-quinazolinamine 2-(1,1'-biphényl-4-yl)-*N*-(1*H*-indazol-5-yl)-4-quinazolinamine, 2-[4-(diméthylamino)phényl]-*N*-(1*H*-indazol-5-yl)-4-quinazolinamine, 2-(1-benzothie*N*-2-yl)-*N-*(1*H*-indazol-5-yl)-4-quinazolinamine, 4-[4-(1*H*-indazol-5-ylamino)-2-quinazolinyl]phénol, 2-dibenzo[b,d]furan-1-yl-*N*-(1*H*-indazol-5-yl)-4-quinazolinamine, 2-(2-fluoro-1,1'-biphényl-4-yl)-*N*-(1*H*-indazol-5-yl)-4-quinazolinamine, 7-chloro-*N*-(1*H*-indazol-5-yl)-2-phényl-4-quinazolinamine, *N*-(1*H*-indazol-5-yl)-6-nitro-2-phényl-4-quinazolinamine, 2-(4-fluorophényl)-*N*-(1*H*-indazol-5-yl)-6-nitro-4-quinazolinamine, 6-chloro-*N*-(1*H*-indazol-5-yl)-2-(4-méthylphény)-4-quinazolinamine, 6-chloro-2-(4-fluorophényl)-*N*-(1*H*-indazol-5-yl)-6-nitro-4-quinazolinamine, 2-(4-bromophényl)-6-chloro-*N*-(1*H*-indazol-5-yl)-4-quinazolinamine, *N*-(1*H*-indazol-5-yl)-2-(2-quinoxalinyl)-4-quinazolinamine, 5-fluoro-*N-*(1*H*-indazol-5-yl)-2-(2-méthylphényl)-4-quinazolinamine, 5-fluoro-2-(4-fluorphényl)-*N*-(1*H-*indazol-5-yl)-4-quinazolinamine, 2-(3-chlorophényl)-5-fluoro-*N*-(1*H*-indazol-5-yl)-4-quinazolinamine, 2-(4-bromophényl)-5-fluoro-*N*-(1*H*-indazol-5-yl)-4-quinazolinamine, chlorhydrate de 5-fluoro-*N*-(1*H*-indazol-5-yl)-2-(3-méthylphényl)-4-quinazolinamine, chlorhydrate de 2-(3-bromophényl)-5-fluoro-*N*-(1*H*-indazol-5-yl)-4-quinazolinamine, 2-(2-chlorophényl)-5-fluoro-*N*-(1*H*-indazol-5-yl)-4-quinazolinamine, 5-fluoro-*N*-(1*H*-indazol-5-yl)-2-(2-quinoxalinyl)-4-quinazolinamine tris(trifluoroacétate), 5-fluoro-*N*-(1*H*-indazol-5-yl)-2-(1-naphthyl)-4-quinazolinamine bis(trifluoroacétate), 5-fluoro-*N*-(1*H*-indazol-5-yl)-2-(2-naphthyl)-4-quinazolinamine bis(trifluoroacétate), 5-fluoro-*N*-(1*H*-indazol-5-yl)-2-(4-pyridinyl)-4-quinazolinamine tris(trifluoroacétate), *N*-(1*H*-indazol-5-yl)-7-méthyl-2-(2-quinoxalinyl)-4-quinazolinamine, 2-(3-chlorophényl)-*N*-(1*H*-indazol-5-yl)-7-méthyl-4-quinazolinamine, 2-(4-fluorophényl)-*N*-(1*H*-indazol-5-yl)-7-méthyl-4-quinazolinamine, *N-*(1*H*-indazol-5-yl)-7-méthyl-2-(4-méthylphényl)-4-quinazolinamine", 2-(4-bromophényl)-*N-*(1*H*-indazol-5-yl)-7-méthyl-4-quinazolinamine, *N*-(1*H*-indazol-5-yl)-7-méthyl-2-(2-méthylphényl)-4-quinazolinamine bis(trifluoroacétate), *N*-(1*H*-indazol-5-yl)-7-méthyl-2-(3-méthylphényl)-4-quinazolinamine bis(trifluoroacétate), -*N*-[2-(3-fluorophényl)-7-méthyl-4-quinazolinyl]-*N*-(1*H*-indazol-5-yl)amine bis(trifluoroacétate), 2-(3-bromophényl)-*N*-(1*H-*indazol-5-yl)-7-méthyl-4-quinazolinamine bis(trifluoroacétate), *N*-[2-(2-chlorophényl)-7-méthyl-4-quinazolinyl]-*N*-(1*H*-indazol-5-yl)amine bis(trifluoroacétate), 2-(3-furyl)-*N*-(1*H-*indazol-5-yl)-7-méthyl-4-quinazolinamine bis(trifluoroacétate), *N*-(1*H*-indazol-5-yl)-7-méthyl-2-(1-naphthyl)-4-quinazolinamine bis(trifluoroacétate), *N*-(1*H*-indazol-5-yl)-7-méthyl-2-(2-naphthyl)-4-quinazolinamine bis(trifluoroacétate), *N*-(1*H*-indazol-5-yl)-7-méthyl-2-(3-pyridinyl)-4-quinazolinamine tris(trifluoroacétate), *N*-(1*H*-indazol-5-yl)-7-méthyl-2-(4-pyridinyl)-4-quinazolinamine tris(trifluoroacétate), 7-chloro-2-(3-chlorophényl)-*N*-(1*H*-indazol-5-yl)-4-quinazolinamine, 7-chloro-*N*-(1*H*-indazol-5-yl)-2-(4-méthylphényl)-4-quinazolinamine, 2-(4-bromophényl)-7-chloro-*N*-(1*H*-indazol-5-yl)-4-quinazolinamine, chlorhydrate de 7-chloro-*N*-(1*H*-indazol-5-yl)-2-(3-méthylphényl)-4-quinazolinamine, 7-chloro-2-(3-fluorophényl)-*N*-(1*H*-indazol-5-yl)-4-quinazolinamine bis(trifluoroacétate), 2-(3-bromophényl)-7-chloro-*N*-(1*H*-indazol-5-yl)-4-quinazolinamine bis(trifluoroacétate), *N*-[7-chloro-2-(2-furyl)-4-quinazolinyl]-*N*-(1*H*-indazol-5-yl)amine bis(trifluoroacétate), 7-chloro-*N*-(1*H*-indazol-5-yl)-2-(2-quinoxalinyl)-4-quinazolinamine tris(trifluoroacétate), 7-chloro-*N-*(1*H*-indazol-5-yl)-2-(1-naphthyl)-4-quinazolinamine bis(trifluoroacétate), 7-chloro-*N*-(1*H-*indazol-5-yl)-2-(2-naphthyl)-4-quinazolinamine bis(trifluoroacétate), 7-chloro-*N*-(1*H-*indazol-5-yl)-2-(3-pyridinyl)-4-quinazolinamine tris(trifluoroacétate), 2-(4-fluorophényl)-*N-*(1*H*-indazol-5-yl)-6,7-diméthoxy-4-quinazolinamine, 2-(1,1'-biphényl-4-yl)-*N*-(1*H*-indazol-5-yl)-6,7-diméthoxy-4-quinazolinamine, *N*-(1*H*-indazol-5-yl)-6,7-diméthoxy-2-(4-vinylphényl)-4-quinazolinamine, *N*-cyclopentyl-4-(1*H*-indazol-5-ylamino)-4-quinazolinecarboxamide, *N-*(3-fluorophényl)-*N*-[4-(1*H*-indazol-5-ylamino)-6,7-diméthoxy-2-quinazolinyl]amine, *N*-(2,4-difluorobenzyl)-*N*-[4-(1*H*-indazol-5-ylamino)-6,7-diméthoxy-2-quinazolinyl]amine, *N*-(2-fluorobenzyl)-*N*-[4-(1*H*-indazol-5-ylamino)-6,7-diméthoxy-2-quinazolinyl]amine, *N*-(4-bromophényl)-*N*-[4-(1*H*-indazol-5-ylamino)-6,7-diméthoxy-2-quinazolinyl]amine, *N*-(6,7-diméthoxy-2-{[4-(trifluorométhyl)phényl]amino}-4-quinazolinyl)-*N*-(1*H*-indazol-5-yl)amine, *N*-(6,7-diméthoxy-2-{[4-(trifluorométhyl)benzyl]amino}-4-quinazolinyl)-*N*-(1*H*-indazol-5-yl)amine, *N*-[3-fluoro-5-(trifluorométhyl)benzyl]-*N*-[4-(1*H*-indazol-5-ylamino)-6,7-diméthoxy-2-quinazolinyl]amine, *N*-(3-fluorobenzyl)-*N*-[4-(1*H*-indazol-5-ylamino)-6,7-diméthoxy-2-quinazolinyl]amine, *N*-(2,4-difluorobenzyl)-*N*-[4-(1*H*-indazol-5-ylamino)-6,7-diméthoxy-2-quinazolinyl]amine, *N*-(4-fluorobenzyl)-*N*-[4-(1*H*-indazol-5-ylamino)-6,7-diméthoxy-2-quinazolinyl]amine, *N*-(2,6-difluorobenzyl)-*N*-[4-(1*H*-indazol-5-ylamino)-6,7-diméthoxy-2-quinazoliny]amine, *N*-(3,5-difluorobenzyl)-*N*-[4-(1*H*-indazol-5-ylamino)-6,7-diméthoxy-2-quinazolinyl]amine, *N*-(3-bromophényl)-*N*-[4-(1*H*-indazol-5-ylamino)-6,7-diméthoxy-2-quinazolinyl]amine, *N*-(2,6-difluorophényl)-*N*-[4-(1*H*-indazol-5-ylamino)-6,7-diméthoxy-2-quinazolinyl]amine, *N*-(2,5-difluorophényl)-*N*-[4-(1*H*-indazol-5-ylamino)-6,7-diméthoxy-2-quinazolinyl]amine, *N*-(2,4-difluorophényl)-*N*-[4-(1*H*-indazol-5-ylamino)-6,7-diméthoxy-2-quinazolinyl]amine, *N*-(2,3-difluorophényl)-*N*-[4-(1*H*-indazol-5-ylamino)-6,7-diméthoxy-2-quinazolinyl]amine, *N*-(3,4-difluorophényl)-*N*-[4-(1*H*-indazol-5-ylamino)-6,7-diméthoxy-2-quinazolinyl]amine, *N*-(3,5-difluorophényl)-*N*-[4-(1*H*-indazol-5-ylamino)-6,7-diméthoxy-2-quinazolinyl]amine, *N*-{6,7-diméthoxy-2-[(2,3,4-trifluorophényl)amino]-4-quinazolinyl}-*N*-(1*H*-indazol-5-yl)amine, *N*-{6,7-diméthoxy-2-[(2,4,5-trifluorophényl)amino]-4-quinazolinyl}-*N*-(1*H*-indazol-5-yl)amine, *N*-{6,7-diméthoxy-2-[(2,4,6-trifluorophényl)amino-4-quinazolinyl}-*N*-(1*H*-indazol-5-yl)amine, *N*-{6,7-diméthoxy-2-[(2,3,6-trifluorophényl)amino]-4-quinazolinyl}-*N*-(1*H*-indazol-5-yl)amine, *N*-(4-bromophényl)-*N*-[4-(1*H*-indazol-5-ylamino)-6,7-diméthoxy-2-quinazolinyl]amine, 2-(3-aminophényl)-*N*-(1*H*-indazol-5-yl)-4-quinazolinamine, *N*-{3-[4-(1*H*-indazol-5-ylamino)-2-quinazolinyl]phényl}isonicotinamide, *N*-{3[4-(1*H*-indazol-5-ylamino)-2-quinazolinyl]phényl}acétamide, *N*-(4-chlorophényl)-*N*-[4-(1*H*-indazol-5-ylamino)-2-quinazolinyl]amine, *N*-(3-bromophényl)-*N*-[4-(1*H*-indazol-5-ylamino)-2-quinazolinyl]amine, *N*-(2-chlorophényl)-*N*-[4-(1*H*-indazol-5-ylamino)-2-quinazolinyl]amine, *N*-(3-fluorophényl)-*N*-[4-(1*H*-indazol-5-ylamino)-2-quinazolinyl]amine, *N*-(2-fluorophényl)-*N*-[4-(1*H*-indazol-5-ylamino)-2-quinazolinyl]amine, *N*-(3-chlorophényl)-*N*-[4-(1*H*-indazol-5-ylamino)-2-quinazolinyl]amine, *N*-(4-bromophényl)-*N*-[4-(1*H*-indazol-5-ylamino)-2-quinazolinyl]amine, *N*-(1*H*-indazol-5-yl)-*N*-(2-{[3-(trifluorométhyl)phényl]amino}-4-quinazolinyl]amine, *N*-(1*H-*indazol-5-yl)-*N*-(2-[(4-phénoxyphényl)amino]-4-quinazolinyl}amine, *N*-(1*H*-indazol-5-yl)-*N-*(2-{[4-(trifluorométhoxy)phényl]amino}-4-quinazolinyl)amine, *N*-(1*H*-indazol-5-yl)-*N*-(2-{[3-(trifluorométhoxy)phényl]amino}-4-quinazolinyl)amine, *N*-(4-fluorophényl)-*N*-[4-(1*H-*indazol-5-ylamino)-2-quinazolinyl]amine, *N*-(2-anilino-4-quinazolinyl)-*N*-(1*H*-indazol-5-yl)amine, 2-[4-(2-chlorophényl)-1-pipérazinyl]-*N*-(1*H*-indazol-5-yl)-4-quinazolinamine, *N-*(1*H*-indazol-5-yl)-2-[4-(2-pyrimidinyl)-1-pipérazinyl]-4-quinazolinamine, *N*-(1*H*-indazol-5-yl)-2-[4-(2-méthoxyphényl)-1-pipérazinyl]-4-quinazolinamine, 1-(4-{4-[4-(1*H*-indazol-5-ylamino)-2-quinazolinyl]-1-pipérazinyl}phényl)éthanone, 4-(1*H*-indazol-5-ylamino)-2-quinazolinecarboxamide", 4-(1*H*-indazol-5-ylamino)-*N*-(4-pyridinyl)-2-quinazolinecarboxamide, 4-(1*H*-indazol-5-ylamino)-*N*-(4-méthoxyphényl)-2-quinazolinecarboxamide, *N*-cyclohéxyl-4-(1*H*-indazol-5-ylamino)-2-quinazolinecarboxamide, *N*-cyclopentyl-4-(1*H*-indazol-5-ylamino)-2-quinazolinecarboxamide, 4-(1*H*-indazol-5-ylamino)-*N*-(2-pyridinyl)-2-quinazolinecarboxamide, 4-(1*H*-indazol-5-ylamino)-*N*-(3-quinolinyl)-2-quinazolinecarboxamide, 4-(1*H*-indazol-5-ylamino)-*N*-méthyl-2-quinazolinecarboxamide, *N*-(1*H*-indazol-5-yl)-2-(4-morpholinylcarbonyl)-4-quinazolinamine, 2-(2,3-dihydro-1-benzofuran-5-yl)-*N*-(1*H*-indazol-5-yl)-4- quinazolinamine, 2-cyclopropyl-*N*-(1*H*-indazol-5-yl)-4-quinazolinamine, dichlorhydrate de 2-(2-fluoro-1,1'-biphényl-4-yl)-*N-*(1*H*-indazol-5-yl)-4-quinazolinamine, 2-(2-fluoro-1,1'-biphényl-4-yl)-*N*-(1*H*-indazol-5-yl)-4-quinazolinamine diméthanesulfonate, 2-(2-fluoro-1,1'-biphényl-4-yl)-*N*-(1*H*-indazol-5-yl)-4-quinazolinamine benzènesulfonate, 2-(2-fluoro-1,1'-biphényl-4-yl)-*N*-(1*H*-indazol-5-yl)-4-quinazolinamine 4-méthylbenzènesulfonate, ou 2-dibenzo[*b*,*d*]furan-1-yl-*N*-(1*H*-indazol-5-yl)-4-quinazolinamine trifluoroacétate, 2-(3-fluoro-1,1-biphényl-4-yl)-*N*-(1*H*-indazol-5-yl)-4-quinazolinamine ; 2-(2-furyl)-*N*-(1*H*-indazol-5-yl)-7-méthyl-4-quinazolinamine ; 2-(1-benzofuranyl)-*N*-(1*H*-indazol-5-yl)-6,7-diméthoxy-4-quinazolinamine ; *N*-(2,5-difluorobenzyl)-*N*-[4-(1*H*-indazol-5-ylamino)-6,7-diméthoxy-2-quinazolinyl]amine; *N*-{6,7-diméthoxy-2-[(2,3,5-trifluorophényl)amino]-4-quinazolinyl}-*N*-(1*H*-indazol-5-yl)amine ; *N-*(1*H*-indazol-5-yl)-2-[5-(1*H*-indolyl)amino]-4-quinazolinamine ; *N*-(1*H*-indazol-5-yl)-2-[4-phénoxyanilino]-4-quinazolinamine ; *N*-(1*H*-indazol-5-yl)-2-[2-naphthylamino]-4-quinazolinamine.

15. Composé de formule :
*N*-(1*H*-indazol-5-yl)-2-(trifluorométhyl)-4-quinazolinamine ; 2-chloro-*N*-(3-éthyl-1*H-*indazol-5-yl)-4-quinazolinamine ; 2-chloro-*N*-(1*H*-indazol-5-yl)-4-quinazolinamine.

16. Procédé de préparation d'un composé selon revendication 1, comprenant les étapes consistant à
(a) faire réagir un composé de formule II 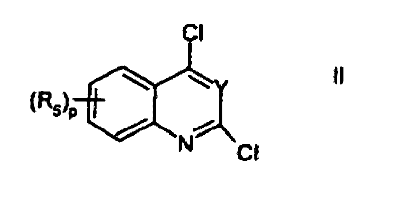 avec un composé de formule III 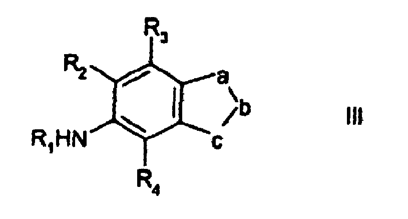 en présence d'une base, pour produire un composé de formule IV 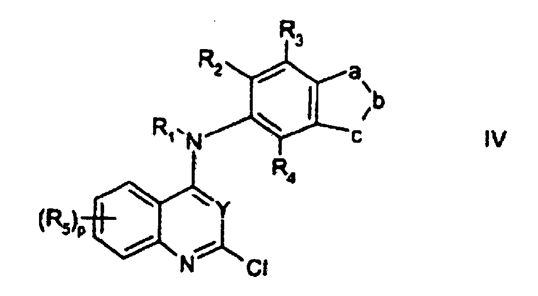 et facultativement faire en outre réagir IV avec un acide arylboronique ou A-NH₂, ou
(b) faire réagir un chlorure de benzoyle substitué avec de la diméthylamine pour produire un composé de formule V 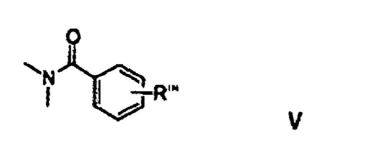
dans laquelle R''' est (i) un groupe alkyle en C₁ à C₁₀ ou alcényle en C₂ à C₁₀, chacun facultativement substitué par un atome d'halogène jusqu'au perhalogéno ; (ii) un groupe cycloalkyle en C₃ à C₁₀ ; (iii) un groupe aryle ; (iv) un groupe hétéroaryle ; (v) un atome d'halogène ; (vi) -CO-OR₈ ; (vii) -CO-R₈ ; (viii) un groupe cyano ; (ix) -OR₈ ; (x) -NR₈R₁₃ ; (xi) un groupe nitro ; (xii) -CO-NR₈R₉ ; (xiii) -(alkyl en C_{1 à 10})-NR₈R₉ ; (xiv) -NR₈₋CO-R₁₂ ; (xv) -NR₈CO-OR₉ ; (xvi) -NR₈-SO₂-R₉ ; (xvii) -SR₈ ; (xviii) -SO₂-R₈ ; (xix) -SO₂NR₈R₉ ; ou (xx) NR₈-CO-NHR₉,
faire réagir V avec du chloro-2-amino-benzonitrile pour produire un composé de formule VI 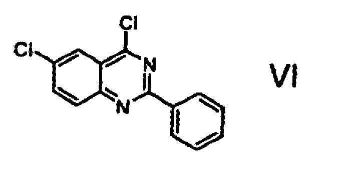 et faire réagir VI avec de l'aminoindazole.

17. Procédé de préparation de 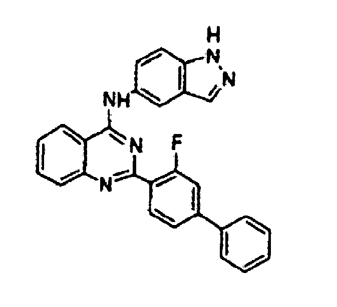 comprenant les étapes consistant à faire réagir de l'acide 3-fluoro-4-phénylbenzoïque 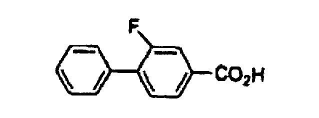 avec du 4-bromo-2-fluorobiphényle pour produire du 2[(3-fluoro-4-phénylphényl)carbonylamino]benzamide 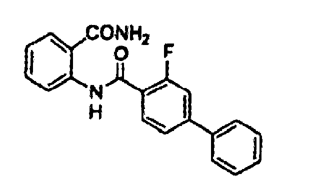 cycliser pour produire de la 2-(3-fluoro-1,1'-biphényl-4-yl)-4(3*H*)-quinazolinone 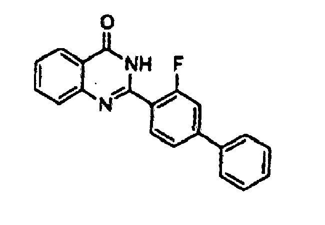 faire réagir pour produire de la 4-chloro-2-(3-fluoro-4-phénylphényl)quinazoline 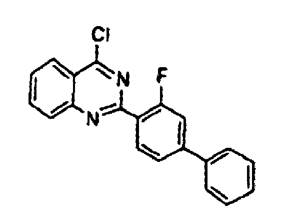 puis faire réagir avec de l'aminoindazole pour produire

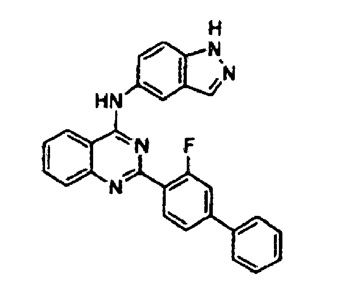

18. Composition pharmaceutique, **caractérisée en ce qu'**elle comprend un ou plusieurs composés selon l'une quelconque des revendications 1 à 17, en association avec un ou plusieurs véhicules pharmaceutiquement acceptables non toxiques, et, facultativement, d'autres ingrédients actifs.

19. Utilisation d'un composé selon l'une quelconque des revendications 1 à 18 pour la préparation d'un médicament destiné à traiter une indication médiée par la Rho-kinase.

20. Utilisation d'un composé selon la revendication 13, pour la préparation d'un médicament destiné à traiter une indication médiée par la Rho-kinase.

21. Utilisation d'un composé selon la revendication 14, pour la préparation d'un médicament destiné à traiter une indication médiée par la Rho-kinase.

22. Utilisation d'un composé selon la revendication 15, pour la préparation d'un médicament destiné à traiter une indication médiée par la Rho-kinase.

23. Utilisation d'un composé selon l'une quelconque des revendications 1 à 18, pour la préparation d'un médicament destiné à traiter une hypertension, une athérosclérose, une resténose, une ischémie cérébrale, un vasospasme cérébral, une dégénérescence neuronale, une lésion de la moelle épinière, un cancer du sein, du colon, de la prostate, des ovaires, du cerveau ou des poumons, des troubles thrombotiques, l'asthme, un glaucome, l'ostéoporose ou un dysfonctionnement érectile.

24. Utilisation d'un composé selon la revendication 13, pour la préparation d'un médicament destiné à traiter une hypertension, une athérosclérose, une resténose, une ischémie cérébrale, un vasospasme cérébral, une dégénérescence neuronale, une lésion de la moelle épinière, un cancer du sein, du colon, de la prostate, des ovaires, du cerveau ou des poumons, des troubles thrombotiques, l'asthme, un glaucome, l'ostéoporose ou un dysfonctionnement érectile.

25. Utilisation d'un composé selon la revendication 14, pour la préparation d'un médicament destiné à traiter une hypertension, une athérosclérose, une resténose, une ischémie cérébrale, un vasospasme cérébral, une dégénérescence neuronale, une lésion de la moelle épinière, un cancer du sein, du colon, de la prostate, des ovaires, du cerveau ou des poumons, des troubles thrombotiques, l'asthme, un glaucome, l'ostéoporose ou un dysfonctionnement érectile.

26. Utilisation d'un composé selon la revendication 15, pour la préparation d'un médicament destiné à traiter une hypertension, une athérosclérose, une resténose, une ischémie cérébrale, un vasospasme cérébral, une dégénérescence neuronale, une lésion de la moelle épinière, un cancer du sein, du colon, de la prostate, des ovaires, du cerveau ou des poumons, des troubles thrombotiques, l'asthme, un glaucome, l'ostéoporose ou un dysfonctionnement érectile.

27. Utilisation selon l'une quelconque des revendications 18 à 26, dans laquelle l'hôte est un humain.
